(19) Europäisches Patentamt
European Patent Office
Office européen des brevets

(11) EP 1 749 820 A1

(12) **EUROPEAN PATENT APPLICATION**

(43) Date of publication:
**07.02.2007 Bulletin 2007/06**

(51) Int Cl.:
***C07D 231/06*** *(2006.01)* ***A61K 31/4155*** *(2006.01)*
***A61P 25/00*** *(2006.01)*

(21) Application number: **05384025.2**

(22) Date of filing: **15.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LABORATORIOS DEL DR. ESTEVE, S.A.**
**08041 Barcelona (ES)**

(72) Inventors:
- **Torrens Jover, Antonio**
  **08221 Terrasa (Barcelona) (ES)**
- **Mas Prio, José**
  **08191-Rubi Barcelona (ES)**
- **Cuberes Altisen, Maria Rosa**
  **San Cugat del Valles, Barcelona (ES)**
- **Sola, Luis**
  **Av. Paisos Catalans 16,**
  **43007 Tarragona (ES)**
- **Benet Buschholz, Jordi**
  **Av. Paisos Catalans 16,**
  **43007 Tarragona (ES)**

(54) **Salts of substituted pyrazoline compounds, their preparation and use as medicaments**

(57) The present invention relates to salts of substituted pyrazoline compounds, methods for their preparation, medicaments comprising these compounds as well as their use for the preparation of a medicament for the treatment of humans and animals.

**Figure 1**

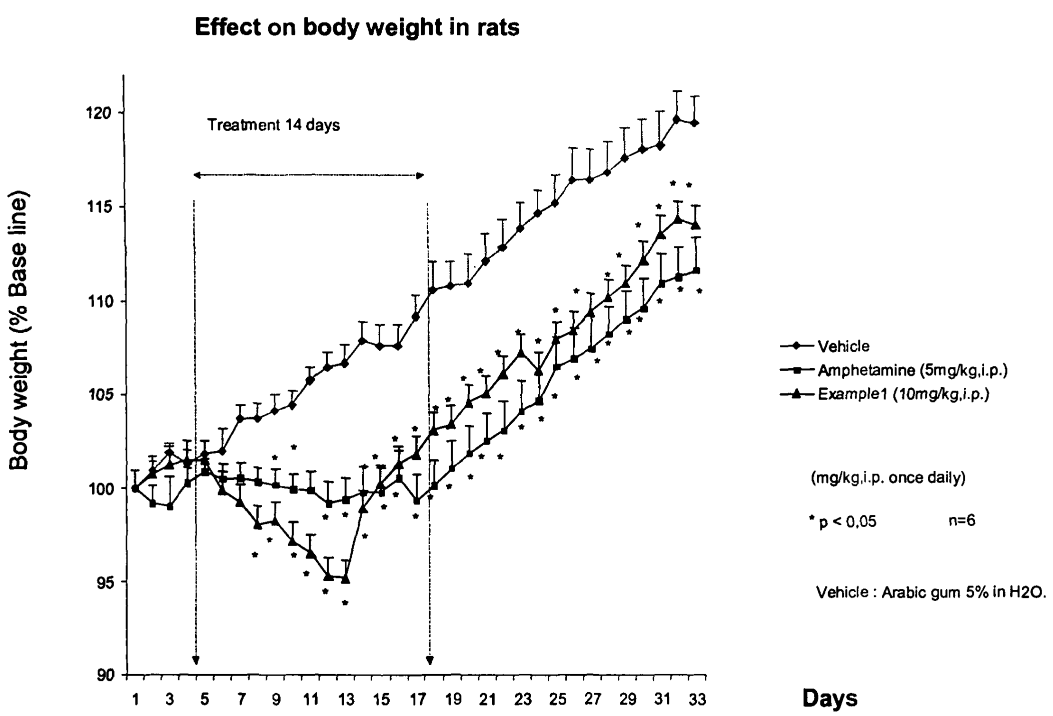


**EP 1 749 820 A1**

## Description

The present invention relates to salts of substituted pyrazoline compounds, methods for their preparation, medicaments comprising these compounds as well as their use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of Cannabinoid receptors, more particularly for the modulation of Cannabinoid 1 ($CB_1$) receptors.

Said object was achieved by providing the salts of substituted pyrazoline compounds of general formula I given below, their stereoisomers, corresponding salts and corresponding solvates thereof.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to a salt of a substituted pyrazoline compounds of general formula I,

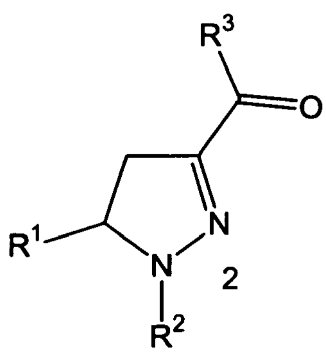

I

wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted mono- or polycydic ring system, or $R^3$ represents an optionally at least mono-substituted aryl or heteroaryl group, which may be condensed with an optionally at least mono-substituted mono- or polycydic ring system, or $R^3$ represents an — $NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-subsbtuted aryl or heteroaryl group, which may be condensed with an optionally at least mono-substituted mono- or polycyclic ring system and/or bonded via a linear or branched alkylene group, an -$SO_2$-$R^6$-moiety, or an -$NR^7R^8$-moiety,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof,
with an acid with a $pk_a \leq 3.0$,
optionally in form of a corresponding solvate thereof .

Especially preferably the following proviso (disclaimer applies) in regards to the acid: with the proviso that the acid is not selected from hydrochloric acid, hydrobromic acid, phosphoric acid, sulphuric acid, nitric acid, citric acid, maleic acid, fumaric acid, tartaric acid, p-toluenesulfonic acid, methanesulfonic acid or camphersulfonic acid
Particularly preferably the following provisos (disclaimers) apply for the pyrazoline compounds of general formula I given above:

that $R^4$ and $R^5$ do not both represent a hydrogen atom, and

that if one of the residues $R^4$ and $R^5$ represents a hydrogen atom or an alkyl group, which is optionally at least mono-substituted with an alkoxy group, an alkoxyalkoxy group, a halogen atom or a phenyl group, the other one of these residues $R^4$ and $R^5$ does not represent a pyrid-2-yl group, which is optionally mono-substituted in the 5-position, a pyrid-5-yl group, which is optionally mono-substituted in the 2-position, a pyrimid-5-yl group, which is optionally mono-substituted in the 2-position, a pyridaz-3-yl group, which is optionally mono-substituted in the 6-position, a pyrazin-5-yl group, which is optionally mono-substituted in the 2-position, a thien-2-yl group, which is optionally mono-substituted in the 5 position, a thien-2-yl group, which is optionally at least mono-substituted in the 4-position, a benzyl group, which is optionally mono-substituted in the 4-position of the ring, a phenethyl group, which is optionally mono-substituted in the 4-position of the ring, an optionally mono-, di- or tri-substituted phenyl group, a di-substituted phenyl group, wherein the two substituents together form an —$OCH_2O$-, -$OCH_2CH_2O$- or -$CH_2CH_2O$- chain, which is optionally substituted with one or more halogen atoms or one or two methyl groups, an —NH-phenyl-moiety, wherein the phenyl group may be mono-substituted in the 4-position, and

that if one of the residues $R^4$ and $R^5$ represents an alkynyl group, the other one of these residues $R^4$ and $R^5$ does not represent a phenyl group, which is optionally substituted in the 4-position, and

that if one of the residues $R^4$ and $R^5$ represents a hydrogen atom or a linear or branched, saturated or unsaturated,

unsubstituted or substituted aliphatic radical, the other one of these residues $R^4$ and $R^5$ does not represent an unsubstituted or substituted thiazole group or an unsubstituted or substituted [1,3,4]thiadiazole group.

In a preferred embodiment of the salt according to the invention the substituted pyrazoline compound of general formula I, has a general formula according to general formula Ia or Ib

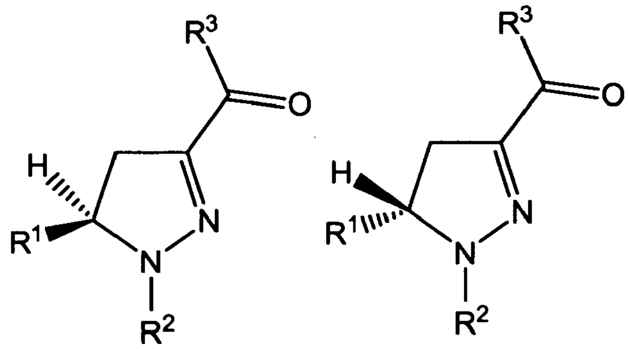

Ia Ib

A mono- or polycyclic ring-system according to the present invention means a mono- or polycydic hydrocarbon ring-system that may be saturated, unsaturated or aromatic. If the ring system is polycydic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

The term "condensed" according to the present invention means that a ring or ring-system is attached to another ring or ring-system, whereby the terms "annulated" or "annelated" are also used by those skilled in the art to designate this kind of attachment.

If one or more of the residues $R^3$-$R^8$ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, oxo, amino, carboxy, amido, cyano, nitro, $-SO_2NH_2$, -CO-$C_{1-4}$-alkyl, -SO-$C_{1-4}$-alkyl, $-SO_2$-$C_{1-4}$-alkyl, -NH-$SO_2$-$C_{1-4}$-alkyl, wherein the $C_{1-4}$-alkyl may in each case be branched or unbranched, and a phenyl group, more preferably be selected from the group consisting of hydroxy, F, Cl, Br, methyl, ethyl, methoxy, ethoxy, oxo, $CF_3$ and a phenyl group.

If one or more of the residues $R^3$-$R^8$ represents or comprises a cycloaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteratoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl, homo-Piperazinyl and Morpholinyl.

If one or more of the residues $R^3$-$R^8$ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, amino, carboxy, oxo, amido, cyano, nitro, $-SO_2NH_2$, -CO-$C_{1-4}$-alkyl, -SO-$C_{1-4}$-alkyl, $-SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, $CF_3$, oxo and a phenyl group.

If one or more of the residues $R^1$-$R^8$ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl

group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a nitro group, a carboxy group, a —CO-O-$C_{1-6}$-alkyl group, a —CO-$NR^AR^B$- moiety, a -CO-NH-$NR^CR^D$-moiety, an —SH, an -S-$C_{1-6}$-alkyl group, an -SO-$C_{1-6}$-alkyl group, an -$SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, an —$NH_2$-moiety, an NHR'-moiety or an NR'R"-moiety, wherein R' and R" independently represent a linear or branched $C_{1-6}$-alkyl group, a $C_{1-6}$-alkyl group substituted by one or more hydroxy groups and a -$C_{1-6}$-alkylene-$NR^ER^F$ group,

whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,

$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a — CO-O- $C_{1-6}$-alkyl group, a -CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a —CO-$NH_2$ group and/or which may contain at least one further heteroatom selected from the group consisting of nitrogen, oxygen and sulphur as a ring member, and

wherein $R^E$, $R^F$, identical or different, represent hydrogen or a $C_{1-6}$-alkyl group, or $R^E$ and $R^F$ together with the bridging nitrogen atom form a saturated, mono- or bicydic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues $R^3$-$R^8$ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a carboxy group, a —CO-O-$C_{1-6}$-alkyl group, a —CO-$NR^AR^B$- moiety, a -CO-NH-$NR^CR^D$-moiety, an -S-$C_{1-6}$-alkyl group, an -SO-$C_{1-6}$-alkyl group, an - $SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, a $C_{1-6}$-alkyl group substituted by one or more hydroxy groups and a —$C_{1-6}$-alkylene-$NR^ER^F$ group,

whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,

$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicydic, 3-10 membered heterocyclic ring system, which may be at least mono-substituted by one or more substituents independently selected from the group consisting of $C_{1-6}$ alkyl group, a -CO-$C_{1-6}$-alkyl group, a — CO-O- $C_{1-6}$-alkyl group, a -CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a —CO-$NH_2$ group and/or which may contain at least one further heteroatom selected from the group consisting of nitrogen, oxygen and sulphur as a ring member, and

wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo [2,1-b]thiazolyl, triazolyl and pyrazolyl.

If one or more of the residues $R^4$-$R^8$ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine,

chlorine, bromine, branched or unbranched $C_{1-4}$-alkoxy, branched or unbranched $C_{1-4}$-perfluoroalkoxy, branched or unbranched $C_{1-4}$-perfluoroalkyl, amino, carboxy, amido, cyano, nitro, $-SO_2NH_2$, - CO-$C_{1-4}$-alkyl, -SO-$C_{1-4}$-alkyl, $-SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methoxy, ethoxy, $CF_3$ and a phenyl group.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues $R^4$-$R^8$ represents or comprises a linear or branched alkylene group, said alkylene group may preferably be selected from the group consisting of —methylene $-(CH_2)-$, ethylene $-(CH_2-CH_2)-$, n-propylene $-(CH_2-CH_2-CH_2)-$ or iso-propylene —$(-C(CH_3)_2)-$.

Preferred in the salts according to the invention are substituted pyrazoline compounds of general formula I, Ia or Ib given above, wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an - $NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an $-SO_2-R^6$-moiety, or an $-NR^7R^8$-moiety,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycydic ring system, or an optionally at least mono-substituted aryl or heteroaryl group, which may be condensed with an optionally at least mono-substituted mono- or polycydic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof,
whereby preferably the following provisos (disclaimers) apply:

that $R^4$ and $R^5$ do not both represent a hydrogen atom, and that if one of the residues $R^4$ and $R^5$ represents a hydrogen atom or a linear or branched, saturated or unsaturated, substituted or unsubstituted aliphatic group, the other one of these residues $R^4$ and $R^5$ does not represent a substituted or unsubstituted pyridyl group, a substituted or unsubstituted pyrimidyl group, a substituted or unsubstituted pyridazyl group, a substituted or unsubstituted pyrazinyl group, a substituted or unsubstituted thienyl group, a substituted or unsubstituted benzyl group, a substituted or unsubstituted phenethyl group, a substituted or unsubstituted phenyl group, a substituted or unsubstituted phenyl group, which is condensed (attached) to at least one, optionally substituted ring or ringsystem, an —NH-phenyl-moiety, wherein the phenyl group may be at least mono-substituted, an unsubstituted or substituted thiazole group, or an unsubstituted or substituted [1,3,4]thiadiazole group.

Preferred are also salts of the substituted pyrazoline compounds of general formula I, Ia or Ib given above, wherein $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and - (C=O)-NR'R", whereby R' and R" for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is substituted with a chlorine atom in the 4-position, and $R^2$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are salts of the substituted pyrazoline compounds of general formula I, Ia or Ib given above, wherein $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and - (C=O)-NR'R" whereby R' and R" for each substituent independently represent linear or branched $C_{1-6}$ alkyl, preferably $R^2$ represents a phenyl group, which is optionally substituted by one or more substituents selected from the group consisting of methyl, ethyl, F, Cl, Br and $CF_3$, more preferably $R^2$ a phenyl group, which is di-substituted with two chlorine atoms in the 2- and 4-position, and $R^1$ and $R^3$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Preference is also given to salts of the substituted pyrazoline compounds of general formula I, Ia or Ib given above, wherein $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an - $NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an -$NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an —$NR^4R^5$-moiety and $R^1$, $R^2$ and $R^4$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Furthermore, salts of the substituted pyrazoline compounds of general formula I, Ia, Ib given above are preferred, wherein $R^4$ and $R^5$, identical or different, represent a hydrogen atom, an unbranched or branched, saturated or unsaturated, optionally at least mono-substituted $C_{1-6}$-aliphatic radical, a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-$CH_2$-) or ethylene (-$CH_2$-$CH_2$)-group, an —$SO_2$-$R^6$-moiety, or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an —$SO_2$-$R^6$-moiety, or an -$NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group, an optionally at least mono-substituted piperidinyl group, an optionally at least mono-substituted piperazinyl group, an optionally at least mono-substituted triazolyl group, an —$SO_2$-$R^6$-moiety, or an -$NR^7R^8$-moiety, or $R^4$ and $R^5$, identical or different, represent a methyl group, an ethyl group, an n-propyl group, an isopropyl group, an n-butyl group, a sec-butyl group or a tert.-butyl group, and $R^1$-$R^3$ and $R^6$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are salts of the substituted pyrazoline compounds of general formula 1, Ia or Ib given above, wherein $R^6$ represents a linear or branched, saturated or unsaturated, optionally at least mono-substituted $C_{1-6}$ aliphatic group, a saturated or unsaturated, optionally at least mono-substituted, optionally at least one heteroatom as ring member containing $C_{3-8}$ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene ($-CH_2-$) or ethylene ($-CH_2-CH_2$)-group, preferably $R^6$ represents a $C_{1-6}$-alkyl group, a saturated, optionally at least mono-substituted cycloaliphatic group, which may be condensed with a mono- or polycyclic ring-system, or a phenyl group, which is optionally substituted with one or more $C_{1-6}$ alkyl groups, and $R^1$-$R^5$, $R^7$ and $R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Moreover salts of the substituted pyrazoline compounds of general formula I, Ia or Ib given above are preferred, wherein $R^7$ and $R^8$, identical or different, represent a hydrogen atom, an unbranched or branched, saturated or unsaturated, optionally at least mono-substituted $C_{1-6}$ aliphatic radical, a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene ($-CH_2-$) or ethylene ($-CH_2-CH_2$)-group, preferably represent a hydrogen atom or a $C_{1-6}$ alkyl radical, and $R^1$-$R^6$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Particularly preferred are salts of compounds of general formula I, Ia or Ib given below,

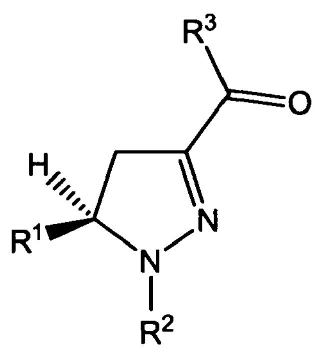
Ia

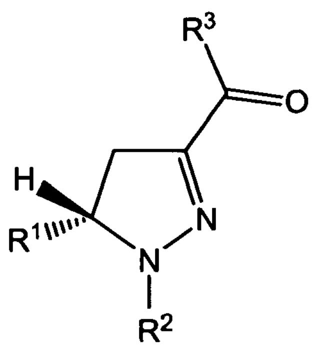
Ib

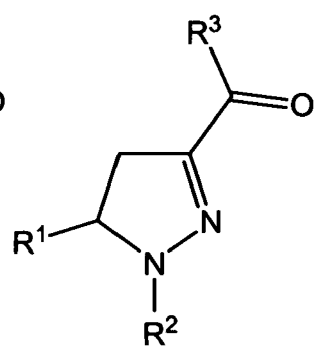
I

wherein

$R^1$ represents a phenyl ring, which is mono-substituted with a halogen atom, preferably a chlorine atom, in its 4-position,

$R^2$ represents a phenyl ring, which is di-substituted with two halogen atoms, preferably chlorine atoms, in its 2- and 4-position,

$R^3$ represents a pyrrolidinyl group, a piperidinyl group, a piperazinyl group, a homo-piperazinyl group, a morpholinyl group, or an $-NR^4R^5$-moiety,

$R^4$ represents a hydrogen atom or a linear or branched $C_{1-6}$-alkyl group,

$R^5$ represents a linear or branched $C_{1-6}$ alkyl group, a pyrrolidinyl group, a piperidinyl group, a piperazinyl group, a homo-piperazinyl group, a morpholinyl group, a triazolyl group, whereby each of the heterocyclic rings may be substituted with one or more, identical or different, $C_{1-6}$-alkyl groups, or an $-SO_2-R^6$-moiety, and

$R^6$ represents a phenyl group, which is optionally substituted with one or more $C_{1-6}$ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Most particularly preferred are salts of the substituted pyrazoline compounds selected from the group consisting of:

N-piperidinyl-5-(4-chloro-phenyl)-1-(2,4-dichlorophenyl)-4,5-dihydro-1H-pyrazole-3-carboxamide,

(rac)-N-piperidinyl-5-(4-chloro-phenyl)-1-(2,4-dichlorophenyl)-4,5-dihydro-1H-pyrazole-3-carboxamide,

(R)-N-piperidinyl-5-(4-chloro-phenyl)-1-(2,4-dichlorophenyl)-4,5-dihydro-1H-pyrazole-3-carboxamide,

(S)-N-piperidinyl-5-(4-chloro-phenyl)-1-(2,4-dichlorophenyl)-4,5-dihydro-1H-pyrazole-3-carboxamide,

5-(4-Chloro-phenyl)-1-(2,4-dichloro-phenyl)-4,5-dihydro-1H-pyrazole-3-carboxylic acid-[1,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone,

N-[5-(4-Chloro-phenyl)-1-(2,4-dichlorophenyl)-4,5-dihydro-1H-pyrazole-3-carbonyl]-4-methylphenylsufonamide,

1-{[5-(4-Chloro-phenyl)-1-(2,4-dichloro-phenyl)-4,5-dihydro-1H-pyrazole-3-carbonyl]-amino}-piperidinium; chloride,

1-{[5(R)-(4-Chloro-phenyl)-1-(2,4-dichloro-phenyl)-4,5-dihydro-1H-pyrazole-3-carbonyl]-amino}-piperidinium; chloride,

1-{[5(S)-(4-Chloro-phenyl)-1-(2,4-dichloro-phenyl)-4,5-dihydro-1H-pyrazole-3-carbonyl]-amino}-piperidinium; chloride

1-{[5-(4-Chloro-phenyl)-1-(2,4-dichloro-phenyl)-4,5-dihydro-1H-pyrazole-3-carbonyl]-amino}-piperidinium; nitrate,

1-{[5(R)-(4-Chloro-phenyl)-1-(2,4-dichloro-phenyl)-4,5-dihydro-1H-pyrazole-3-carbonyl]-amino}-piperidinium; nitrate,

1-{[5(S)-(4-Chloro-phenyl)-1-(2,4-dichloro-phenyl)-4,5-dihydro-1H-pyrazole-3-carbonyl]-amino}-piperidinium; nitrate,

1-{[5-(4-Chloro-phenyl)-1-(2,4-dichloro-phenyl)-4,5-dihydro-1H-pyrazole-3-carbonyl]-amino}-piperidinium; hydrogen sulfate,

1-{[5(R)-(4-Chloro-phenyl)-1-(2,4-dichloro-phenyl)-4,5-dihydro-1H-pyrazole-3-carbonyl]-amino}-piperidinium; hydrogen sulfate,

1-{[5(S)-(4-Chloro-phenyl)-1-(2,4-dichloro-phenyl)-4,5-dihydro-1H-pyrazole-3-carbonyl]-amino}-piperidinium; hydrogen sulfate,

Methanesulfonate1-{[5-(4-chloro-phenyl)-1-(2,4-dichloro-phenyl)-4,5-dihydro-1H-pyrazole-3-carbonyl]-amino}-piperidinium,

Methanesulfonate1-{[5(R)-(4-chloro-phenyl)-1-(2,4-dichloro-phenyl)-4,5-dihydro-1 H-pyrazole-3-carbonyl]-amino}-piperidinium,

Methanesulfonate1-{[5(S)-(4-chloro-phenyl)-1-(2,4-dichloro-phenyl)-4,5-dihydro-1 H-pyrazole-3-carbonyl]-amino}-piperidinium,

1-{[5-(4-Chloro-phenyl)-1-(2,4-dichloro-phenyl)-4,5-dihydro-1H-pyrazole-3-carbonyl]-amino}-piperidinium; bromide,

1-{[5(R)-(4-Chloro-phenyl)-1-(2,4-dichloro-phenyl)-4,5-dihydro-1H-pyrazole-3-carbonyl]-amino}-piperidinium; bromide,

1-{[5(S)-(4-Chloro-phenyl)-1-(2,4-dichloro-phenyl)-4,5-dihydro-1H-pyrazole-3-carbonyl]-amino}-piperidinium; bromide,

Ethanesulfonate1-{[5-(4-chloro-phenyl)-1-(2,4-dichloro-phenyl)-4,5-dihydro-1H-pyrazole-3-carbonyl]-amino}-piperidinium,

Ethanesulfonate1-{[5(R)-(4-chloro-phenyl)-1-(2,4-dichloro-phenyl)-4,5-dihydro-1 H-pyrazole-3-carbonyl]-amino}-piperidinium,

Ethanesulfonate1-{[5(S)-(4-chloro-phenyl)-1-(2,4-dichloro-phenyl)-4,5-dihydro-1 H-pyrazole-3-carbonyl]-amino}-piperidinium,

Toluene-4-sulfonate1-{[5-(4-chloro-phenyl)-1-(2,4-dichloro-phenyl)-4,5-dihydro-1 H-pyrazole-3-carbonyl]-amino}-piperidinium,

Toluene-4-sulfonate1-{[5(R)-(4-chloro-phenyl)-1-(2,4-dichloro-phenyl)-4,5-dihydro-1H-pyrazole-3-carbonyl]-amino}-piperidinium,

Toluene-4-sulfonate1-{[5(S)-(4-chloro-phenyl)-1-(2,4-dichloro-phenyl)-4,5-dihydro-1H-pyrazole-3-carbonyl]-amino}-piperidinium,

Benzenesulfonate1-{[5-(4-chloro-phenyl)-1-(2,4-dichloro-phenyl)-4,5-dihydro-1H-pyrazole-3-carbonyl]-amino}-piperidinium,

Benzenesulfonate1-{[5(R)-(4-chloro-phenyl)-1-(2,4-dichloro-phenyl)-4,5-dihydro-1 H-pyrazole-3-carbonyl]-amino}-piperidinium,

Benzenesulfonate1-{[5(S)-(4-chloro-phenyl)-1-(2,4-dichloro-phenyl)-4,5-dihydro-1 H-pyrazole-3-carbonyl]-amino}-piperidinium,

2,5-Dihydroxy-benzenesulfonate1-{[5-(4-chloro-phenyl)-1-(2,4-dichloro-phenyl)-4,5-dihydro-1H-pyrazole-3-carbonyl]-amino}-piperidinium,

2,5-Dihydroxy-benzenesulfonate1-{[5(R)-(4-chloro-phenyl)-1-(2,4-dichlorophenyl)-4,5-dihydro-1H-pyrazole-3-carbonyl]-amino}-piperidinium,

2,5-Dihydroxy-benzenesulfonate1-{[5(S)-(4-chloro-phenyl)-1-(2,4-dichlorophenyl)-4,5-dihydro-1H-pyrazole-3-carbonyl]-amino}-piperidinium,

Naphthalene-2-sulfonate1-{[5-(4-chloro-phenyl)-1-(2,4-dichloro-phenyl)-4,5-dihydro-1H-pyrazole-3-carbonyl]-amino}-piperidinium,

Naphthalene-2-sulfonate1-{[5(R)-(4-chloro-phenyl)-1-(2,4-dichloro-phenyl)-4,5-dihydro-1H-pyrazole-3-carbonyl]-amino}-piperidinium,

Naphthalene-2-sulfonate1-{[5(S)-(4-chloro-phenyl)-1-(2,4-dichloro-phenyl)-4,5-dihydro-1H-pyrazole-3-carbonyl]-amino}-piperidinium,

5-Sulfo-naphthalene-1-sulfonate1-{[5-(4-chloro-phenyl)-1-(2,4-dichloro-phenyl)-4,5-dihydro-1H-pyrazole-3-carbonyl]-amino}-piperidinium,

5-Sulfo-naphthalene-1-sulfonate1-{[5(R)-(4-chloro-phenyl)-1-(2,4-dichlorophenyl)-4,5-dihydro-1H-pyrazole-3-carbonyl]-amino}-piperidinium,

5-Sulfo-naphthalene-1-sulfonate1-{[5(S)-(4-chloro-phenyl)-1-(2,4-dichlorophenyl)-4,5-dihydro-1H-pyrazole-3-carbonyl]-amino}-piperidinium,

Cyclamate1-{[544-chloro-phenyl)-1-(2,4-dichloro-phenyl)-4,5-dihydro-1H-pyrazole-3-carbonyl]-amino}-piperidinium,

Cyclamate1-{[5(R)-(4-chloro-phenyl)-1-(2,4-dichloro-phenyl)-4,5-dihydro-1H-pyrazole-3-carbonyl]-amino}-piperidinium,

Cyclamate1-{[5(S)-(4-chloro-phenyl)-1-(2,4-dichloro-phenyl)-4,5-dihydro-1H-pyrazole-3-carbonyl]-amino}-piperidinium,

Dodecane-1-sulfonate1-{[5-(4-chloro-phenyl)-1-(2,4-dichloro-phenyl)-4,5-dihydro-1 H-pyrazole-3-carbonyl]-amino}-piperidinium,

Dodecane-1-sulfonate1-{[5(R)-(4-chloro-phenyl)-1-(2,4-dichloro-phenyl)-4,5-dihydro-1H-pyrazole-3-carbonyl]-amino}-piperidinium,

Dodecane-1-sulfonate1-{[5(S)-(4-chloro-phenyl)-1-(2,4-dichloro-phenyl)-4,5-dihydro-1H-pyrazole-3-carbonyl]-amino}-piperidinium,

Thiocyanate1-{[5-(4-chloro-phenyl)-1-(2,4-dichloro-phenyl)-4,5-dihydro-1H-pyrazole-3-carbonyl]-amino}-piperidinium,

Thiocyanate1-{[5(R)-(4-chloro-phenyl)-1-(2,4-dichloro-phenyl)-4,5-dihydro-1H-pyrazole-3-carbonyl]-amino}-piperidinium,

Thiocyanate1-{[5(S)-(4-chloro-phenyl)-1-(2,4-dichloro-phenyl)-4,5-dihydro-1H-pyrazole-3-carbonyl]-amino}-piperidinium,

1-{[5-(4-Chloro-phenyl)-1-(2,4-dichloro-phenyl)-4,5-dihydro-1H-pyrazole-3-carbonyl]-amino}-piperidinium; dihydrogen phosphate,

1-{[5(R)-(4-Chloro-phenyl)-1-(2,4-dichtoro-phenyl)-4,5-dihydro-1H-pyrazole-3-carbonyl]-amino}-piperidinium; dihydrogen phosphate,

1-{[5(S)-(4-Chloro-phenyl)-1-(2,4-dichloro-phenyl)-4,5-dihydro-1H-pyrazole-3-carbonyl]-amino}-piperidinium; dihydrogen phosphate,

3-Carboxy-acrylate1-{[5-(4-chloro-phenyl)-1-(2,4-dichloro-phenyl)-4,5-dihydro-1H-pyrazole-3-carbonyl]-amino}-piperidinium,

3-Carboxy-acrylate1-{[5(R)-(4-chloro-phenyl)-1-(2,4-dichloro-phenyl)-4,5-dihydro-1 H-pyrazole-3-carbonyl]-amino}-piperidinium,

3-Carboxy-acrylate1-{[5(S)-(4-chloro-phenyl)-1-(2,4-dichloro-phenyl)-4,5-dihydro-1 H-pyrazole-3-carbonyl]-amino}-piperidinium,

(7,7-Dimethyl-2-oxo-bicyclo[2.2.1]hept-1-yl)-methanesulfonate1-{[5-(4-chlorophenyl)-1-(2,4-dichloro-phenyl)-4,5-dihydro-1H-pyrazole-3-carbonyl]-amino}-piperidinium,

(7,7-Dimethyl-2-oxo-bicyclo[2.2.1]hept-1-yl)-methanesulfonate1-{[5(R)-(4-chlorophenyl)-1-(2,4-dichloro-phenyl)-

4,5-dihydro-1H-pyrazole-3-carbonyl]-amino}-piperidinium,

(7,7-Dimethyl-2-oxo-bicyclo[2.2.1]hept-1-yl)-methanesulfonate1-{[5(S)-(4-chlorophenyl)-1-(2,4-dichloro-phenyl)-4,5-dihydro-1H-pyrazole-3-carbonyl]-amino}-piperidinium,
optionally in the form of a corresponding N-oxide, or a corresponding solvate.

In another aspect the present invention also provides a process for the preparation of a salt of substituted pyrazoline compounds of general formula I, Ia or Ib given above, according to which at least one benzaldehyde compound of general formula II

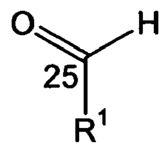

(II)

wherein $R^1$ has the meaning given above, is reacted with a pyruvate compound of general formula (III)

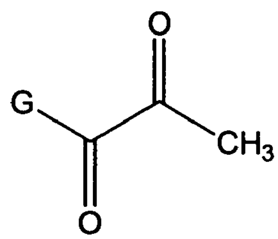

(III),

wherein G represents an OR group with R being a branched or unbranched $C_{1-6}$ alkyl radical, preferably an ethyl radical, or G represents an $O^-K$ group with K being a cation, preferably a monovalent cation, more preferably an alkali metal cation, even more preferably a sodium cation, to yield a compound of general formula (IV)

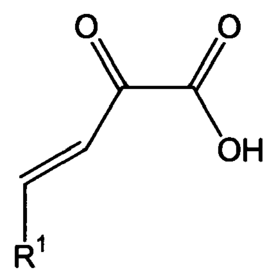

(IV)

wherein $R^1$ has the meaning given above, which is optionally isolated and/or optionally purified, and which is reacted with an optionally substituted phenyl hydrazine of general formula (V)

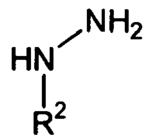

(V)

or a corresponding salt thereof, wherein $R^2$ has the meaning given above, under an inert atmosphere, to yield a compound of general formula (VI)

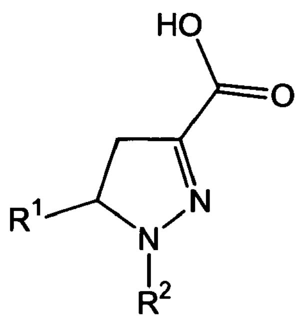

(VI)

wherein $R^1$ and $R^2$ have the meaning as given above, which is optionally isolated and/or optionally purified, and optionally transferred under inert atmosphere to a compound of general formula (VII)

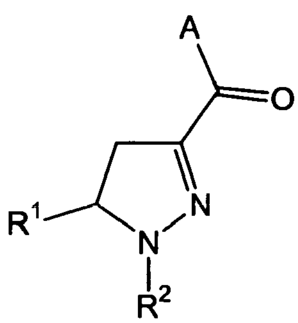

(VII)

wherein the substituents $R^1$ and $R^2$ have the meaning given above and A represents a leaving group, via the reaction with an activating agent, said compound being optionally isolated and/or optionally purified, and at least one compound of general formula (VI) is reacted with a compound of general formula $R^3H$, wherein $R^3$ represents an —$NR^4R^5$-moiety, wherein $R^4$ and $R^5$ have the meaning given above, to yield a substituted pyrazoline compound of general formula I,

wherein $R^3$ represents an —$NR^4R^5$-moiety,
and/or at least one compound of general formula (VII) is reacted with a compound of the general formula $R^3H$, in which $R^3$ has the meaning given above to yield a compound of general formula (I) given above, which is optionally isolated and/or optionally purified,
wherein the enantiomers according to general formula Ia or Ib of a compound of general formula I are optionally formed by either separating the enantiomers chromatographically or by reacting the compound of general formula I with a chiral base,
wherein the compound of general formula I or Ia or Ib is reacted with an acid with a pka ≤ 2.0 to form a salt, according to one or more of claims 1-10, which is optionally isolated and/or optionally purified .

The first part of the inventive process is also illustrated in scheme I given below:

**Scheme I:**

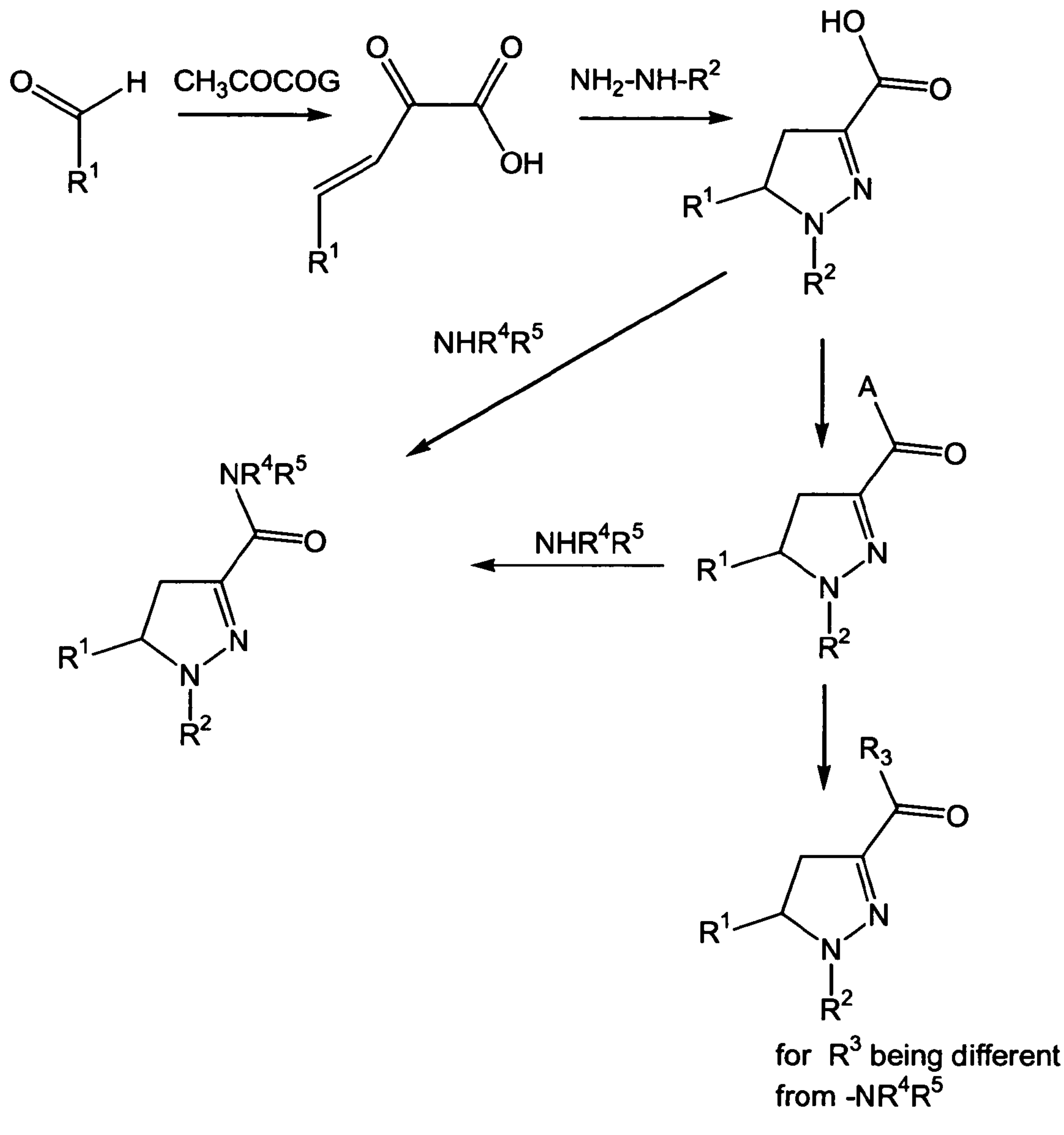


The reaction of the benzaldehyde compound of general formula II with a pyruvate compound of general formula III is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate

compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10°C to the boiling point of the reaction medium. Suitable reaction times may vary for example from several minutes to several hours.

Also preferred the reaction of the benzaldehyde compound of general formula II with a pyruvate compound of general formula III is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (IV) with an optionally substituted phenyl hydrazin of general formula (V) is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VI) may be activated for further reactions by the introduction of a suitable leaving group according to conventional methods well known to those skilled in the art. Preferably the compounds of general formula (VI) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VII) is an acid chloride, it is preferably prepared by reaction of the corresponding acid of general formula (VI) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0° C to the boiling point of the solvent and reaction times from several minutes to several hours.

If said activated compound of general formula (VII) is a mixed anhydride, said anhydride may preferably be prepared, for example, by reaction of the corresponding acid of general formula (VI) with ethyl chloroformiate in the presence of a base such as triethylamine or pyridine, in a suitable solvent.

The reaction of general formula (VII) with a compound of general formula $HR^3$ to yield compounds of general general I, wherein $R^3$ represents an $-NR^4R^5$ moiety is preferably carried out in presence of a base such as triethylamine in a reaction medium such as methylenchloride. The temperature is preferably in the range from 0°C to the boiling point of the reaction medium. The reaction time may vary over a broad range, e.g. from several hours to several days.

The reaction of general formula (VII) with a compound of general formula $HR^3$ to yield compounds of general formula I, wherein $R^3$ represents a saturated or unsaturated, optionally at least mono-substituted, optionally at least one heteroatom as ring member containing cycloaliphatic group, which may be condensed with an optionally at least mono-substituted mono- or polycydic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341(7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofurane or similar. The temperature is preferably in teh range from 0°C to the boiling point of the reaction medium, more preferably from 15 to 25 °C. The reaction time may vary over a broad range, e.g. from several minutes to several hours.

The afore mentioned reactions involving the synthesis of the 4,5-dihydropyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula I themselves are obtained in form of a mixture of stereoisomers, particularly enantiomers of general formula Ia or Ib or diastereomers, said mixtures may be separated by standard procedures known to those skilled in the art, e.g. chromatographic methods or fractionalized crystallization with chiral reagents. It is also possible to obtain pure stereoisomers via stereoselective synthesis, especially using chiral bases like brucine, quinine, (-)-Cinchonidine, (+)-Cinchonine or R-(+)-1-Phenylethylamine.

In a further aspect the present invention also provides a process for the preparation of salts of substituted pyrazoline compounds of general formula I Ia or Ib, wherein at least one compound of general formula I, Ia or Ib having at least one basic group is reacted with at least one acid, preferably in the presence of a suitable reaction medium. Suitable reaction media include, for example, any of the ones given above. Suitable acids are inorganic and organic acids with a pka < 3.0, preferably < 2.0, more preferably < 1.5, yet more preferably < 1.0, most preferably < 0.7. Preferably the acid is selected from naphthalene-1 ,5-disulfonic acid, ethanesulfonic acid, thiocyanic acid, 2-naphthalenesulfonic acid or benzenesulfonic acid.

Other acids than can be also used are Hydrobromic acid, Hydrochloric acid, Sulfuric acid, Ethane-1,2-disulfonic acid, Cyclohexylsulfamic acid, p-Toluenesulfonic acid, Methanesulfonic acid, Dodecylsulfuric acid, Benzenesulfonic acid or Nitric acid.

Phosophoric acid, Fumaric acid, 2,5-Dihydroxybenzenesulfonic acid, Aspartic acid, Camphor-10-sulfonic acid, Glutamic acid

Preferably the resulting salts are pharmaceutically acceptable salts.

Solvates, preferably hydrates, of the substituted pyrazoline compounds of general formula (I), of corresponding stereoisomers, of corresponding N-oxides or of corresponding salts thereof may also be obtained by standard procedures known to those skilled in the art.

Substituted pyrazoline compounds of general formula I, Ia or Ib which comprise nitrogen-atom containing saturated, unsaturated or aromatic rings may also be obtained in the form of their N-oxides by methods well known to those skilled in the art.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002) . The respective description is hereby incorporated by reference and forms part of the disclosure.

The purification and isolation of the inventive salts of the substituted pyrazoline compounds of general formula I, Ia or Ib, of a corresponding stereoisomer, or N-oxide, or solvate or any intermediate thereof may, if required, be carried out by conventional methods known to those skilled in the art, e.g. chromatographic methods or recrystallization.

The salts of the substituted pyrazoline compounds of general formula I, Ia or Ib given below, their stereoisomers, corresponding N-oxides, and corresponding solvates are toxicologically acceptable and are therefore suitable as pharmaceutical active substances for the preparation of medicaments.

It has been found that the salts of substituted pyrazoline compounds of general formula I, Ia or Ib given below, N-oxides thereof and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these pyrazoline compounds show relatively weak Herg channel affinity, thus a low risk of prolongation of the QT-interval is to be expected for these compounds.

In summary, the inventively used pyrazoline compounds are distinguished by a broad spectrum of beneficial effects, while at the same time showing relatively little undesired effects, i.e. effects which do not positively contribute to or even interfere with the well being of the patient.

Thus, an other aspect of the present invention relates to a medicament comprising at least one salt of a substituted pyrazoline compound of general formula I,

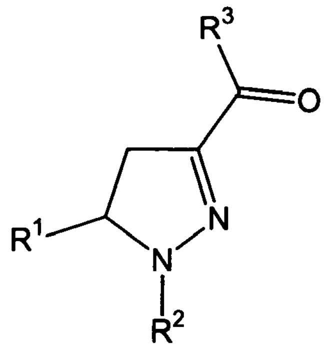

I

wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cydoaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an - $NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cyGoaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an —$SO_2$-$R^6$-moiety, or an -$NR^7R^8$-moiety, with the proviso that $R^4$ and $R^5$ do not identically represent hydrogen,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof,
with an acid with a $pk_a \leq 3.0$,
optionally in form of a corresponding solvate thereof,
and optionally one or more pharmaceutically acceptable excipients.

Preferably the acids are inorganic and organic acids with a pka < 3.0, preferably < 2.0, more preferably < 1.50, yet more preferably < 1.0, most preferably < 0.7. Preferably the acid is selected from naphthalene-1,5-disulfonic acid,

ethanesulfonic acid, thiocyanic acid, 2-naphthalenesulfonic acid or benzenesulfonic acid.
Other acids than can be also used are Hydrobromic acid, Hydrochloric acid, Sulfuric acid, Ethane-1,2-disulfonic acid, Cyclohexylsulfamic acid, p-Toluenesulfonic acid, Methanesulfonic acid, Dodecylsulfuric acid, Benzenesulfonic acid or Nitric acid.

Preferably the resulting salts are pharmaceutically acceptable salts.

A mono- or polycyclic ring-system according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.
The term "condensed" according to the present invention means that a ring or ring-system is attached to another ring or ring-system, whereby the terms "annulated" or "annelated" are also used by those skilled in the art to designate this kind of attachment.

If one or more of the residues $R^3$-$R^8$ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, oxo, amino, carboxy, amido, cyano, nitro, $-SO_2NH_2$, -CO-$C_{1-4}$-alkyl, -SO-$C_{1-4}$-alkyl, $-SO_2$-$C_{1-4}$-alkyl, -NH-$SO_2$-$C_{1-4}$-alkyl, wherein the $C_{1-4}$-alkyl may in each case be branched or unbranched, and a phenyl group, more preferably be selected from the group consisting of hydroxy, F, Cl, Br, methyl, ethyl, methoxy, ethoxy, oxo, $CF_3$ and a phenyl group.

If one or more of the residues $R^3$-$R^8$ represents or comprises a cydoaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteroatoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cydohexenyl, Cycloheptenyl, Cyclooctenyl, Pyrrolidinyl, Piperidinyl, Piperazinyl, homo-Piperazinyl and Morpholinyl.

If one or more of the residues $R^3$-$R^8$ comprises a mono- or polycydic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, amino, carboxy, oxo, amido, cyano, nitro, $-SO_2NH_2$, -CO-$C_{1-4}$-alkyl, -SO-$C_{1-4}$-alkyl, $-SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, $CF_3$, oxo and a phenyl group.

If one or more of the residues $R^1$-$R^8$ represents or comprises an aryl group, induding a phenyl group, which is substituted by one or more, e.g. 1, 2, 3, 4 or 5 substituents, unless defined otherwise, each of the substituents may be independently selected from the group consisting of a halogen atom (F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a nitro group, a carboxy group, a -CO-O-$C_{1-6}$-alkyl group, a —CO-$NR^AR^B$-moiety, a -CO-NH-$NR^CR^D$-moiety, an -SH, an - S-$C_{1-6}$-alkyl group, an -SO-$C_{1-6}$-alkyl group, an $-SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, an —$NH_2$-moiety, an NHR'-moiety or an NR'R"-moiety, wherein R' and R" independently represent a linear or branched $C_{1-6}$-alkyl group, a $C_{1-6}$-alkyl group substituted by one or more hydroxy groups and a —$C_{1-6}$-alkylene-$NR^ER^F$ group, whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a — CO-O- $C_{1-6}$-alkyl group, a -CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a —CO-$NH_2$ group and/or which may contain at least one further heteroatom selected from the group consisting of nitrogen, oxygen and sulphur as a ring member, and
wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues $R^3$-$R^8$ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a carboxy group, a -CO-O-$C_{1-6}$-alkyl group, a -CO-$NR^AR^B$- moiety, a -CO-NH-$NR^CR^D$-moiety, an -S-$C_{1-6}$-alkyl group, an -SO-$C_{1-6}$-alkyl group, an - $SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, a $C_{1-6}$-alkyl group substituted by one or more hydroxy groups and a -$C_{1-6}$-alkylene-$NR^ER^F$ group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a — CO-O- $C_{1-6}$-alkyl group, a -CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a —CO-$NH_2$ group and/or which may contain at least one further heteroatom selected from the group consisting of nitrogen, oxygen and sulphur as a ring member, and
wherein $R^E$, $R^F$, identical or different, represent hydrogen or a $C_{1-6}$-alkyl group, or $R^E$ and $R^F$ together with the bridging nitrogen atom form a saturated, mono- or bicydic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo [2,1-b]thiazolyl, triazolyl and pyrazolyl.

If one or more of the residues $R^4$-$R^8$ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-4}$-alkoxy, branched or unbranched $C_{1-4}$-perfluoroalkoxy, branched or unbranched $C_{1-4}$-perfluoroalkyl, amino, carboxy, amido, cyano, nitro, -$SO_2NH_2$, - CO-$C_{1-4}$-alkyl, -SO-$C_{1-4}$-alkyl, -$SO_2$-$C_{1-4}$-alkyl, -NH-$SO_2$-$C_{1-4}$-alkyl, wherein the $C_{1-4}$-alkyl may in each case be branched or unbranched, and a phenyl group, more preferably be selected from the group consisting of hydroxy, F, Cl, Br, methoxy, ethoxy, $CF_3$ and a phenyl group.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues $R^4$-$R^8$ represents or comprises a linear or branched alkylene group, said alkylene group may preferably be selected from the group consisting of —methylene -($CH_2$)-, ethylene -($CH_2$-$CH_2$)-, n-propylene -($CH_2$-$CH_2$-$CH_2$)- or iso-proylene —(-$C(CH_3)_2$)-.

Particularly preferred is a medicament comprising at least one salt of a compound of general formula I

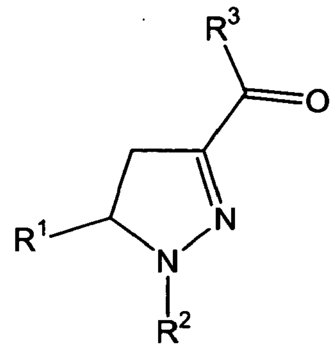

I

wherein

$R^1$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and —(C=O)-NR'R", whereby R' and R" at each ocurrence independently represent a linear or branched $C_{1-6}$ alkyl group,

$R^2$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and —(C=O)-NR'R", whereby R' and R" at each ocurrence independently represent a linear or branched $C_{1-6}$ alkyl group,

$R^3$ represents a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl, linear or branched $C_{1-6}$ alkoxy, OH, F, Cl, Br, I, CN, $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, or $R^3$ represents an -$NR^4R^5$-moiety,

$R^4$ represents a hydrogen atom or a linear or branched $C_{1-6}$-alkyl group,

$R^5$ represents a linear or branched $C_{1-6}$ alkyl group; an -$SO_2$-$R^6$-moiety; a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl group, a linear or branched $C_{1-6}$ alkoxy group, OH, F, Cl, Br, I, CN $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, and

$R^6$ represents a phenyl group, which is optionally substituted with 1, 2, 3, 4 or 5 substituents independently selected from the group consisting of a linear or branched $C_{1-6}$-alkyl group, a linear or branched $C_{1-6}$-alkoxy group, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and R" at each ocurrence independently represent a linear or branched $C_{1-6}$ alkyl group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Furthermore a medicament is particularly preferred, which comprises at least one salt of a compound of general formula I, Ia or Ib given below,

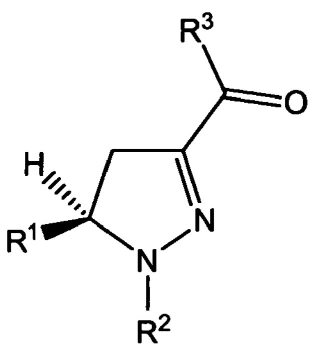
Ia

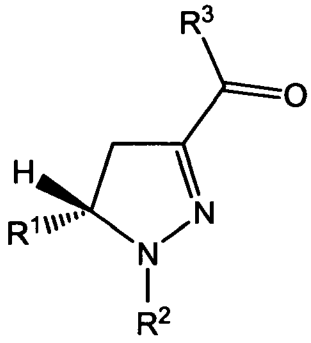
Ib

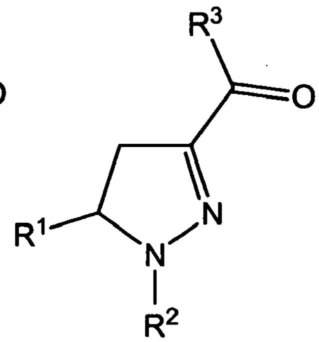
I

wherein

$R^1$ represents a phenyl ring, which is mono-substituted with a halogen atom, preferably a chlorine atom, in its 4-position,

$R^2$ represents a phenyl ring, which is di-substituted with two halogen atoms, preferably chlorine atoms, in its 2- and 4-position,

$R^3$ represents a pyrrolidinyl group, a piperidinyl group, a piperazinyl group, a homo-piperazinyl group, a morpholinyl group, or an —$NR^4R^5$-moiety,

$R^4$ represents a hydrogen atom or a linear or branched $C_{1-6}$-alkyl group,

$R^5$ represents a linear or branched $C_{1-6}$ alkyl group, a pyrrolidinyl group, a piperidinyl group, a piperazinyl group, a homo-piperazinyl group, a morpholinyl group, a triazolyl group, whereby each of the heterocyclic rings may be substituted with one or more, identical or different, $C_{1-6}$-alkyl groups, or an -$SO_2$-$R^6$-moiety, and

$R^6$ represents a phenyl group, which is optionally substituted with one or more $C_{1-6}$ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof or a corresponding solvate thereof.

Most particularly preferred is a medicament comprising at least one salt of a substituted pyrazoline compound selected from the group consisting of:

N-piperidinyl-5-(4-chloro-phenyl)-1-(2,4-dichlorophenyl)-4,5-dihydro-1H-pyrazole-3-carboxamide,

(rac)-N-piperidinyl-5-(4-chloro-phenyl)-1-(2,4-dichlorophenyl)-4,5-dihydro-1H-pyrazole-3-carboxamide,

(R)-N-piperidinyl-5-(4-chloro-phenyl)-1-(2,4-dichlorophenyl)-4,5-dihydro-1H-pyrazole-3-carboxamide,

(S)-N-piperidinyl-5-(4-chloro-phenyl)-1-(2,4-dichlorophenyl)-4,5-dihydro-1H-pyrazole-3-carboxamide,

5-(4-Chloro-phenyl)-1-(2,4-dichloro-phenyl)-4,5-dihydro-1H-pyrazole-3-carboxylic acid-[1,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone,

N-[5-(4-Chloro-phenyl)-1-(2,4-dichlorophenyl)-4,5-dihydro-1H-pyrazole-3-carbonyl]-4-methylphenylsufonamide,

1-{[5-(4-Chloro-phenyl)-1-(2,4-dichloro-phenyl)-4,5-dihydro-1H-pyrazole-3-carbonyl]-amino}-piperidinium; chloride,

1-{[5(R)-(4-Chloro-phenyl)-1-(2,4-dichloro-phenyl)-4,5-dihydro-1H-pyrazole-3-carbonyl]-amino}-piperidinium; chloride,

1-{[5(S)-(4-Chloro-phenyl)-1-(2,4-dichloro-phenyl)-4,5-dihydro-1H-pyrazole-3-carbonyl]-amino}-piperidinium; chloride

1-{[5-(4-Chloro-phenyl)-1-(2,4-dichloro-phenyl)-4,5-dihydro-1H-pyrazole-3-carbonyl]-amino}-piperidinium; nitrate,

1-{[5(R)-(4-Chloro-phenyl)-1-(2,4-dichloro-phenyl)-4,5-dihydro-1H-pyrazole-3-carbonyl]-amino}-piperidinium; nitrate,

1-{[5(S)-(4-Chloro-phenyl)-1-(2,4-dichloro-phenyl)-4,5-dihydro-1H-pyrazole-3-carbonyl]-amino}-piperidinium; nitrate,

1-{[5-(4-Chloro-phenyl)-1-(2,4-dichloro-phenyl)-4,5-dihydro-1H-pyrazole-3-carbonyl]-amino}-piperidinium; hydrogen sulfate,

1-{[5(R)-(4-Chloro-phenyl)-1-(2,4-dichloro-phenyl)-4,5-dihydro-1H-pyrazole-3-carbonyl]-amino}-piperidinium; hydrogen sulfate,

1-{[5(S)-(4-Chloro-phenyl)-1-(2,4-dichloro-phenyl)-4,5-dihydro-1H-pyrazole-3-carbonyl]-amino}-piperidinium; hydrogen sulfate,

Methanesulfonate1-{[5-(4-chloro-phenyl)-1-(2,4-dichloro-phenyl)-4,5-dihydro-1H-pyrazole-3-carbonyl]-amino}-piperidinium,

Methanesulfonate1-{[5(R)-(4-chloro-phenyl)-1-(2,4-dichloro-phenyl)-4,5-dihydro-1 H-pyrazole-3-carbonyl]-amino}-piperidinium,

Methanesulfonate1-{[5(S)-(4-chloro-phenyl)-1-(2,4-dichloro-phenyl)-4,5-dihydro-1 H-pyrazole-3-carbonyl]-amino}- piperidinium,

1-{[5-(4-Chloro-phenyl)-1-(2,4-dichloro-phenyl)-4,5-dihydro-1H-pyrazole-3-carbonyl]-amino}-piperidinium; bromide,

1-{[5(R)-(4-Chloro-phenyl)-1-(2,4-dichloro-phenyl)-4,5-dihydro-1H-pyrazole-3-carbonyl]-amino}-piperidinium; bromide,

1-{[5(S)-(4-Chloro-phenyl)-1-(2,4-dichloro-phenyl)-4,5-dihydro-1H-pyrazole-3-carbonyl]-amino}-piperidinium; bromide,

Ethanesulfonate1-{[5-(4-chtoro-phenyl)-1-(2,4-dichloro-phenyl)-4,5-dihydro-1H-pyrazole-3-carbonyl]-amino}-piperidinium,

Ethanesulfonate1-{[5(R)-(4-chloro-phenyl)-1-(2,4-dichloro-phenyl)-4,5-dihydro-1 H-pyrazole-3-carbonyl]-amino}-piperidinium,

Ethanesulfonate 1-{[5(S)-(4-chloro-phenyl)-1-(2,4-dichloro-phenyl)-4,5-dihydro-1 H-pyrazole-3-carbonyl]-amino}-piperidinium,

Toluene-4-sulfonate1-{[5-(4-chloro-phenyl)-1-(2,4-dichloro-phenyl)-4,5-dihydro-1 H-pyrazole-3-carbonyl]-amino}-piperidinium,

Toluene-4-sulfonate1-{[5(R)-(4-chloro-phenyl)-1-(2,4-dichloro-phenyl)-4,5-dihydro-1H-pyrazole-3-carbonyl]-amino}-piperidinium,

Toluene-4-sulfonate1-{[5(S)-(4-chloro-phenyl)-1-(2,4-dichloro-phenyl)-4,5-dihydro-1H-pyrazole-3-carbonyl]-amino}-piperidinium,

Benzenesulfonate1-{[5-(4-chloro-phenyl)-1-(2,4-dichloro-phenyl)-4,5-dihydro-1H-pyrazole-3-carbonyl]-amino}-piperidinium,

Benzenesulfonate1-{[5(R)-(4-chloro-phenyl)-1-(2,4-dichloro-phenyl)-4,5-dihydro-1 H-pyrazole-3-carbonyl]-amino}-piperidinium,

Benzenesulfonate1-{[5(S)-(4-chloro-phenyl)-1-(2,4-dichloro-phenyl)-4,5-dihydro-1 H-pyrazole-3-carbonyl]-amino}-piperidinium,

2,5-Dihydroxy-benzenesulfonate1-{[5-(4-chloro-phenyl)-1-(2,4-dichloro-phenyl)-4,5-dihydro-1H-pyrazole-3-carbonyl]-amino}-piperidinium,

2,5-Dihydroxy-benzenesulfonate1-{[5(R)-(4-chloro-phenyl)-1-(2,4-dichlorophenyl)-4,5-dihydro-1H-pyrazole-3-carbonyl]-amino}-piperidinium,

2,5-Dihydroxy-benzenesulfonate1-{[5(S)-(4-chloro-phenyl)-1-(2,4-dichlorophenyl)-4,5-dihydro-1H-pyrazole-3-carbonyl]-amino}-piperidinium,

Naphthalene-2-sulfonate1-{[5-(4-chloro-phenyl)-1-(2,4-dichloro-phenyl)-4,5-dihydro-1H-pyrazole-3-carbonyl]-amino}-piperidinium,

Naphthalene-2-sulfonate1-{[5(R)-(4-chloro-phenyl)-1-(2,4-dichloro-phenyl)-4,5-dihydro-1H-pyrazole-3-carbonyl]-amino}-piperidinium,

Naphthalene-2-sulfonate1-{[5(S)-(4-chloro-phenyl)-1-(2,4-dichloro-phenyl)-4,5-dihydro-1H-pyrazole-3-carbonyl]-amino}-piperidinium,

5-Sulfo-naphthalene-1-sulfonate1-{[5-(4-chloro-phenyl)-1-(2,4-dichloro-phenyl)-4,5-dihydro-1H-pyrazole-3-carbonyl]-amino}-piperidinium,

5-Sulfo-naphthalene-1-sulfonate1-{[5(R)-(4-chloro-phenyl)-1-(2,4-dichlorophenyl)-4,5-dihydro-1H-pyrazole-3-carbonyl]-amino}-piperidinium,

5-Sulfo-naphthalene-1-sulfonate1-{[5(S)-(4-chloro-phenyl)-1-(2,4-dichlorophenyl)-4,5-dihydro-1H-pyrazole-3-carbonyl]-amino}-piperidinium,

Cyclamate1-{[5-(4-chloro-phenyl)-1-(2,4-dichloro-phenyl)-4,5-dihydro-1H-pyrazole-3-carbonyl]-amino}-piperidinium,

Cyclamate1-{[5(R)-(4-chloro-phenyl)-1-(2,4-dichloro-phenyl)-4,5-dihydro-1H-pyrazole-3-carbonyl]-amino}-piperidinium,

Cyclamate1-{[5(S)-(4-chloro-phenyl)-1-(2,4-dichloro-phenyl)-4,5-dihydro-1H-pyrazole-3-carbonyl]-amino}-piperidinium,

Dodecane-1-sulfonate1-{[5-(4-chloro-phenyl)-1-(2,4-dichloro-phenyl)-4,5-dihydro-1 H-pyrazole-3-carbonyl]-amino}-piperidinium,

Dodecane-1-sulfonate1-{[5(R)-(4-chloro-phenyl)-1-(2,4-dichloro-phenyl)-4,5-dihydro-1H-pyrazole-3-carbonyl]-amino}-piperidinium,

Dodecane-1-sulfonate1-{[5(S)-(4-chloro-phenyl)-1-(2,4-dichloro-phenyl)-4,5-dihydro-1H-pyrazole-3-carbonyl]-amino}-piperidinium,

Thiocyanate1-{[5-(4-chloro-phenyl)-1-(2,4-dichloro-phenyl)-4,5-dihydro-1H-pyrazole-3-carbonyl]-amino}-piperidinium,

Thiocyanate1-{[5(R)-(4-chloro-phenyl)-1-(2,4-dichloro-phenyl)-4,5-dihydro-1H-pyrazole-3-carbonyl]-amino}-piperidinium,

Thiocyanate1-{[5(S)-(4-chloro-phenyl)-1-(2,4-dichloro-phenyl)-4,5-dihydro-1H-pyrazole-3-carbonyl]-amino}-piperidinium,

1-{[5-(4-Chloro-phenyl)-1-(2,4-dichloro-phenyl)-4,5-dihydro-1H-pyrazole-3-carbonyl]-amino}-piperidinium; dihydrogen phosphate,

1-{[5(R)-(4-Chloro-phenyl)-1-(2,4-dichloro-phenyl)-4,5-dihydro-1H-pyrazole-3-carbonyl]-amino}-piperidinium; dihydrogen phosphate,

1-{[5(S)-(4-Chloro-phenyl)-1-(2,4-dichloro-phenyl)-4,5-dihydro-1H-pyrazole-3-carbonyl]-amino}-piperidinium; dihydrogen phosphate,

3-Carboxy-acrylate1-{[5-(4-chloro-phenyl)-1-(2,4-dichloro-phenyl)-4,5-dihydro-1H-pyrazole-3-carbonyl]-amino}-piperidinium,

3-Carboxy-acrylate1-{[5(R)-(4-chloro-phenyl)-1-(2,4-dichloro-phenyl)-4,5-dihydro-1 H-pyrazole-3-carbonyl]-amino}-piperidinium,

3-Carboxy-acrylate1-{[5(S)-(4-chloro-phenyl)-1-(2,4-dichloro-phenyl)-4,5-dihydro-1 H-pyrazole-3-carbonyl]-amino}-piperidinium,

(7,7-Dimethyl-2-oxo-bicyclo[2.2.1]hept-1-yl)-methanesulfonate1-{[5-(4-chlorophenyl)-1-(2,4-dichloro-phenyl)-4,5-dihydro-1H-pyrazole-3-carbonyl]-amino}-piperidinium,

(7,7-Di methyl-2-oxo-bicyclo[2.2.1]hept-1-yl)-methanesulfonate1-{[5(R)-(4-chlorophenyl)-1-(2,4-dichloro-phenyl)-4,5-dihydro-1H-pyrazole-3-carbonyl]-amino}-piperidinium,

(7,7-Dimethyl-2-oxo-bicyclo[2.2.1]hept-1-yl)-methanesulfonate1-{[5(S)-(4-chloro-phenyl)-1-(2,4-dichloro-phenyl)-4,5-dihydro-1H-pyrazole-3-carbonyl]-amino}-piperidinium,
optionally in the form of a corresponding N-oxide, or a corresponding solvate.

The inventive medicament may preferably also comprise any of the inventive salts of a pyrazoline compounds or combinations of at least two of these pyrazoline compounds given above with at<least one being in form of a salt according to the invention.

Said medicament may also comprise any combination of one or more of the substituted pyrazoline compounds of general formula I given above, stereoisomers thereof, corresponding N-oxides thereof, physiologically acceptable salts thereof or physiologically acceptable solvates thereof, with one salt according top the invention.

Preferably said medicament is suitable for the modulation (regulation) of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferably said medicament is suitable for the prophylaxis and/or treatment of psychosis.

Also particularly preferably said medicament is suitable for the prophylaxis and/or treatment of food intake disorders, preferably bulimia, anorexia, cachexia, obesity and/or type II diabetus mellitus (non-insuline dependent diabetes mellitus), more preferably obesity. The inventive medicament also seems to be active in the prophylaxis and/or treatment of appetency disorders, e.g. the pyrazoline compounds of general formula I also reduce the desire for sweets.

Also particularly preferably said medicament is suitable for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said medicament is suitable for the prophylaxis and/or treatment of alcohol abuse and/or

alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments and/or drugs, which are frequently the subject of misuse include opioids, barbiturates, cannabis, cocaine, amphetamines, phencydidine, hallucinogens and benzodiazepines.

The medicament is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Another aspect of the present invention is the use of at least one salt of a substituted pyrazoline compound of general formula I given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferred is the use of at least one one salt of the respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof, and optionally one or more pharmaceutically acceptable excipients, for the preparation of a medicament for the prophylaxis and/or treatment of psychosis.

Also particularly preferred is the use of at least one one salt of a respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also particularly preferred is the use of at least one one salt of a respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also particularly preferred is the use of at least one one salt of a respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencydidine, hallucinogens and benzodiazepines.

Also preferred is the use of at least one one salt of a respective pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide

thereof, or a corresponding solvate thereof, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, , medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.
The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

## Pharmacological Methods

### I. In-vitro determination of affinity to CB1/CB2-Receptors

The in-vitro determination of the affinity of the inventive substituted pyrazoline compounds to $CB_1/CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

### II. In-vivo bioassay system for determination of cannabinoid activity

### Mouse tetrad model

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of

Pharmacology XXVII. Classification of Cannabinoid Receptors, Pharmacol Rev 54, 161-202, 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277, 2, 586-594, 1996. The corresponding parts of the description are hereby incorporated by reference.

**Material and Methods**

Male NMRI mice with a weight of 20-30 g (Harlan, Barcelona, Spain) are used in all of the following experiments.
Before testing in the behavioral procedures given below, mice are acclimatized to the experimental setting. Pre-Treatment control values are determined for analgesia hot plate latency (in seconds), rectal temperature, sedation and catalepsy.
In order to determine the agonistic activty of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.
In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

**Hot plate analgesia**

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.
The mice are placed on a hot plate (Harvard Analgesimeter) at 55 ± 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).
Fifteen minuts after the administration of the substance to be tested, the mice are again placed on the hot plate and the afore described procedure is repeated.
This period is called the post-treatment reading (PT).
The degree of analgesia is calculated from the formula :

$$\% \text{ MPE of Analgesia} = (PT - B) / (PC - B) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.
The chosen scoring system is

**0**: no ataxia;
**1**: doubful;
**2:** obvious calmness and quiet;
**3** pronounced ataxia;
prior to as well as after treatment.

The percentage of sedation is determined according to the formula:

$$\% \text{ of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.
The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥-2 °C are considered to represent activity.

**Catalepsy:**

Catalepsy is determined according to the method described in Alpermann H. G. et al. Pharmacological effets of Hoe 249: A new potential antidepressant", Drugs Dev. Res. 25, 267-282. 1992. The respective description is hereby incorporated by reference and forms part of the present disclosure.
The cataleptic effect of the substance to be tested is evaluated according to the duration of catalepsy, whereby the animals are placed head downwards with their kinlegs upon the top of the wooden block.

The chosen scoring system is:

**Catalepsy for:**

more than 60 seconds = 6; 50 -60 seconds = 5, 40-50 seconds = 4, 30-40 seconds = 3, 20-30 seconds = 2, 5-10 seconds = 1, and less than 5 seconds =0.
The percentage of catalepsy is determined according ot the following formula:

$$\%\ \text{Catalepsy} = \text{arithmetic mean} / 6 \times 100$$

**III. In vivo testing for antiobesic activity**

The in-vivo testing for antiobesic activity of the inventive pyrazoline compounds is carried out as described in the publication of G. Colombo et al., Appetite Suppression and Weight Loss after the Cannabinoid Antagonist SR 141716"; Life Sciences, 63 (8), 113-117, (1998). The respective part of the description is hereby incorporated by reference and forms part of the present disclosure.

**IV. In vivo testing for antidepressant activity**

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.
The present invention is illustrated below with the aid of examples. These illustrations are given solely by way of example and do not limit the general spirit of the present invention.

**Examples:**

**Example 1:**

**N-piperidinyl-5-(4-chlorophenyl)-1-(2,4-dichlorophenyl)-4,5-dihydro-1H-pyrazole-3-carboxamide**

**a) 4-(4-chlorophenyl)-2-oxo-3-butenoic acid**

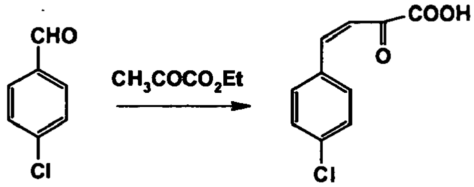


In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0˚C and an aqueous solution of NaOH (3.8 g in 45 mL water) was added dropwise keeping the temperature below or equal to 10˚C, whereby a yellow-orange colored precipitate was formed. The reaction mixture was stirred for 1 hour at 0˚C and an additional 1.5 hours at room temperature (approximately 25 ˚C). Afterwards the reaction mixture was cooled down to approximately 5˚C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).
IR (KBr, $cm^{-1}$): 3500-2500, 1719,3, 1686,5, 1603,4, 1587,8, 1081,9.
$^1H$ NMR($CDCl_3$, δ) : 7,4 (d, J=8,4Hz, 2H), 7,5 (d, J=16,1Hz, 1H), 7,6 (d, J=8,4Hz, 2H), 8,1(d, J=16,1Hz, 1H).

**b) 5-(4-chlorophenyl)-1-(2,4-dichlorophenyl)-4,5-dihydro-pyrazole-3-carboxylic acid**

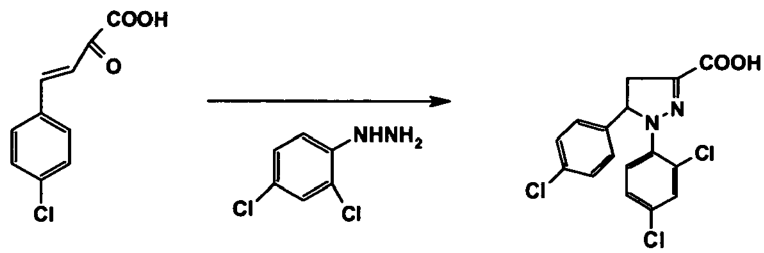

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 ˚C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).
IR (KBr, $cm^{-1}$) : 3200-2200, 1668,4, 1458, 1251,4, 1104,8.
$^1H$ NMR ($CDCl_3$, δ) : 3,3 (dd, 1 H), 3,7 (dd, 1 H), 5,9 (dd, 1 H), 7,09-7,25 (m, 7H).

**(c) 5-(4-chlorophenyl)-1-(2,4-dichlorophenyl)-4,5-dihydro-pyrazole-3-carboxylic acid chloride**

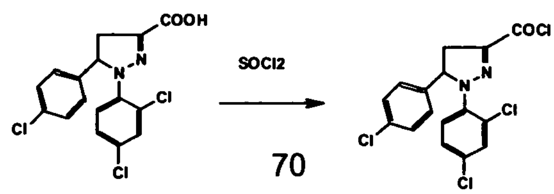


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.
IR (KBr, $cm^{-1}$) : 1732,3, 1700, 1533,3, 1478,1, 1212,9, 826,6.

**d) N-piperidinyl-5-(4-chlorophenyl)-1-(2,4-dichlorophenyl)-4,5-dihydropyrazole-3-carboxamide** [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

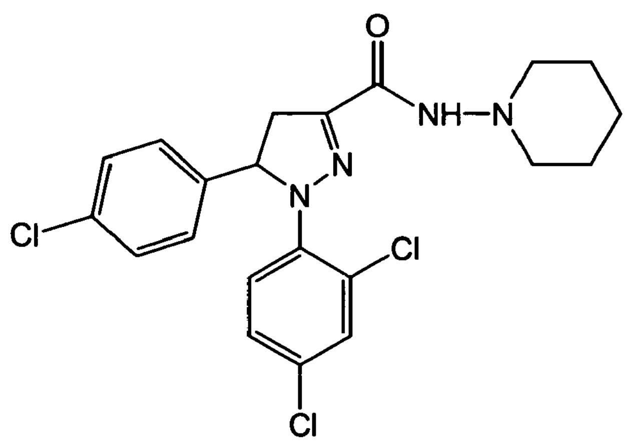

Under nitrogen atmosphere N-aminopiperidine (0.6 mL, 5.6 mmoles) and triethylamine (4 mL) were dissolved in methylene chloride (25 mL). The resulting mixture was ice-cooled down to 0˚C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 ˚C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186˚C.
IR (KBr, $cm^{-1}$): 3222,9, 2934,9, 1647,4, 1474,7, 1268,3, 815,6.
$^1H$ NMR ($CDCl_3$, $\delta$) : 1,4 (m, 2H), 1,7 (m, 4H), 2,8 (m, 4H), 3,3 (dd, J=6,1 y 18,3Hz, 1 H), 3,7 (dd, J=12,5 and 18,3 Hz, 1 H), 5,7 (dd, J=6,1 and 12,5 Hz, 1H), 7,0-7,2 (m, 6H), 7,4 (s, 1 H).

The compounds according to the following examples 2-6 have been prepared analogously to the process described in Example 1.

**Example 2:**

**5-(4-Chloro-phenyl)-1-(2,4-dichloro-phenyl)-4,5-dihydro-1H-pyrazole-3-carboxylic acid-[1,2,4]triazol-4-yl amide**

Melting point: 134-138 ˚C.
IR (KBr, $cm^{-1}$): 3448, 1686, 1477, 1243, 1091, 821.
$^1H$ NMR($CDCl_3$, δ): 3,1 (dd, J=6,2 and 17,9Hz, 1 H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1 H).

**Example 3:**

**5-(4-Chloro-phenyl)-1-(2,4-dichloro-phenyl)-4,5-dihydro-1H-pyrazole-3-carboxylic acid-(4-methyl-piperazin-1-yl)-amide hydrochloride**

Melting point: 150-155˚C.
IR (KBr, $cm^{-1}$) : 3433, 1685, 1477, 1296, 1246, 1088, 1014, 825.
$^1H$ NMR ($CDCl_3$, δ): 2,7 (d, J=4,2Hz, 3H), 3,0-3,4 (m, 9H), 3,6 (dd, J=11,9 and 17,9 Hz, 1 H), 5,8 (dd, J=5,5 and 11,9 Hz, 1 H), 7,1 (d, J=8,4Hz, 2H), 7,25 (2d, J= 8,4 and 8,7 Hz, 3H), 7,4 (d, J=2,2Hz, 1 H), 7,5 (d, J=8,7Hz, 1 H), 9,8 (s, 1 H), 11,2 (bs).

**Example 4:**

**5-(4-Chloro-phenyl)-1-(2,4-dichloro-phenyl)-4,5-dihydro-1H-pyrazole-3-carboxylic acid diethylamide**

This compound was obtained in form of an oil.
IR (film, $cm^{-1}$) : 2974, 1621, 1471, 1274, 1092, 820.
$^1H$ NMR ($CDCl_3$, δ): 1,2 (m, 6H), 3,3-3,9 (m, 6H), 5,6 (dd, J=5,8 and 11,7 Hz, 1 H), 7-7,25 (m, 7H).

**Example 5: [5-(4-Chloro-phenyl)-1-(2,4-dichloro-phenyl)-4,5-dihydro-1H-pyrazol-3-yl]-piperidin-1-yl-methanone**

Melting point: 105-110˚C.
IR (KBr, $cm^{-1}$): 2934, 1622, 1470, 1446, 1266, 1010, 817.
$^1H$ NMR ($CDCl_3$, δ): 1,7 (m, 6H), 3,4 (dd, J=5,7 and 17,9Hz, 1 H), 3,7 (m, 3H), 3,9 (m, 2H), 5,6 (dd, J=6,1 y 11,9 Hz, 1 H), 7-7,25 (m, 7H).

**Example 6:**

**N-[5-(4-Chloro-phenyl)-1-(2,4-dichloro-phenyl)-4,5-dihydro-1H-pyrazole-3-carbonyl]-4-methyl-phenylsulfonamide**

This compound was obtained in form of an amorph solid.
IR (KBr, $cm^{-1}$) : 1697, 1481, 1436, 1340, 1169, 1074, 853.
$^1H$ NMR ($CDCl_3$, δ): 2,4 (s, 3H), 3,2 (dd, J=6,6 and 18,3Hz, 1 H), 3,6 (dd, J=12,8 and 18,3Hz, 1 H), 5,8 (dd, J=6,6 and 12,8Hz, 1 H), 7 (d, J=8,2Hz, 2H), 7,2 (s, 1 H), 7,3-7,4 (m, 6H), 8 (d, J=8,1 Hz, 2H), 9 (s, 1 H).

**Example 7:**

**N-oxide of N-piperidinyl-5-(4-chlorophenyl)-1-(2,4-dichlorophenyl)-4,5-dihydropyrazole-3-carboxamide**

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 ˚C and m-chloroperbenzoic acid (0,204 g, 0,83 mmoles) added in several portions. After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 °C.
IR (KBr, $cm^{-1}$): 3202, 1678, 1654, 1474, 1309, 1107.
$^1$H-NMR ($CDCl_3$, δ): 1.6 (m, 2H), 1.8-2.0 (m, 4H), 2.55 (m, 2H), 3.3 (dd, J = 6.3 Hz and 18.2 Hz, 1 H), 3.7 (m, 3H), 5.8 (dd, J = 6.3 Hz and 12.5 Hz, 1H), 7.0-7.3 (m, 7H), 8.5 (s, 1 H.)

**Example 8:**

**Preparation of 5-(4-chlorophenyl)-1-(2,4-dichlorophenyl)-*N*-(piperidin-1-yl)-4,5-dihydro-1H-pyrazole-3-carboxamide hydrobromide**

An aqueous solution (48%) of hydrobromic acid (0.36 mL, 6.64 mmol, 1 eq) is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at 0°C. The crystals formed are filtered off, washed with ethyl acetate, and dried under vacuum (10 mm Hg) at 50°C for 5 hours to give the expected salt as a pale yellow solid (1.79 g, 51% yield).
DSC analysis: melting point at 218-223°C followed by decomposition.
TG analysis: Weight loss, due to decomposition, at temperatures higher than 209°C.
FTI R (ATR) $\upsilon_{max}$: 2551, 1684, 1521, 1481, 1235, 1150, 1105, 1016, 825 and 786 $cm^{-1}$.
$^1$H NMR (400 MHz, $CD_3OD$) δ: 1.59-1.67 (m, 2H), 1.91-1.96 (m, 4H), 3.21 (dd, *J* = 7 Hz, J = 18 Hz, 1 H), 3.24-3.28 (bs), 3.48-3.57 (m, 4H), 3.69 (dd, J = 12 Hz, J = 18 Hz, 1 H), 5.89 (dd, J = 7 Hz, J = 12 Hz, 1 H), 7.12-7.35 (m, 7H).

**Example 9:**

**Preparation of 5-(4-chlorophenyl)-1-(2,4-dichlorophenyl)-*N*-(piperidin-1-yl)-4,5 dihydro-1H-pyrazole-3-carboxamide hydrochloride**

An aqueous solution (37%) of hydrochloric acid (0.203 mL, 6.64 mmol, 1 eq) is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at room temperature. The crystals formed are filtered off, washed with ethyl acetate, and dried under vacuum (10 mm Hg) at 50°C for 5 hours to give the expected salt as a white solid (1.91g, 59% yield).
DSC analysis: between 180 and 205°C.
TG analysis: Weight loss, due to decomposition, at temperatures higher than 197°C
FTIR (ATR) $\upsilon_{max}$: 2422,1682,1538,1479,1308,1239,1107 and 825 $cm^{-1}$.
$^1$H NMR (400 MHz, $CD_3OD$) δ: 1.59-1.66 (m, 2H), 1.89-1.95 (m, 4H), 3.22 (dd, J = 7 Hz, J = 18 Hz, 1H), 3.25-3.31 (bs), 3.40-3.47 (m, 4H), 3.71 (dd, J = 11 Hz, J = 18 Hz, 1 H), 5.90 (dd, J = 7 Hz, J = 13 Hz, 1 H), 7.13-7.38 (m, 7H).

**Example 10:**

**Preparation of 5-(4-chlorophenyl)-1-(2,4-dichlorophenyl)-*N*-(piperidin-1-yl)-4,5-dihydro-1H-pyrazole-3-carboxamide naphthalene-1,5-disulfonate**

A solution of naphthalene disulfonic acid tetrahydrate (2.38 g, 6.64 mmol, 1 eq) in 12 mL of ethanol is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at 70°C. The resulting suspension is cooled to room temperature, and kept at 3°C for 24 hours. The crystals formed are filtered off, washed with ethyl acetate, and dried under vacuum (10 mm Hg) at 50°C for 5 hours to give the expected salt as a pale yellow solid (3.86 g, 49% yield).
DSC analysis: Melting with decomposition, no measurable melting point.
TG analysis: Weight loss of 3.2% between 50 and 83°C; weight loss due to decomposition, at temperatures higher than 200°C.
FTIR (ATR) $\upsilon_{max}$: 1664, 1481, 1239, 1170, 1116, 1018, 826 and 793 $cm^{-1}$
$^1$H NMR (400 MHz, $(CD3)_2SO$) δ: 1.43-1.52 (m, 4H), 1.76-1.83 (m, 8H), 3.14 (dd, J = 7 Hz, J = 18 Hz, 2H), 3.24-3.31 (m, 8H), 3.74 (dd, J = 12 Hz, J = 18 Hz, 2H), 4.03-4.23 (bs), 5.88 (dd, J = 7 Hz, J = 12 Hz, 2H), 7.21-7.25 (m, 16H), 7.94 (d, J = 7 Hz, 2H), 8.88 (d, J = 9 Hz, 2H).
$^{13}$C NMR (100 MHz, $(CD3)_2$ SO) δ: 21.1, 22.9, 40.8, 56.4, 66.1, 123.8, 124.0, 125.9, 126.0, 127.7, 128.7 (x2), 129.1, 129.5 (x2), 129.6, 132.8, 138.1, 139.4, 143.0,143.8,159.4.
K.F. analysis: Water content of 2.8%.

**Example 11:**

**Preparation of 5-(4-chlorophenyl)-1-(2,4-dichlorophenyl)-*N*-(piperidin-1-yl)-4,5-dihydro-1H-pyrazole-3-carboxamide hydrogensulphate**

Concentrated sulphuric acid (0.35 mL, 6.64 mmol, 1 eq) is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at 0˚C. The crystals formed are filtered off, washed with ethyl acetate, and dried under vacuum (10 mm Hg) at 50˚C for 5 hours to give the expected salt as a white solid (2.811 g, 77% yield).

DSC analysis: Broad melting point at 190-197˚C followed by decomposition.
TG analysis: Weight loss, due to decomposition, at temperatures higher than 209˚C.
FTIR(ATR) $\upsilon_{max}$: 2441, 1656, 1478, 1168, 1110, 1056, 862 and 822 $cm^{-1}$
$^1$H NMR (400 MHz, $CD_3OD$) δ: 1.61-1.70 (m, 2H), 1.92-1.98 (m, 4H), 3.20 (dd, J = 6 Hz, J = 18 Hz, 1 H), 3.25-3.29 (bs), 3.52-3.60 (m, 4H), 3.70 (dd, J = 12 Hz, J = 18 Hz, 1H), 5.91 (dd, J = 5 Hz, J = 11 Hz, 1 H), 7.13-7.40 (m, 7H).

**Example 12:**

**Preparation of 5-(4-chlorophenyl)-1-(2,4-dichlorophenyl)-*N*-(piperidin-1-yl)-4,5-dihydro-1H-pyrazole-3-carboxamide ethanesulfonate**

Ethanesulfonic acid (0.54 mL, 6.64 mmol, 1 eq) is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at 70˚C. The resulting solution is cooled to room temperature, and kept at 3˚C for 24 hours. The crystals formed are filtered off, washed with ethyl acetate, and dried under vacuum (10 mm Hg) at 50˚C for 5 hours to give the expected salt as a white solid (3.43 g, 92% yield).

DSC analysis: Broad endothermic peak at 72-99˚C due to water loss; broad melting point at 140-147˚C.
TG analysis: Weight loss of 3.09% between 80 and 101˚C; weight loss due to decomposition, at temperatures higher than 255˚C.
FTIR (ATR) $\upsilon_{max}$: 3362, 1687, 1479, 1241, 1205, 1146, 1086, 1023 and 740 $cm^{-1}$ $^1$H NMR (400 MHz, $CD_3OD$) δ: 1.23 (t, J = 7 Hz, 3H), 1.61-1.69 (m, 2H), 1.92-1.98 (m, 4H), 2.74 (q, J = 7 Hz, 2H), 3.22 (dd, J = 7 Hz, J = 19 Hz, 1 H), 3.23-3.25 (bs), 3.50-3.58 (m, 4H), 3.70 (dd, J = 12 Hz, J = 18 Hz, 1 H), 5.89 (dd, J = 7 Hz, J = 13 Hz, 1 H), 7.12-7.35 (m, 7H).
K.F. analysis: Water content of 3.7%.

**Example 13:**

**Preparation of 5-(4-chlorophenyl)-1-(2,4-dichlorophenyl)-*N*-(piperidin-1-yl)-4,5-dihydro-1H-pyrazole-3-carboxamide nitrate**

An aqueous solution (65%) of nitric acid (0.294 mL, 6.64 mmol, 1 eq) is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at 0˚C. The crystals formed are filtered off, washed with ethyl acetate, and dried under vacuum (10 mm Hg) at 50˚C for 5 hours to give the expected salt as a pale yellow solid (2.638 g, 77% yield).

DSC analysis: Decomposition of the sample before the melting point.
TG analysis: Weight loss due to decomposition, at temperatures higher than 141˚C.
FTIR (ATR) $\upsilon_{max}$: 3235, 1660, 1479, 1314, 1239, 1104, 1021, 871 and 822 $cm^{-1}$
$^1$H NMR (400 MHz, $CD_3OD$) δ: 1.55-1.63 (m, 2H), 1.86-1.92 (m, 4H), 3.19 (dd, J = 7 Hz, J = 18 Hz, 1 H), 3.23-3.28 (bs), 3.36-3.42 (m, 4H), 3.68 (dd, J = 12 Hz, J = 17 Hz, 1H), 5.87 (dd, J = 7 Hz, J = 13 Hz, 1 H), 7.10-7.33 (m, 7H).

**Example 14:**

**Preparation of 5-(4-chlorophenyl)-1-(2,4-dichlorophenyl)-*N*-(piperidin-1-yl)-4,5-dihydro-1H-pyrazole-3-carboxamide *p*-toluenesulfonate**

A solution of p-toluenesulfonic acid (1.26 g, 6.64 mmol, 1 eq) in 12 mL of ethyl acetate is slowly added to a solution of 5-(4-chlorophenyl)-1-(2,4-dichlorophenyl)-*N*-(pipeddin-1-yl)-4,5-dihydro-1H-pyrazole-3-carboxamide according to example 1 (3.0 g, 6.64 mmol, 1 eq.) in 30 mL of ethyl acetate at 70˚C. The resulting suspension is cooled to room temperature, and kept at 3˚C for 24 hours. The crystals formed are filtered off, washed with ethyl acetate, and

dried under vacuum (10 mm Hg) at 50˚C for 5 hours to give the expected salt as a white solid (3.84 g, 93% yield).
DSC analysis: Melting point at 212-215˚C followed by decomposition.
TG analysis: Weight loss due to decomposition, at temperatures higher than 223˚C.
FTIR (ATR) $\upsilon_{max}$: 2592, 1688, 1480, 1235,'1152, 1108, 1037, 1008, 826 and 675 $cm^{-1}$
$^1H$ NMR (400 MHz, $CD_3OD$) δ: 1.61-1.68 (m, 2H), 1.91-1.97 (m, 4H), 2.31 (s, 3H), 3.22 (dd, *J* = 13 Hz, *J* = 20 Hz, 1H), 3.26-3.31 (bs), 3.52-3.59 (m, 4H), 3.71 (dd, *J* = 7 Hz, *J* = 19 Hz, 1 H), 5.91 (dd, *J* = 7 Hz, *J* = 13 Hz, 1H), 7.07-7.37 (m, 9H), 7.66 (d, J = 8 Hz, 2H).

**Example 15:**

**Preparation of 5-(4-chlorophenyl)-1-(2,4-dichlorophenyl)-*N*-(piperidin-1-yl)-4,5-dihydro-1H-pyrazole-3-carboxamide thiocyanate**

A solution of thiocyanic acid (392 mg, 6.64 mmol, 1 eq) in 11.4 mL of ethyl acetate is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at room temperature, and kept at 3˚C for 24 hours. The crystals formed are filtered off, washed with ethyl acetate, and dried under vacuum (10 mm Hg) at 50˚C for 5 hours to give the expected salt as a white solid (3.25 g, 96% yield).

DSC analysis: Broad endothermic peak between 128 and 144˚C that coincides with a weight loss of 8.6%. The melting point is not clear.
TG analysis: Weight loss of 8.6%, between 115 and 150˚C; weight loss due to decomposition, at temperatures higher than 200˚C.
FTI R (ATR) $\upsilon_{max}$: 3174, 2046, 1731, 1665, 1481, 1240, 1111 and 822 $cm^{-1}$
$^1H$ NMR (400 MHz, $CD_3OD$) δ: 1.57-1.64 (m, 2H), 1.87-1.93 (m, 4H), 3.22 (dd, J = 7 Hz, J = 19 Hz, 1 H), 3.24 (bs), 3.35-3.43 (m, 4H), 3.69 (dd, J = 13 Hz, J = 18 Hz, 1 H), 5.89 (dd, J = 7 Hz, J = 12 Hz, 1 H), 7.12-7.36 (m, 7H).

**Example 16:**

**Preparation of 5-(4-chlorophenyl)-1-(2,4-dichlorophenyl)-*N*-(piperidin-1-yl)-4,5-dihydro-1H-pyrazole-3-carboxamide methanesulfonate**

Methanesulfonic acid (0.42 mL, 6.64 mmol, 1 eq) is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at 70˚C. The resulting solution is cooled to room temperature, and kept at 3˚C for 24 hours. The crystals formed are filtered off, washed with ethyl acetate, and dried under vacuum (10 mm Hg) at 50˚C for 5 hours to give the expected salt as a white solid (3.46 g, 95% yield).

DSC analysis: Broad endothermic peak at 61-90˚C due to water loss; sharp melting point at 175-177˚C.
TG analysis: Weight loss of 3.1% between 79 and 97˚C; weight loss due to decomposition, at temperatures higher than 251 ˚C.
FTIR (ATR) $\upsilon_{max}$: 3366, 1690, 1480, 1216, 1161, 1089, 1040, 1028, 825 and 773 $cm^{-1}$
$^1H$ NMR (400 MHz, $CD_3OD$) δ: 1.60-1.68 (m, 2H), 1.91-1.97 (m, 4H), 2.63 (s, 3H), 3.21 (dd, J = 6 Hz, J = 17 Hz, 1 H), 3.24-3.28 (bs), 3.48-3.55 (m, 4H), 3.69 (dd, *J* = 14 Hz, *J* = 19 Hz, 1H), 5.88 (dd, *J* = 6 Hz, *J* = 13 Hz, 1 H), 7.12-7.34 (m, 7H).
K.F. analysis: Water content of 4.1 %.

**Example 17:**

**Preparation of 5-(4-chlorophenyl)-1-(2,4-dichlorophenyl)-*N*-(piperidin-1-yl)-4,5-dihydro-1H-pyrazole-3-carboxamide 2-naphthalenesulfonate**

A solution of 2-naphthalenesulfonic acid (1.38 g, 6.64 mmol, 1 eq) in 12 mL of ethyl acetate is slowly added to a solution of 5-(4-chlorophenyl)-1-(2,4-dichlorophenyl)-*N*-(piperidin-1-yl)-4,5-dihydro-1H-pyrazole-3-carboxamide according to example 1 (3.0 g, 6.64 mmol, 1 eq.) in 30 mL of ethyl acetate at 70˚C. The resulting solution is cooled to room temperature, and kept at 3˚C for 24 hours. The crystals formed are filtered off, washed with ethyl acetate, and dried under vacuum (10 mm Hg) at 50˚C for 5 hours to give the expected salt as a white solid (2.99 g, 68% yield).

DSC analysis: Broad endothermic peak between 124 and 142˚C; melting point at 169-174˚C followed by decomposition.
TG analysis: Weight loss due to decomposition, at temperatures higher than 172˚C
FTIR (ATR) $\upsilon_{max}$: 3166, 1668, 1480, 1235, 1166, 1118, 1089, 1020, 822 and 670 $cm^{-1}$

$^1H$ NMR (400 MHz, $CD_3OD$) δ: 1.57-1.64 (m, 2H), 1.89-1.94 (m, 4H), 3.18 (dd, J = 6 Hz, J = 17 Hz, 1H), 3.23-3.28 (bs), 3.49-3.55 (m, 4H), 3.67 (dd, J = 14 Hz, J = 19 Hz, 1 H), 5.87 (dd, J = 7 Hz, J = 13 Hz, 1 H), 7.04-7.83 (m, 13H), 8.28 (s, 1H).

**Example 18:**

**Preparation of 5-(4-chlorophenyl)-1-(2,4-dichlorophenyl)-*N*-(piperidin-1-yl)-4,5-dihydro-1H-pyrazole-3-carboxamide benzenesulfonate**

A solution of benzenesulfonic acid (1.05 g, 6.64 mmol, 1 eq) in 12 mL of ethanol is slowly added to a solution of 5-(4-chlorophenyl)-1-(2,4-dichlorophenyl)-N-(piperidin-1-yl)-4,5-dihydro-1H-pyrazole-3-carboxamide according to example 1 (3.0 g, 6.64 mmol, 1 eq.) in 30 mL of ethyl acetate at room temperature, and the solvent is evaporated to dryness. The resulting crude is dissolved in 15 mL of hot methanol and allowed to cool to 3°C. The crystals formed are filtered off, washed with ethyl acetate, and dried under vacuum (10 mm Hg) at 50°C for 5 hours to give the expected salt as a pale yellow solid (0.937 g, 23% yield).

DSC analysis: Melting point at 176-181°C followed by decomposition.
TG analysis: Weight loss due to decomposition, at temperatures higher than 259°C
FTIR (ATR) $\upsilon_{max}$: 3150, 1667, 1478, 1310, 1221, 1166, 1106, 999, 821, 757, 722 and 692 $cm^{-1}$
$^1H$ NMR (400 MHz, $CD_3OD$) δ: 1.62-1.69 (m, 2H), 1.92-1.97 (m, 4H), 3.22 (dd, J = 8 Hz, J = 18 Hz, 1 H), 3.26-3.27 (bs), 3.51-3.57 (m, 4H), 3.70 (dd, J = 11 Hz, J = 17 Hz, 1H), 5.91 (dd, *J* = 7 Hz, *J* = 13 Hz, 1 H), 7.10-7.33 (m, 10H), 7.79 (d, *J* = 11 Hz, 2H).

**Example 19**

**5-(4-Chloro-phenyl)-1-(2,4-dichloro-phenyl)-4,5-dihydro-1H-pirazole-3-carboxylic acid piperidin-1-ylamide hydrochloride**

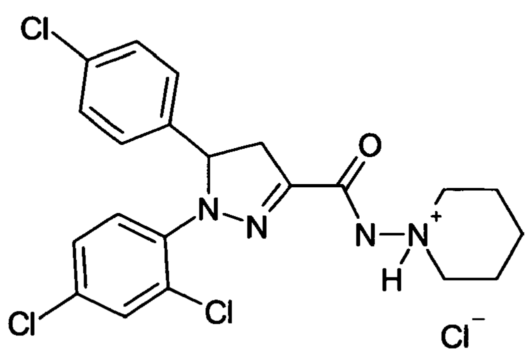

The 5-(4-Chloro-phenyl)-1-(2,4-dichloro-phenyl)-4,5-dihydro-1H-pirazole-3-carboxylic acid piperidin-1-ylamide (12 g, 26,5 mmoles) was dissolved in ethyl acetate (600 ml) and the hydrocloric solution 2,8 N in ethanol (31.8 mmoles) was added dropwise. After 5 min a white precipitate was observed. This was isolated by filtration and was washed with ethyl acetate, yielding de hydrochloric salt (11,74g, 91%).

Melting point. : 223-226 °C (capilar)
FTIR.(KBr) : 3023, 2947, 2542, 2455, 1685, 1481, 1313, 1241,117, 826 $cm^{-1}$
$^1H$ NMR (400 MHz, $CD_3OD$) δ: 1.59-1.66 (m, 2H), 1.89-1.95 (m, 4H), 3.22 (dd, J = 7 Hz, J = 18 Hz, 1 H), 3.25-3.31 (bs), 3.40-3.47 (m, 4H), 3.71 (dd, J = 11 Hz, J = 18 Hz, 1H), 5.90 (dd, J = 7 Hz, J = 13 Hz, 1 H), 7.13-7.38 (m, 7H).

**Example 20**

**5(R)-(4-Chloro-phenyl)-1-(2,4-dichloro-phenyl)-4,5-dihydro-1H-pirazole-3-carboxylic acid piperidin-1-ylamide hydrochloride**

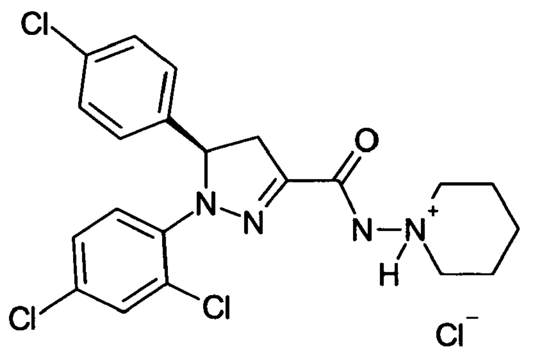

Melting point. : 227-230 ￭C (capilar)
FTI R. (KBr) : 3023, 2947, 2542, 2455, 1685, 1481, 1313, 1241,117, 826 $cm^{-1}$
$^1H$ NMR (400 MHz, $CD_3OD$) δ: 1.59-1.66 (m, 2H), 1.89-1.95 (m, 4H), 3.22 (dd, J = 7 Hz, J = 18 Hz, 1 H), 3.25-3.31 (bs), 3.40-3.47 (m, 4H), 3.71 (dd, J = 11 Hz, J = 18 Hz, 1 H), 5.90 (dd, J = 7 Hz, J = 13 Hz, 1 H), 7.13-7.38 (m, 7H).

**Example 21**

**5(S)-(4-Chloro-phenyl)-1-(2,4-dichloro-phenyl)-4,5-dihydro-1H-pirazole-3-carboxylic acid piperidin-1-ylamide hydrochloride**

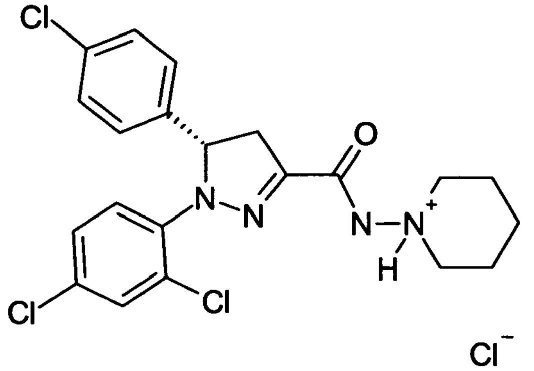

Melting point. : 230-232 ￭C (capilar)
FTIR.(KBr) : 3023, 2947, 2542, 2455, 1685, 1481, 1313, 1241,117, 826 $cm^{-1}$
$^1H$ NMR (400 MHz, $CD_3OD$) δ: 1.59-1.66 (m, 2H), 1.89-1.95 (m, 4H), 3.22 (dd, J = 7 Hz, J = 18 Hz, 1 H), 3.25-3.31 (bs), 3.40-3.47 (m, 4H), 3.71 (dd, J = 11 Hz, J = 18 Hz, 1 H), 5.90 (dd, J = 7 Hz, J = 13 Hz, 1 H), 7.13-7.38 (m, 7H).

**Example 22**

**5-(4-Chloro-phenyl)-1-(2,4-dichloro-phenyl)-4,5-dihydro-1H-pirazole-3-carboxylic acid piperidin-1-ylamide nitrate**

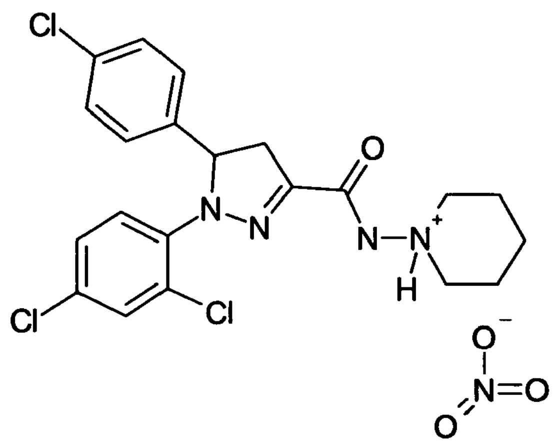

The 5-(4-Chloro-phenyl)-1-(2,4-dichloro-phenyl)-4,5-dihydro-1H-pirazole-3-carboxylic acid piperidin-1-ylamide (0.34 g, 0.75 mmoles) was dissolved in ethyl acetate (20 ml) and the (0,9 mmoles) nitric acid solution in (2 ml) ethanol was added dropwise. After 10 min a white precipitate was observed. This was isolated by filtration and was washed with ethyl acetate, yielding de nitrate salt (0,27 g, 68%).
Melting point. : 146-152 °C (capilar)
FTIR.(KBr) : 3241, 2952, 1666, 1477, 1383, 1115, 1023, 878, 816 $cm^{-1}$
$^1H$ NMR (400 MHz, $CD_3OD$) δ: 1.55-1.63 (m, 2H), 1.86-1.92 (m, 4H), 3.19 (dd, J = 7 Hz, J = 18 Hz, 1 H), 3.23-3.28 (bs), 3.36-3.42 (m, 4H), 3.68 (dd, J = 12 Hz, J = 17 Hz, 1 H), 5.87 (dd, J = 7 Hz, J = 13 Hz, 1 H), 7.10-7.33 (m, 7H).

The compounds 5-6 have been prepared with analogous process described in Example 4

**Example 23**

**5-(4-Chloro-phenyl)-1-(2,4-dichloro-phenyl)-4,5-dihydro-1H-pirazole-3-carboxylic acid piperidin-1-ylamide hydrogen sulphate**

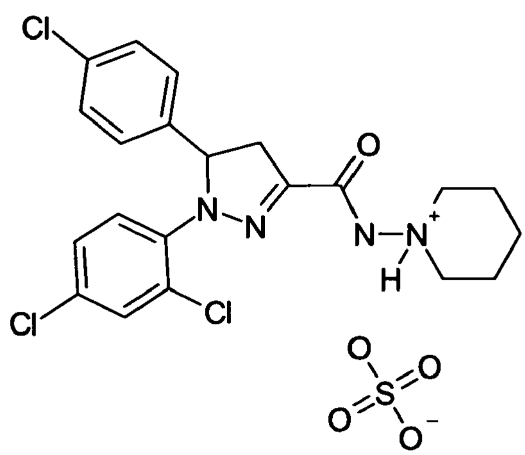

The 5-(4-Chloro-phenyl)-1-(2,4-dichloro-phenyl)-4,5-dihydro-1H-pirazole-3-carboxylic acid piperidin-1-ylamide (0.34 g,

0.75 mmoles) was dissolved in ethyl acetate (20 ml) and the (0,9 mmoles) sulfuric acid solution in (2 ml) ethanol was added dropwise. After 5-10 min a white precipitate was observed. This was isolated by filtration and was washed with ethyl acetate, yielding de hydrogen sulfate salt (0,30 g, 73 %).
Melting point. : 198-199 °C (capilar)
FTIR.(KBr) : 3422, 2948, 1655, 1477, 1244, 1170, 1115, 1058, 862, 824 $cm^{-1}$

**Example 24**

**5-(4-Chloro-phenyl)-1-(2,4-dichloro-phenyl)-4,5-dihydro-1H-pirazole-3-carboxylic acid piperidin-1-ylamide methanesulfonate.**

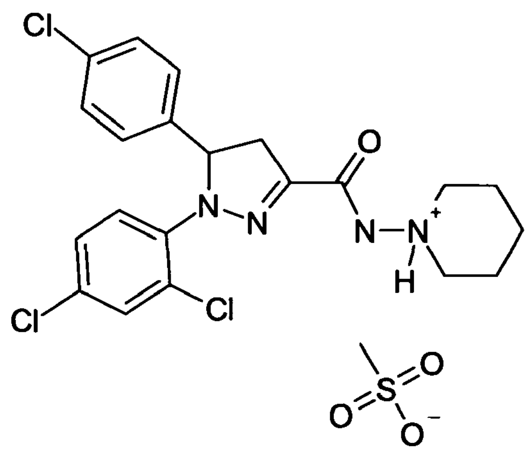

The 5-(4-Chloro-phenyl)-1-(2,4-dichloro-phenyl)-4,5-dihydro-1H-pirazole-3-carboxylic acid piperidin-1-ylamide (0.34 g, 0.75 mmoles) was dissolved in ethyl acetate (20 ml) and the (0,9 mmoles) metanesulfonic acid solution in (2 ml) ethanol was added dropwise. After 10 min a white precipitate was observed. This was isolated by filtration and was washed with ethyl acetate, yielding de methanesulfonate salt (0,31 g, 75%).
Melting point. : 175-177 °C (capilar)
FTIR.(KBr) : 3398,2948, 1692, 1478, 1245, 1206, 1161, 1039, 816 $cm^{-1}$
$^1$H NMR (400 MHz, $CD_3OD$) $\delta$: 1.60-1.68 (m, 2H), 1.91-1.97 (m, 4H), 2.63 (s, 3H), 3.21 (dd, J = 6 Hz, J = 17 Hz, 1 H), 3.24-3.28 (bs), 3.48-3.55 (m, 4H), 3.69 (dd, *J* = 14 Hz, *J* = 19 Hz, 1H), 5.88 (dd, *J* = 6 Hz, *J* = 13 Hz, 1 H), 7.12-7.34 (m, 7H).

**Example 25**

**5(R)-(4-Chloro-phenyl)-1-(2,4-dichloro-phenyl)-4,5-dihydro-1H-pirazole-3-carboxylic acid piperidin-1-ylamide methanesulfonate.**

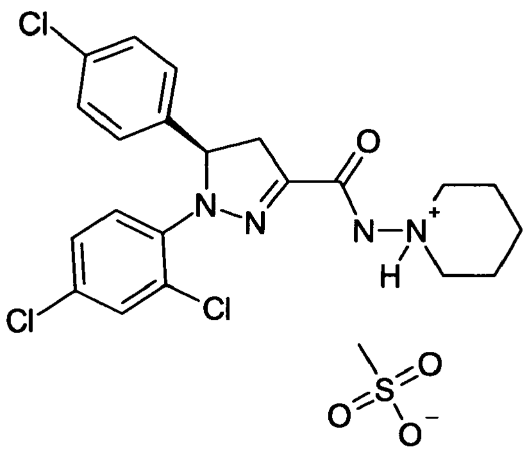

Melting point. : 157-160 C (capilar)
FTIR.(KBr) : 3431,2949, 1683, 1478, 1245, 1204, 1171, 1043, 822 $cm^{-1}$
$^1H$ NMR (400 MHz, $CD_3OD$) δ: 1.60-1.68 (m, 2H), 1.91-1.97 (m, 4H), 2.63 (s, 3H), 3.21 (dd, J = 6 Hz, J = 17 Hz, 1H), 3.24-3.28 (bs), 3.48-3.55 (m, 4H), 3.69 (dd, J = 14 Hz, J = 19 Hz, 1 H), 5.88 (dd, J = 6 Hz, J = 13 Hz, 1 H), 7.12-7.34 (m, 7H).

**Example 26**

**5-(4-Chloro-phenyl)-1-(2,4-dichloro-phenyl)-4,5-dihydro-1H-pirazole-3-carboxylic acid piperidin-1-ylamide hydrobromide**

An aqueous solution (48%) of hydrobromic acid (0.36 mL, 6.64 mmol, 1 eq) is slowly added to a solution of E-6776 (3.0 g, 6.64 mmol, 1 eq.) in 30 mL of ethyl acetate at 0˚C. The crystals formed are filtered off, washed with ethyl acetate, and dried under vacuum (10 mm Hg) at 50˚C for 5 hours to give the expected salt as a pale yellow solid (1.79 g, 51% yield).
DSC analysis: melting point at 218-223˚C followed by decomposition.
TG analysis: Weight loss, due to decomposition, at temperatures higher than 209˚C.
FTIR (ATR) $\upsilon_{max}$ : 2551, 1684, 1521, 1481, 1235, 1150, 1105, 1016, 825 and 786 $cm^{-1}$.
$^1H$ NMR (400 MHz, $CD_3OD$) δ: 1.59-1.67 (m, 2H), 1.91-1.96 (m, 4H), 3.21 (dd, J = 7 Hz, J = 18 Hz, 1H), 3.24-3.28 (bs), 3.48-3.57 (m, 4H), 3.69 (dd, J = 12 Hz, J = 18 Hz, 1 H), 5.89 (dd, J = 7 Hz, J = 12 Hz, 1H), 7.12-7.35 (m, 7H).

**Example 27**

**(4-Chloro-phenyl)-1-(2,4-dichloro-phenyl)-4,5-dihydro-1H-pirazole-3-carboxylic acid piperidin-1-ylamide ethanesulfonate.**

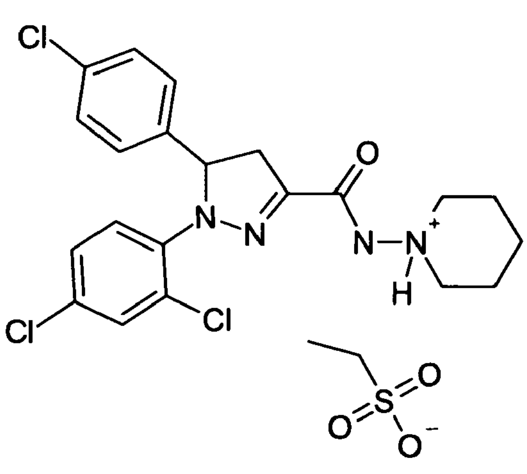

Ethanesulfonic acid (0.54 mL, 6.64 mmol, 1 eq) is slowly added to a solution of E-6776 (3.0 g, 6.64 mmol, 1 eq.) in 30 mL of ethyl acetate at 70˚C. The resulting solution is cooled to room temperature, and kept at 3˚C for 24 hours. The crystals formed are filtered off, washed with ethyl acetate, and dried under vacuum (10 mm Hg) at 50˚C for 5 hours to give the expected salt as a white solid (3.43 g, 92% yield).
DSC analysis: Broad endothermic peak at 72-99˚C due to water loss; broad melting point at 140-147˚C.
TG analysis: Weight loss of 3.09% between 80 and 101˚C; weight loss due to decomposition, at temperatures higher than 255˚C.
FTI R (ATR) $\upsilon_{max}$ : 3362, 1687, 1479, 1241, 1205, 1146, 1086, 1023 and 740 $cm^{-1}$ $^1$H NMR (400 MHz, $CD_3OD$) δ: 1.23 (t, J = 7 Hz, 3H), 1.61-1.69 (m, 2H), 1.92-1.98 (m, 4H), 2.74 (q, J = 7 Hz, 2H), 3.22 (dd, J = 7 Hz, J = 19 Hz, 1 H), 3.23-3.25 (bs), 3.50-3.58 (m, 4H), 3.70 (dd, J = 12 Hz, J = 18 Hz, 1 H), 5.89 (dd, J = 7 Hz, J = 13 Hz, 1 H), 7.12-7.35 (m, 7H).
K.F. analysis: Water content of 3.7%.

**Example 28**

**4-Chloro-phenyl)-1-(2,4-dichloro-phenyl)-4,5-dihydro-1H-pirazole-3-carboxylic acid piperidin-1-ylamide toluene-4-sulfonate.**

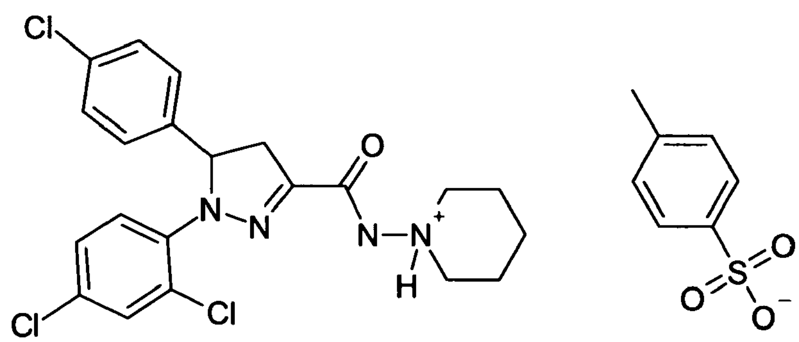

A solution of *p*-toluenesulfonic acid (1.26 g, 6.64 mmol, 1 eq) in 12 mL of ethyl acetate is slowly added to a solution of E-6776 (3.0 g, 6.64 mmol, 1 eq.) in 30 mL of ethyl acetate at 70˚C. The resulting suspension is cooled to room temperature, and kept at 3˚C for 24 hours. The crystals formed are filtered off, washed with ethyl acetate, and dried under vacuum (10 mm Hg) at 50˚C for 5 hours to give the expected salt as a white solid (3.84 g, 93% yield). DSC analysis: Melting point at 212-215˚C followed by decomposition.
TG analysis: Weight loss due to decomposition, at temperatures higher than 223˚C.
FTIR (ATR) $\upsilon_{max}$: 2592, 1688, 1480, 1235, 1152, 1108, 1037, 1008, 826 and 675 $cm^{-1}$
$^1$H NMR (400 MHz, $CD_3OD$) δ: 1.61-1.68 (m, 2H), 1.91-1.97 (m, 4H), 2.31 (s, 3H), 3.22 (dd, J = 13 Hz, J = 20 Hz, 1 H), 3.26-3.31 (bs), 3.52-3.59 (m, 4H), 3.71 (dd, J = 7 Hz, J = 19 Hz, 1H), 5.91 (dd, J = 7 Hz, J = 13 Hz, 1 H), 7.07-7.37 (m, 9H), 7.66 (d, J = 8 Hz, 2H).

**Example 29**

**(4-Chloro-phenyl)-1-(2,4-dichloro-phenyl)-4,5-dihydro-1H-pirazole-3-carboxylic acid piperidin-1-ylamide benzenesulfonate.**

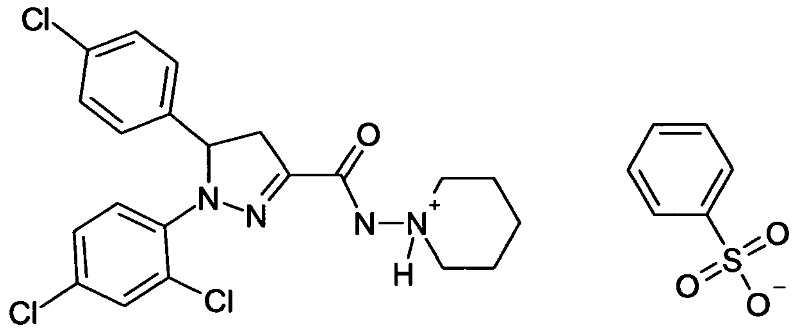

A solution of benzenesulfonic acid (1.05 g, 6.64 mmol, 1 eq) in 12 mL of ethanol is slowly added to a solution of E-6776 (3.0 g, 6.64 mmol, 1 eq.) in 30 mL of ethyl acetate at room temperature, and the solvent is evaporated to dryness. The resulting crude is dissolved in 15 mL of hot methanol and allowed to cool to 3°C. The crystals formed are filtered off, washed with ethyl acetate, and dried under vacuum (10 mm Hg) at 50°C for 5 hours to give the expected salt as a pale yellow solid (0.937 g, 23% yield).

DSC analysis: Melting point at 176-181°C followed by decomposition.
TG analysis: Weight loss due to decomposition, at temperatures higher than 259°C
FTIR(ATR)$\upsilon_{max}$:3150, 1667, 1478, 1310, 1221, 1166, 1106, 999, 821, 757, 722 and 692 $cm^{-1}$
$^1$H NMR (400 MHz, $CD_3OD$ δ: 1.62-1.69 (m, 2H), 1.92-1.97 (m, 4H), 3.22 (dd, J = 8 Hz, J = 18 Hz, 1 H), 3.26-3.27 (bs), 3.51-3.57 (m, 4H), 3.70 (dd, J = 11 Hz, J = 17 Hz, 1 H), 5.91 (dd, *J* = 7 Hz, *J* = 13 Hz, 1 H), 7.10-7.33 (m, 10H), 7.79 (d, *J* = 11 Hz, 2H).

The compounds 23-24 have been prepared with analogous process described in Example 22

**Example 30**

**(4-Chloro-phenyl)-1-(2,4-dichloro-phenyl)-4,5-dihydro-1H-pirazole-3-carboxylic acid piperidin-1-ylamide-2-naphtalenesulfonate**

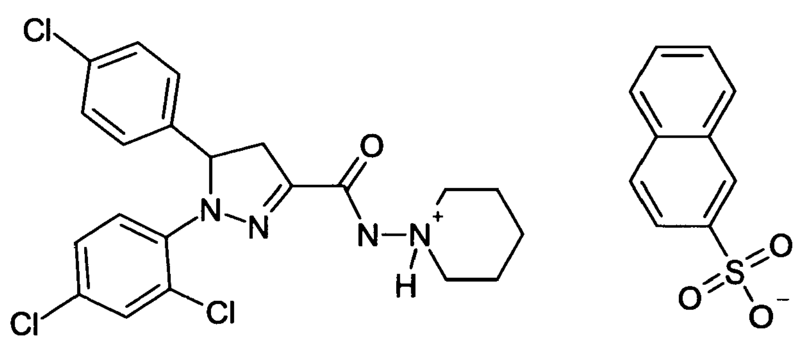

A solution of 2-naphthalenesulfonic acid (1.38 g, 6.64 mmol, 1 eq) in 12 mL of ethyl acetate is slowly added to a solution of E-6776 (3.0 g, 6.64 mmol, 1 eq.) in 30 mL of ethyl acetate at 70°C. The resulting solution is cooled to room temperature, and kept at 3°C for 24 hours. The crystals formed are filtered off, washed with ethyl acetate, and dried under vacuum (10 mm Hg) at 50°C for 5 hours to give the expected salt as a white solid (2.99 g, 68% yield).
DSC analysis: Broad endothermic peak between 124 and 142°C; broad melting point at 169-174°C followed by decomposition.
TG analysis: Weight loss due to decomposition, at temperatures higher than 172°C
FTIR (ATR) $\upsilon_{max}$: 3166, 1668, 1480, 1235, 1166, 1118, 1089, 1020, 822 and 670 $cm^{-1}$
$^1$H NMR (400 MHz, $CD_3OD$) δ: 1.57-1.64 (m, 2H), 1.89-1.94 (m, 4H), 3.18 (dd, J = 6 Hz, J = 17 Hz, 1 H), 3.23-3.28 (bs), 3.49-3.55 (m, 4H), 3.67 (dd, J = 14 Hz, J = 19 Hz, 1 H), 5.87 (dd, J = 7 Hz, J = 13 Hz, 1 H), 7.04-7.83 (m, 13H), 8.28 (s, 1 H).

**Example 31**

**(4-Chloro-phenyl)-1-(2,4-dichloro-phenyl)-4,5-dihydro-1H-pirazole-3-carboxylic acid piperidin-1-ylamide thiocyanate.**

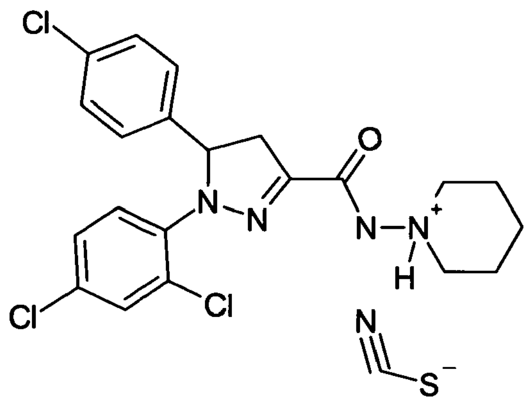

A solution of thiocyanic acid (392 mg, 6.64 mmol, 1 eq) in 11.4 mL of ethyl acetate is slowly added to a solution of E-6776 (3.0 g, 6.64 mmol, 1 eq.) in 30 mL of ethyl acetate at room temperature, and kept at 3˚C for 24 hours. The crystals formed are filtered off, washed with ethyl acetate, and dried under vacuum (10 94 mm Hg) at 50˚C for 5 hours to give the expected salt as a white solid (3.25 g, 96% yield).

DSC analysis: Broad endothermic peak between 128 and 144˚C that coincides with a weight loss of 8.6%. The melting point is not dear.

TG analysis: Weight loss of 8.6%, between 115 and 150˚C; weight loss due to decomposition, at temperatures higher than 200˚C.

FTIR (ATR) $\upsilon_{max}$: 3174, 2046, 1731, 1665, 1481, 1240, 1111 and 822 $cm^{-1}$

$^1H$ NMR (400 MHz, $CD_3OD$) δ: 1.57-1.64 (m, 2H), 1.87-1.93 (m, 4H), 3.22 (dd, J = 7 Hz, J = 19 Hz, 1 H), 3.24 (bs), 3.35-3.43 (m, 4H), 3.69 (dd, J = 13 Hz, J = 18 Hz, 1 H), 5.89 (dd, J = 7 Hz, J = 12 Hz, 1 H), 7.12-7.36 (m, 7H).

| N° | STRUCTURE | Autonom® | FTIR (cm-1) | m.p. °C |
|---|---|---|---|---|
| | 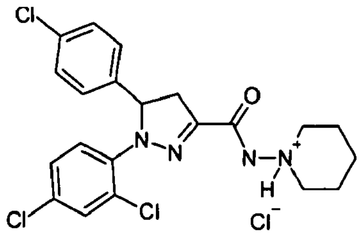 | 1-{[5-(4-Chloro-phenyl)-1-(2,4-dich loro-phenyl)-4,5-dihydro-1H-pyrazol e-3-carbonyl]-amino}-piperidinium; chloride | 2924, 2450, 1685, 1481, 1314, 1244, 1115, 826, | 217-220°C |
| | 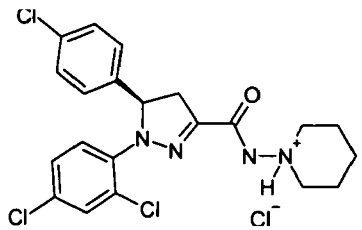 | 1-{[5(R)-(4-Chtoro-phenyl)-1-(2,4-dich loro-phenyl)-4,5-dihydro-1H-pyrazol e-3-carbonyl]-amino}-piperidinium; chloride | 3023, 2947, 2542, 2455, 1685, 1481, 1313, 1241,117, 826 | 227-230°C |
| | 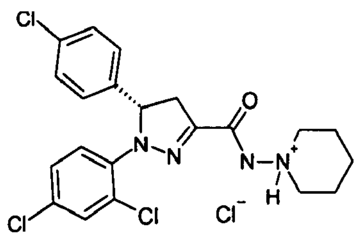 | 1-{[5(S)-(4-Chloro-phenyl)-1-(2,4-dich loro-phenyl)-4,5-dihydro-1H-pyrazol e-3-carbonyl]-amino}-piperidinium; chloride | 3023, 2947, 2542, 2455, 1685, 1481, 1313, 1241, 117, 826 | 230-232°C |
| | 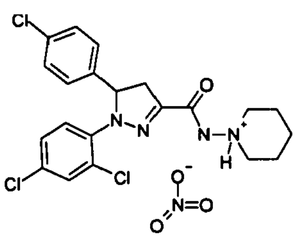 | 1-{[5-(4-Chloro-phenyl)-1-(2,4-dich loro-phenyl)-4,5-dihydro-1H-pyrazol e-3-carbonyl]-amino}-piperidinium; nitrate | 3241, 2952, 1666, 1477, 1383, 1318, 1246, 1114, 870, 816 | 146-152°C |

(continued)

| N° | STRUCTURE | Autonom® | FTIR (cm-1) | m.p. °C |
|---|---|---|---|---|
| | 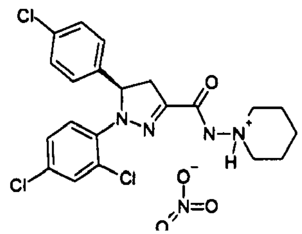 | 1-{[5(R)-(4-Chloro-phenyl)-1-(2,4-dich loro-phenyl)-4,5-dihydro-1H-pyrazol e-3-carbonyl]-amino}-piperidinium; nitrate | | |
| | 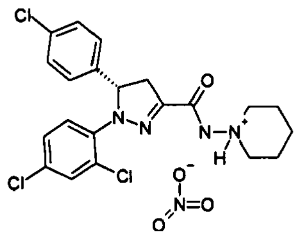 | 1-{[5(S)-(4-Chloro-phenyl)-1-(2,4-dich loro-phenyl)-4,5-dihydro-1H-pyrazol e-3-carbonyl]-amino}-piperidinium; nitrate | | |
| | 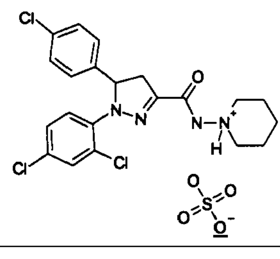 | 1-{[5-(4-Chloro-phenyl)-1-(2,4-dich loro-phenyl)-4,5-dihydro-1H-pyrazol e-3-carbonyl]-amino}-piperidinium; hydrogen sulfate | 3422, 2948, 1655, 1477, 1244, 1170, 1159, 1058, 862,824 | 198-199 °C |
| | 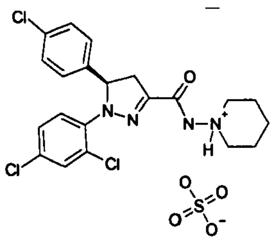 | 1-{[5(R)-(4-Chloro-phenyl)-1-(2,4-dich loro-phenyl)-4,5-dihydro-1H-pyrazol e-3-carbonyl]-amino}-piperidinium; hydrogen sulfate | | |

(continued)

| N° | STRUCTURE | Autonom® | FTIR (cm-1) | m.p. °C |
|---|---|---|---|---|
| | 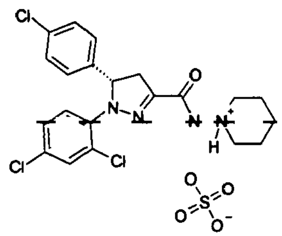 | 1-{[5(S)-(4-Chloro-phenyl)-1-(2,4-dich loro-phenyl)-4,5-dihydro-1H-pyrazol e-3-carbonyl]-amino}-piperidinium; hydrogen sulfate | | |
| | 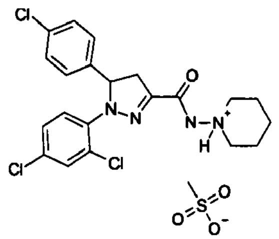 | Methanesulfonate1-{[5-(4-chloro-phe nyl)-1-(2,4-dichloro-phenyl)-4,5-di hydro-1H-pyrazole-3-carbonyl]-amino }-piperidinium; | 3398, 2948, 1692, 1478, 1306, 1245, 1206, 1161, 1105, 1039, 816, 781 | 175-177 °C |
| | 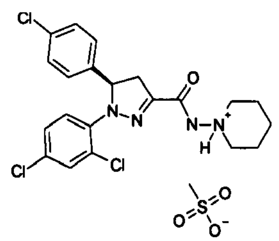 | Methanesulfonatel-{[5(R)-(4-chloro-phe nyl)-1-(2,4-dichloro-phenyl)-4,5-di hydro-1H-pyrazole-3-carbonyl]-amino }-piperidinium; | 3431, 2949, 1683, 1478,1312, 1245, 1204, 1171, 1106, 1043, 822 | 157-160°C |
| | 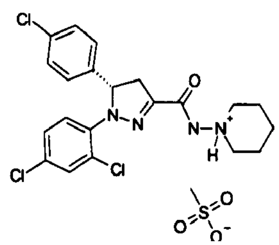 | Methanesulfonate1-{[5(S)-(4-chloro-phe nyl)-1-(2,4-dichloro-phenyl)-4,5-di hydro-1H-pyrazole-3-carbonyl]-amino }-piperidinium; | | |

(continued)

| N° | STRUCTURE | Autonom® | FTIR (cm-1) | m.p. °C |
|---|---|---|---|---|
| | 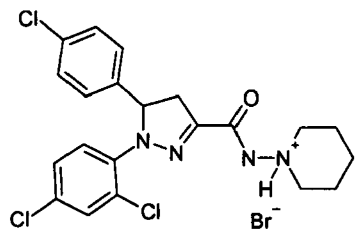 | 1-{[5-(4-Chloro-phenyl)-1-(2,4-dich loro-phenyl)-4,5-dihydro-1H-pyrazol e-3-carbonyl]-amino}-piperidinium; bromide | 2551, 1684, 1521, 1481, 1235, 1150, 1105, 1016, 825, 786 | 218-223°C |
| | 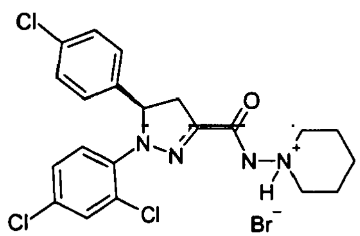 | 1-{[5(R)-(4-Chloro-phenyl)-1-(2,4-dich loro-phenyl)-4,5-dihydro-1H-pyrazol e-3-carbonyl]-amino}-piperidinium; bromide | | |
| | 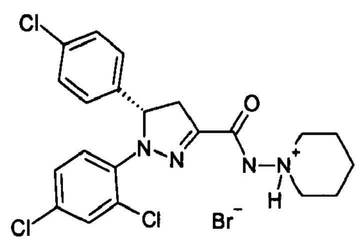 | 1-{[5(S)-(4-Chloro-phenyl)-1-(2,4-dich loro-phenyl)-4,5-dihydro-1H-pyrazol e-3-carbonyl]-amino}-piperidinium; bromide | | |

(continued)

| N° | STRUCTURE | Autonom® | FTIR (cm-1) | m.p. °C |
|---|---|---|---|---|
| | 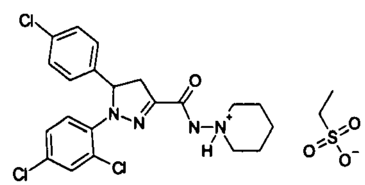 | Ethanesulfonate1-{[5-(4-chloro-phen yl)-1-(2,4-dichloro-phenyl)-4,5-dih ydro-1H-pyrazole-3-carbonyl]-amino} -piperidinium; | 3362, 1687, 1479, 1241, 1205, 1146, 1086, 1023, 740 | 140-147°C. |
| | 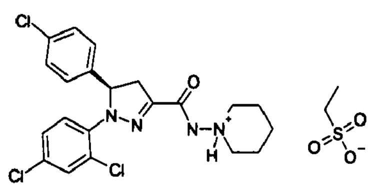 | Ethanesulfonate1-{[5(R)-(4-chloro-phen yl)-1-(2,4-dichloro-phenyl)-4,5-dih ydro-1H-pyrazole-3-carbonyl]-amino} -piperidinium; | | |
| | 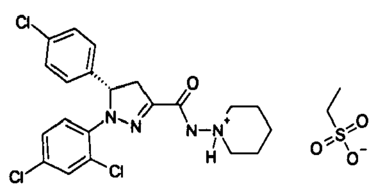 | Ethanesulfonate1-{[5(S)-(4-chloro-phen yl)-1-(2,4-dichloro-phenyl)-4,5-dih ydro-1H-pyrazole-3-carbonyl]-amino} -piperidinium; | | |
| | 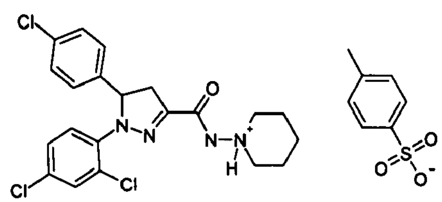 | Toluene-4-sulfonate1-{[5-(4-chlorophenyl)-1-(2,4-dichloro-phenyl)-4,5 -dihydro-1H-pyrazole-3-carbonyl]-am ino}-piperidinium; | 2592, 1688, 1480, 1235, 1152, 1108, 1037, 1008, 826 , 675 | 212-215 °C |

(continued)

| N° | STRUCTURE | Autonom® | FTIR (cm-1) | m.p. °C |
|---|---|---|---|---|
| | 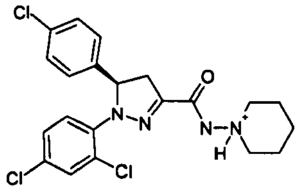 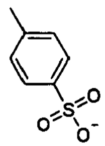 | Toluene-4-sulfonate1-{[5(R)-(4-chlorophenyl)-1-(2,4-dichloro-phenyl)-4,5 -dihydro-1H-pyrazole-3-carbonyl]-am ino}-piperidinium; | | |
| | 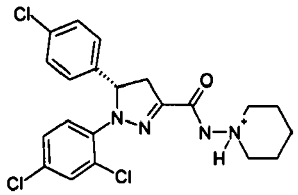 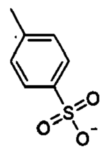 | Toluene-4-sulfonate1-{[5(S)-(4-chlorophenyl)-1-(2,4-dichloro-phenyl)-4,5 -dihydro-1H-pyrazole-3-carbonyl]-am ino}-piperidinium; | | |
| | 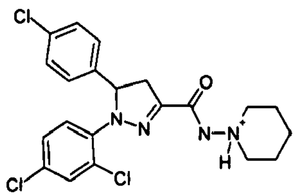 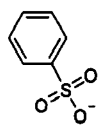 | Benzenesulfonate1-{[5-(4-chloro-phe nyl)-1-(2,4-dichloro-phenyl)-4,5-di hydro-1H-pyrazole-3-carbonyl]-amino }-piperidinium; | 3150, 1667, 1478, 1310, 1221, 1166, 1106, 999, 821, 757, 722, 692 | --- |
| | 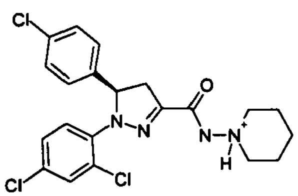 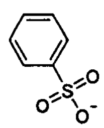 | Benzenesulfonatel-{[5(R)-(4-chloro-phe nyl)-1-(2,4-dichloro-phenyl)-4,5-di hydro-1H-pyrazole-3-carbonyl]-amino }-piperidinium; | | |

(continued)

| N° | STRUCTURE | Autonom® | FTIR (cm-1) | m.p. °C |
|---|---|---|---|---|
| | 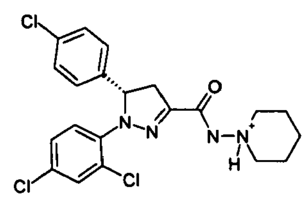 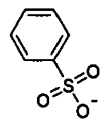 | Benzenesulfonate1-{[5(S)-(4-chloro-phe nyl)-1-(2,4-dichloro-phenyl)-4,5-di hydro-1H-pyrazole-3-carbonyl]-amino }-piperidinium; | | |
| | 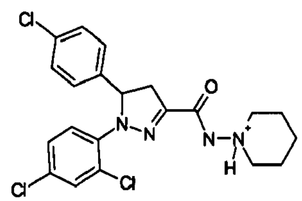 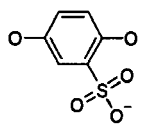 | 2,5-Dihydroxy-benzenesulfonate1-{[5 -(4-chloro-phenyl)-1-(2,4-dichloro-phenyl)-4,5-dihydro-1H-pyrazole-3-c arbonyl]-amino}-piperidinium; | | |
| | 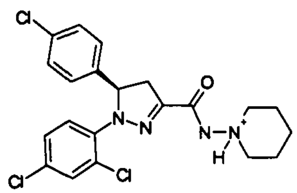 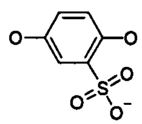 | 2,5-Dihydroxy-benzenesulfonate1-{[5 (R)-(4-chloro-phenyl)-1-(2,4-dichloro-phenyl)-4,5-dihydro-1H-pyrazole-3-c arbonyl]-amino}-piperidinium; | | |
| | 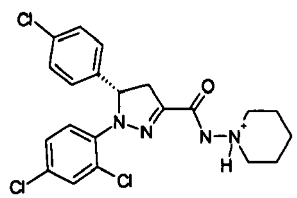 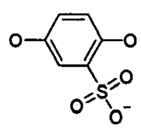 | 2,5-Dihydroxy-benzenesulfonate1-{[5 (S)-(4-chloro-phenyl)-1-(2,4-dichloro-phenyl)-4,5-dihydro-1H-pyrazole-3-c arbonyl]-amino}-piperidinium; | | |

(continued)

| N° | STRUCTURE | Autonom® | FTIR (cm-1) | m.p. °C |
|---|---|---|---|---|
| | 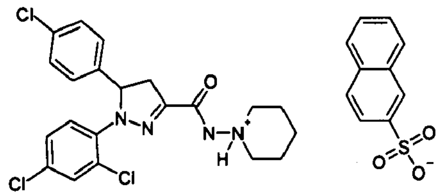 | Naphthalene-2-sulfonate1-{[5-(4-chl oro-phenyl)-1-(2,4-dichloro-phenyl) -4,5-dihydro-1H-pyrazole-3-carbonyl ]-amino}-piperidinium; | 3166, 1668, 1480, 1235, 1166, 1118, 1089, 1020, 822 and 670 | 169-174 °C |
| | 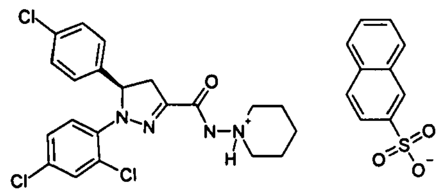 | Naphthalene-2-sulfonate1-{[5(R)-(4-chl oro-phenyl)-1-(2,4-dichloro-phenyl) -4,5-dihydro-1H-pyrazole-3-carbonyl ]-amino}-piperidinium; | | |
| | 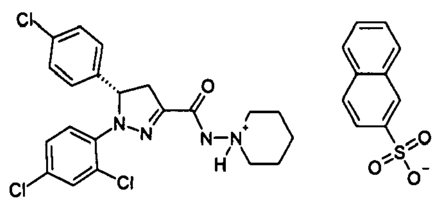 | Naphthalene-2-sulfonate1-{[5(S)-(4-chl oro-phenyl)-1-(2,4-dichloro-phenyl) -4,5-dihydro-1H-pyrazole-3-carbonyl ]-amino}-piperidinium; | | |
| | 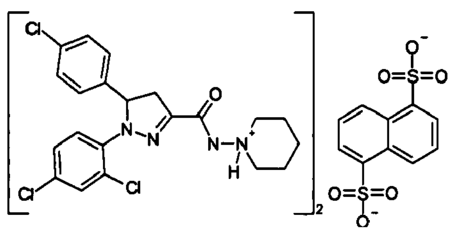 | 5-Sulfo-naphthalene-1-sulfonate1-{[ 5-(4-chloro-phenyl)-1-(2,4-dichloro -phenyl)-4,5-dihydro-1H-pyrazole-3-carbonyl]-amino}-piperidinium; | 1664, 1481, 1239, 1170, 1116, 1018, 826, 793 | |

(continued)

| N° | STRUCTURE | Autonom® | FTIR (cm-1) | m.p. °C |
|---|---|---|---|---|
| | 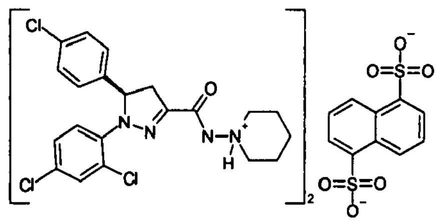 | 5-Sulfo-naphthalene-1-sulfonate1-{[ 5(R)-(4-chloro-phenyl)-1-(2,4-dichloro -phenyl)-4,5-dihydro-1H-pyrazole-3-carbonyl]-amino}-piperidinium; | | |
| | 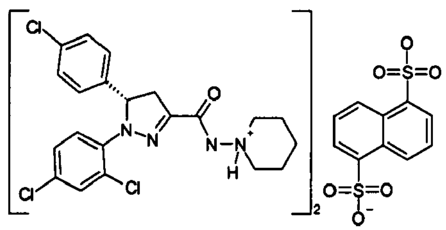 | 5-Sulfo-naphthalene-1-sulfonate1-{[ 5(S)-(4-chloro-phenyl)-1-(2,4-dichloro -phenyl)-4,5-dihydro-1H-pyrazole-3-carbonyl]-amino}-piperidinium; | | |
| | 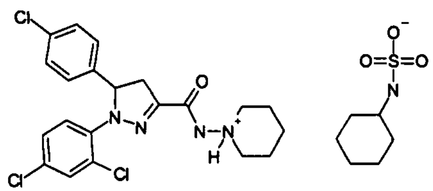 | Cyclamate1-{[5-(4-chl oro-phenyl)-1-(2,4-dichloro-phenyl) -4,5-dihydro-1H-pyrazole-3-carbonyl ]-amino}-piperidinium; | | |
| | 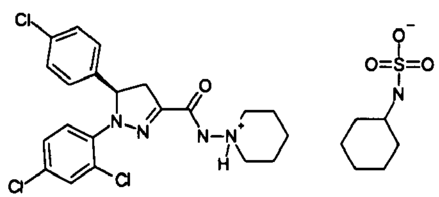 | Cyclamate1-{[5(R)-(4-chl oro-phenyl)-1-(2,4-dichloro-phenyl) -4,5-dihydro-1H-pyrazole-3-carbonyl ]-amino}-piperidinium; | | |

(continued)

| N° | STRUCTURE | Autonom® | FTIR (cm-1) | m.p. °C |
|---|---|---|---|---|
| | 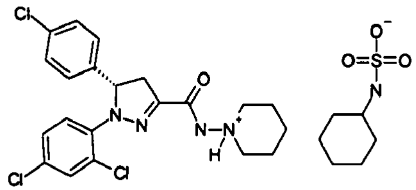 | Cyclamate1-{[5(S)-(4-chl oro-phenyl)-1-(2,4-dichloro-phenyl) -4,5-dihydro-1H-pyrazole-3-carbonyl ]-amino}-piperidinium; | | |
| | 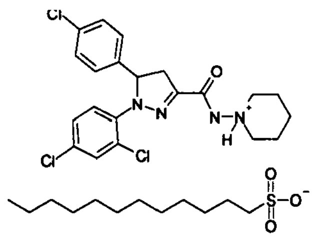 | Dodecane-1-sulfonate1-{[5-(4-chloro -phenyl)-1-(2,4-dichloro-phenyl)-4, 5-dihydro-1H-pyrazole-3-carbonyl]-a mino}-piperidinium; | | |
| | 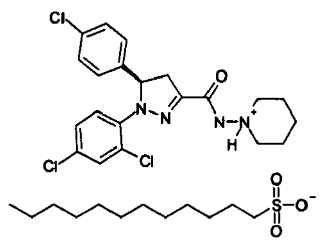 | Dodecane-1-sulfonate1-{[5(R)-(4-chloro -phenyl)-1-(2,4-dichloro-phenyl)-4, 5-dihydro-1H-pyrazole-3-carbonyl]-a mino}-piperidinium; | | |

(continued)

| N° | STRUCTURE | Autonom® | FTIR (cm-1) | m.p. °C |
|---|---|---|---|---|
| | 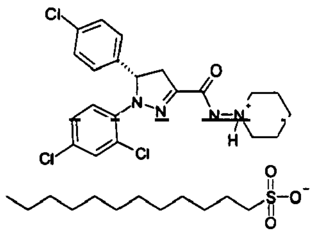 | Dodecane-1-sulfonate1-{[5(S)-(4-chloro -phenyl)-1-(2,4-dichloro-phenyl)-4, 5-dihydro-1H-pyrazole-3-carbonyl]-a mino}-piperidinium; | | |
| | 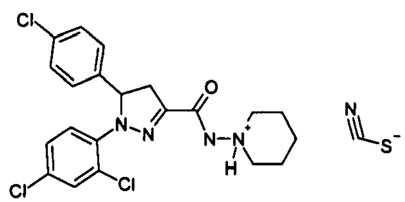 | Thiocyanate1-{[5-(4-chloro-p henyl)-1-(2,4-dichloro-phenyl)-4,5-dihydro-1H-pyrazole-3-carbonyl]-ami no}-piperidinium; | 3174, 2046, 1731, 1665, 1481, 1240, 1111 and 822 | – |
| | 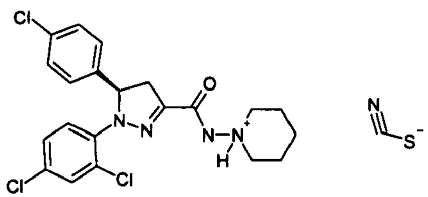 | Thiocyanate1-{[5(R)-(4-chloro-p henyl)-1-(2,4-dichloro-phenyl)-4,5-dihydro-1H-pyrazole-3-carbonyl]-ami no}-piperidinium; | | |
| | 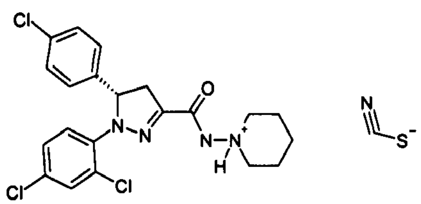 | Thiocyanate1-{[5(S)-(4-ch)oro-p henyl)-1-(2,4-dichloro-phenyl)-4,5-dihydro-1H-pyrazole-3-carbonyl]-ami no}-piperidinium; | | |

(continued)

| N° | STRUCTURE | Autonom® | FTIR (cm-1) | m.p. °C |
|---|---|---|---|---|
| | 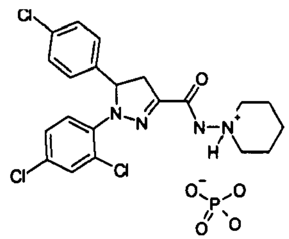 | 1-{[5-(4-Chloro-phenyl)-1-(2,4-dich loro-phenyl)-4,5-dihydro-1H-pyrazol e-3-carbonyl]-amino}-piperidinium; dihydrogen phosphate | | |
| | 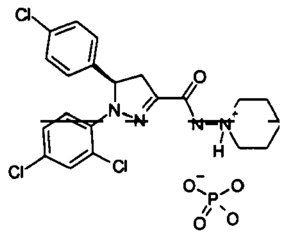 | 1-{[5(R)-(4-Chloro-phenyl)-1-(2,4-dich loro-phenyl)-4,5-dihydro-1H-pyrazol e-3-carbonyl]-amino}-piperidinium; dihydrogen phosphate | | |
| | 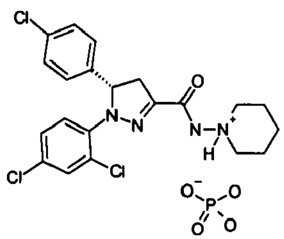 | 1-{[5(S)-(4-Chloro-phenyl)-1-(2,4-dich loro-phenyl)-4,5-dihydro-1H-pyrazol e-3-carbonyl]-amino}-piperidinium; dihydrogen phosphate | | |
| | 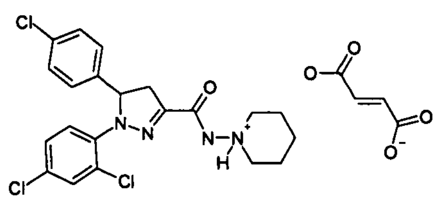 | 3-Carboxy-acrylate1-{[5-(4-ch)oro-p henyl)-1-(2,4-dichloro-phenyl)-4,5-dihydro-1H-pyrazole-3-carbonyl]-ami no}-piperidinium; | | |

(continued)

| N° | STRUCTURE | Autonom® | FTIR (cm-1) | m.p. °C |
|---|---|---|---|---|
| | 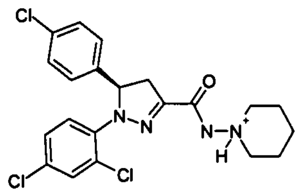 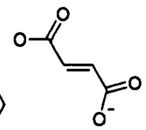 | 3-Carboxy-acrylate1-{[5(R)-(4-chloro-p henyl)-1-(2,4-dichloro-phenyl)-4,5-dihydro-1H-pyrazole-3-carbonyl]-ami no}-piperidinium; | | |
| | 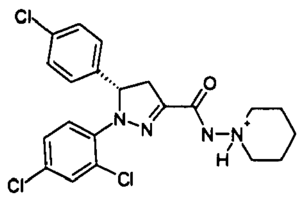 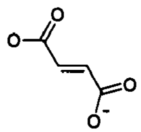 | 3-Carboxy-acrylate1-{[5(S)-(4-chloro-p henyl)-1-(2,4-dichloro-phenyl)-4,5-dihydro-1H-pyrazole-3-carbonyl]-ami no}-piperidinium; | | |
| | 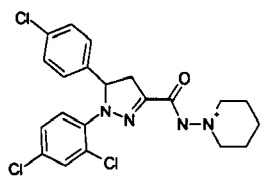 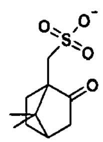 | (7,7-Dimethyl-2-oxo-bicyclo[2.2.1]h ept-1-yl)-methanesulfonate1-{[5-(4-chloro-phenyl)-1-(2,4-dichloro-phen yl)-4,5-dihydro-1H-pyrazole-3-carbo nyl]-amino}-piperidinium; | | |
| | 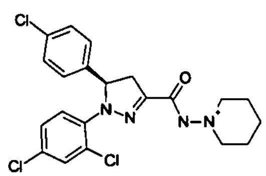 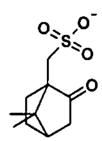 | (7,7-Dimethyl-2-oxo-bicyclo[2.2.1]h ept-1-yl)-methanesulfonate1-{[5(R)-(4-chloro-phenyl)-1-(2,4-dichloro-phen yl)-4,5-dihydro-1H-pyrazole-3-carbo nyl]-amino}-piperidinium; | | |

(continued)

| N° | STRUCTURE | Autonom® | FTIR (cm-1) | m.p. °C |
|---|---|---|---|---|
| | 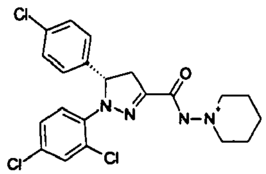 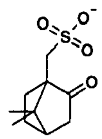 | (7,7-Dimethyl-2-oxo-bicyclo[2.2.1]h ept-1-yl)-methanesulfonate1-{[5(S)-(4-chloro-phenyl)-1-(2,4-dichloro-phen yl)-4,5-dihydro-1H-pyrazole-3-carbo nyl]-amino}-piperidinium; | | |

**Pharmacological Data:**

**I. In-vitro determination of affinity to $CB_1$/$CB_2$-Rezeptors**

The affinity of the inventive substituted pyrazoline compounds to $CB_1$/$CB_2$ receptors was determined as described above. Some of the values obtained are given in the following table I:

**Table I:**

| Compound according to Example | $CB_1$-Receptor Radioligand:[$^3$H]-CP55940 | | $CB_2$-Receptor Radioligand:[$^3$H]-CP55940 | |
|---|---|---|---|---|
| | % Inhibition $10^{-6}$ M | $K_i$(nM) | % Inhibition $10^{-6}$ M | $K_i$(nM) |
| 1 | 93 % | < 25 | 33 % | > 1000 |
| 5 | 79 % | 111 | 54 % | ≈ 1000 |

As can be seen from the values given in table 1 the inventive pyrazoline compounds are particularly suitable for regulating the $CB_1$-Receptor.

Other results obtained are shown in Table Ia:

**Table Ia**

| Compound | $CB_1$-Receptor Radioligand: [$^3$H]-SR141716A |
|---|---|
| | % Inhibition $10^{-7}$ M |
| 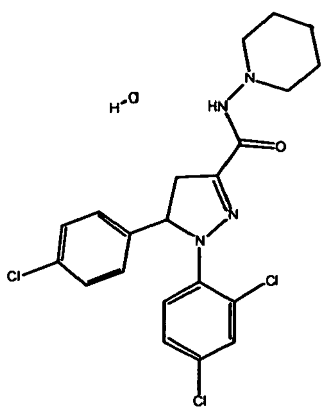 | 82.6 |
| 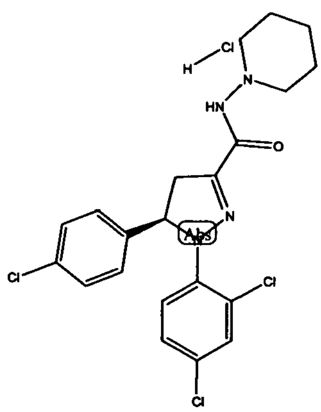 | 82.7 |

(continued)

| Compound | $CB_1$-Receptor Radioligand: $[^3H]$-SR141716A |
|---|---|
| | % Inhibition $10^{-7}$ M |
| 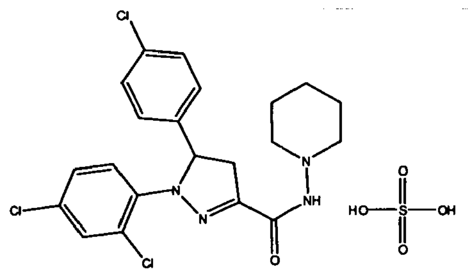 | 99.7 |
| 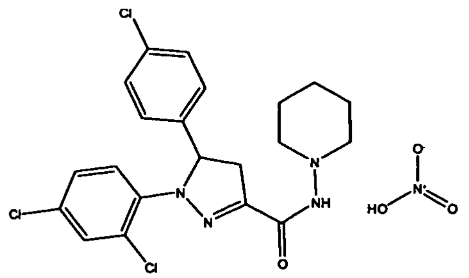 | 83.6 |
| 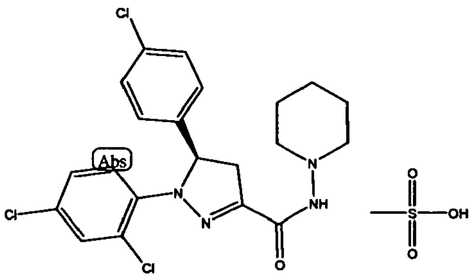 | 83.3 |

**II. In-vivo bioassay system for determination of cannabinoid activity**

The determinination of cannabinoid activity in-vivo was determined as described above. Some of the values obtained are given in the following table II:

Table II:

| Compound according to example: | dosis administered: 5 mg/kg i.v. Agonistic effect | | | | dosis administered 5 mg/kg i.v. prior to Win 55212-2 in a dose of 1,25mg/kg i.v. Antagonistic Effect | | | |
|---|---|---|---|---|---|---|---|---|
| | A | B | C. | D | A | B. | C | D |
| 1 | 0 | 0 | 0 | 0 | 74 | 100 | 100 | 100 |
| 5 | 0 | 50 | 0 | 0 | 50 | 40 | 20 | 20 |

i.v. intravenous
A: Hot-Plate test
B: Hypothermia
C: Catalepsy
D: Sedation

As can be seen from the values given in table II the inventive pyrazoline compounds show an antagonistic effect.

**III. In-vivo testing for antiobesic activity**

The in-vivo testing for antiobesic activity was carried out as described above, whereby four different groups of 10 rats each were treated as follows:

**Group I:**

Group was treated with vehicle, namely arabic gum (5 wt.-%) in water.

**Group II:**

The second group of rats was treated with the inventive compound N-piperidinyl-5-(4-chlorophenyl)-1-(2,4-dichlorophenyl)-4,5-dihydropyrazole-3-carboxamide according to Example 1. Said compound was administered intraperitoneally to the rats over a period of 14 days in a daily dosis of (10 mg/kg body weight).

**Group III:**

The third group of rats was treated with Amphetamine, an active ingredient known to reduce appetite. Said compound was administered intraperitoneally to the rats over a period of 14 days in a daily dosis of (5 mg/kg body weight).
As can be seen from Figure 1 the body weight is lowered due to the administration of the inventive compound according to example 1 and this effect is also observed after the treatment is ended.
Figure 2 shows the reduction of food intake due to the administration of the inventive compound according to example 1.

**IV. In vivo testing for antidepressant activity**

The in-vivo testing for antidepressant activity of the inventive pyrazoline compounds in the water despair test was carried out as described above. In particular, the compound according to example 1 displayed positive effects with respect to immobility time and struggling time.

## Claims

**1.** Salt of a substituted pyrazoline compound of general formula I,

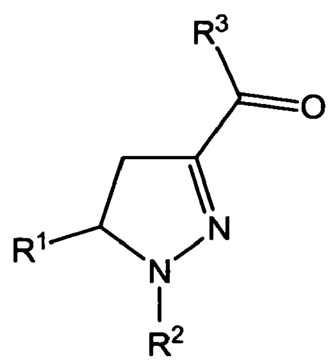

I

wherein

$R^1$ represents an optionally at least mono-substituted phenyl group;
$R^2$ represents an optionally at least mono-substituted phenyl group;

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system; or $R^3$ represents an optionally at least mono-substituted aryl or heteroaryl group, which may be condensed with an optionally at least mono-substituted mono- or polycyclic ring system; or $R^3$ represents an —$NR^4R^5$-moiety,

$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an —$SO_2$-$R^6$-moiety; or an - $NR^7R^8$-moiety,

with the provisos

that $R^4$ and $R^5$ do not both represent a hydrogen atom; and

that if one of the residues $R^4$ and $R^5$ represents a hydrogen atom or an alkyl, group, which is optionally at least mono-substituted with an alkoxy group, an alkoxyalkoxy group, a halogen atom or a phenyl group, the other one of these residues $R^4$ and $R^5$ does not represent a pyrid-2-yl group, which is optionally mono-substituted in the 5-position; a pyrid-5-yl group, which is optionally mono-substituted in the 2-position; a pyrimid-5-yl group, which is optionally mono-substituted in the 2-position; a pyridaz-3-yl group, which is optionally mono-substituted in the 6-position; a pyrazin-5-yl group, which is optionally mono-substituted in the 2-position; a thien-2-yl group, which is optionally mono-substituted in the 5 position; a thien-2-yl group, which is optionally at least mono-substituted in the 4-position; a benzyl group, which is optionally mono-substituted in the 4-position of the ring; a phenethyl group, which is optionally mono-substituted in the 4-position of the ring; an optionally mono-, di- or tri-substituted phenyl group; a di-substituted phenyl group, wherein the two substituents together form an —$OCH_2O$-, -$OCH_2CH_2O$- or -$CH_2CH_2O$-chain, which is optionally substituted with one or more halogen atoms or one or two methyl groups; an —NH-phenyl-moiety, wherein the phenyl group may be mono-substituted in the 4-position, and

that if one of the residues $R^4$ and $R^5$ represents an alkynyl group, the other one of these residues $R^4$ and $R^5$ does not represent a phenyl group, which is optionally substituted in the 4-position, and

that if one of the residues $R^4$ and $R^5$ represents a hydrogen atom or a linear or branched, saturated or unsaturated, unsubstituted or substituted aliphatic radical, the other one of these residues $R^4$ and $R^5$ does not represent an unsubstituted or substituted thiazole group or an unsubstituted or substituted [1,3,4]thiadiazole group;

$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;

$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof,

with an acid with a $pk_a \leq 3.0$

with the proviso that the acid is not selected from hydrochloric acid, hydrobromic acid, phosphoric acid, sulphuric acid, nitric acid, citric acid, maleic acid, fumaric acid, tartaric acid, p-toluenesulfonic acid, methanesulfonic acid or camphersulfonic acid,

optionally in form of a corresponding solvate thereof.

2. Salt according to claim 1, **characterized in that** the substituted pyrazoline compound of general formula I, has a general formula according to general formula Ia or Ib

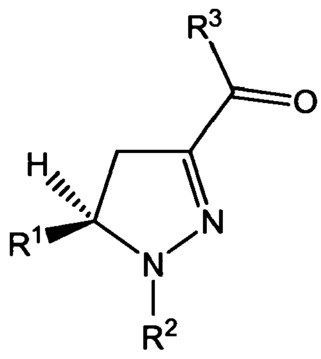

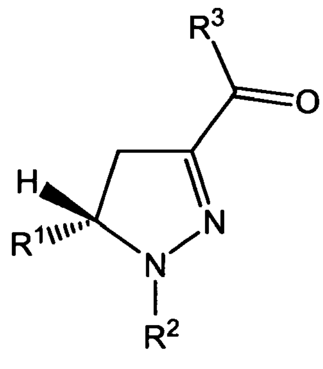

Ia Ib

3. Salt according to any of claim 1 or 2, **characterized in that** $R^1$ in general formula I, Ia or Ib represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R'', -(C=O)-$NH_2$, -(C=O)-NHR' and -(C=O)-NR'R'' whereby R' and R'' for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is mono-substituted with a chlorine atom in the 4-position.

4. Salt according to any of claim 1 to 3, **characterized in that** $R^2$ in general formula I, Ia or Ib represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R'', -(C=O)-$NH_2$, -(C=O)-NHR' and -(C=O)-NR'R'', whereby R' and optionally R'' for each substituent independently represent linear or branched $C_{1-6}$ alkyl, preferably $R^2$ represents a phenyl group, which is optionally substituted by one or more substituents independently selected from the group consisting of methyl, ethyl, F, Cl, Br and $CF_3$, more preferably $R^2$ represents a phenyl group, which is di-substituted with two chlorine atoms in its 2- and 4-position.

5. Salt according to one or more of claims 1-4 **characterized in that** $R^3$ in general formula I, Ia or Ib represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an -$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an —$NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an —$NR^4R^5$-moiety.

6. Salt according to one or more of claims 1-5, **characterized in that** $R^4$ and $R^5$, in general formula I, Ia or Ib, identical or different, represent a hydrogen atom; an unbranched or branched, saturated or unsaturated, optionally at least mono-substituted $C_{1-6}$-aliphatic radical; a saturated or unsaturated, optionally at least mono-substituted, optionally at least one heteroatom as ring member containing $C_{3-8}$-cycloaliphatic group, which may be condensed with an optionally at least mono-substituted mono- or polycydic ring system; or an optionally at least mono-substituted, 5- or 6-membered aryl or heteroaryl group, which may be condensed with an optionally at least mono-substituted mono- or polycyclic ring system and/or bonded via a methylene (-$CH_2$-) or ethylene (-$CH_2$-$CH_2$)-group; an —$SO_2$-$R^6$-moiety; or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least

mono-substituted mono- or polycyclic ring system; an —$SO_2$-$R^6$-moiety; or an -$NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an —$SO_2$-$R^6$-moiety; or an - $NR^7R^8$-moiety, or $R^4$ and $R^5$, identical or different, represent a methyl group, an ethyl group, an n-propyl group, an isopropyl group, an n-butyl group, a sec-butyl group or a tert.-butyl group.

**7.** Salt according to one or more of claims 1-6, **characterized in that** $R^6$ in general formula I, Ia or Ib represents a linear or branched, saturated or unsaturated, optionally at least mono-substituted $C_{1-6}$ aliphatic group; a saturated or unsaturated, optionally at least mono-substituted, optionally at least one heteroatom as ring member containing $C_{3-8}$ cycloaliphatic group, which may be condensed with a mono- or polycyclic ring-system; or an optionally at least mono-substituted, 5- or 6-membered aryl or heteroaryl group, which may be condensed with a mono- or polycyclic ring system and/or bonded via a methylene (-$CH_2$-) or ethylene (-$CH_2$-$CH_2$)-group, preferably $R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups.

**8.** Salt according to one or more of claims 1-7, **characterized in that** $R^7$ and $R^8$, in general formula I, Ia or Ib, identical or different, represent a hydrogen atom; an unbranched or branched, saturated or unsaturated, optionally at least mono-substituted $C_{1-6}$ aliphatic radical; a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system; or an optionally at least mono-substituted, 5- or 6 membered aryl or heteroaryl group, which may be condensed with an optionally at least mono-substituted mono- or polycyclic ring system and/or bonded via a methylene (-$CH_2$-) or ethylene (-$CH_2$-$CH_2$)-group, preferably $R^7$ and $R^8$, identical or different, represent a hydrogen atom; or a $C_{1-6}$ alkyl radical.

**9.** Salt according to one or more of claims 1 to 8, **characterized in that** the compound according to general formula I, Ia or Ib is a compound

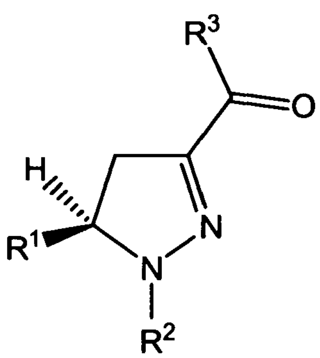
Ia

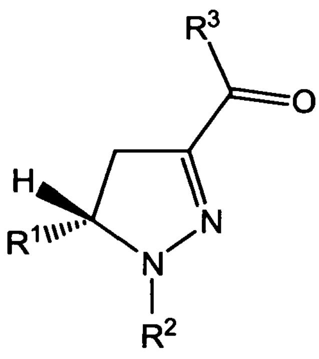
Ib

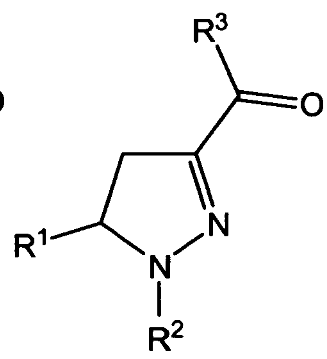
I

wherein

$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group; a piperidinyl group; a piperazinyl group; a homo-piperazinyl group; a morpholinyl group; or an —$NR^4R^5$-moiety,
$R^4$ represents a hydrogen atom or a linear or branched $C_{1-6}$-alkyl group,
$R^5$ represents a linear or branched $C_{1-6}$ alkyl group; an $SO_2R^6$-moiety; a pyrrolidinyl group; a piperidinyl group; a piperazinyl group; a homo-piperazinyl group; a morpholinyl group; or a triazolyl group, whereby each of the

heterocyclic rings may be substituted with one or more, identical or different, $C_{1-6}$-alkyl groups, and $R^6$ represents a phenyl group, which is optionally substituted with one or more $C_{1-6}$ alkyl groups, which may be identical or different,
optionally in form of a racemate or in form of a mixture of at least two of the stereoisomers, preferably enantiomers and/or diastereomers, in any mixing ratio, or a corresponding N-oxide thereof, or a corresponding solvate thereof.

**10.** Salt according to one or more of claims 1 to 9, **characterized in that** the compound general formula I, Ia or Ib is selected from the group consisting of:

N-piperidinyl-5-(4-chloro-phenyl)-1-(2,4-dichlorophenyl)-4,5-dihydro-1H-pyrazol-3-carboxamide,
(rac)-N-pipeddinyl-5-(4-chloro-phenyl)-1-(2,4-dichlorophenyl)-4,5-dihydro-1H-pyrazol-3-carboxamide,
(R)-N-piperidinyl-5-(4-chloro-phenyl)-1-(2,4-dichlorophenyl)-4,5-dihydro-1H-pyrazol-3-carboxamide,
(S)-N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsulfonamide,
1-{[5-(4-Chloro-phenyl)-1-(2,4-dichloro-phenyl)-4,5-dihydro-1H-pyrazole-3-carbonyl]-amino}-piperidinium; chloride,
1-{[5(R)-(4-Chloro-phenyl)-1-(2,4-dichloro-phenyl)-4,5-dihydro-1H-pyrazole-3-carbonyl]-amino}-piperidinium; chloride,
1-{[5(S)-(4-Chloro-phenyl)-1-(2,4-dichloro-phenyl)-4,5-dihydro-1H-pyrazole-3-carbonyl]-amino}-piperidinium; chloride
1-{[5-(4-Chloro-phenyl)-1-(2,4-dichloro-phenyl)-4,5-dihydro-1H-pyrazole-3-carbonyl]-amino}-piperidinium; nitrate,
1-{[5(R)-(4-Chloro-phenyl)-1-(2,4-dichloro-phenyl)-4,5-dihydro-1H-pyrazole-3-carbonyl]-amino}-piperidinium; nitrate,
1-{[5(S)-(4-Chloro-phenyl)-1-(2,4-dichloro-phenyl)-4,5-dihydro-1H-pyrazole-3-carbonyl]-amino}-piperidinium; nitrate,
1-{[5-(4-Chloro-phenyl)-1-(2,4-dichloro-phenyl)-4,5-dihydro-1H-pyrazole-3-carbonyl]-amino}-piperidinium; hydrogen sulfate,
1-{[5(R)-(4-Chloro-phenyl)-1-(2,4-dichloro-phenyl)-4,5-dihydro-1H-pyrazole-3-carbonyl]-amino}-piperidinium; hydrogen sulfate,
1-{[5(S)-(4-Chloro-phenyl)-1-(2,4-dichloro-phenyl)-4,5-dihydro-1H-pyrazole-3-carbonyl]-amino}-piperidinium; hydrogen sulfate,
Methanesulfonate1-{[5-(4-chloro-phenyl)-1-(2,4-dichloro-phenyl)-4,5-dihydro-1 H-pyrazole-3-carbonyl]-amino}-piperidinium,
Methanesulfonate1-{[5(R)-(4-chloro-phenyl)-1-(2,4-dichloro-phenyl)-4,5-dihydro-1H-pyrazole-3-carbonyl]-amino}-piperidinium,
Methanesulfonate1-{[5(S)-(4-chloro-phenyl)-1-(2,4-dichloro-phenyl)-4,5-dihydro-1H-pyrazole-3-carbonyl]-amino}-piperidinium,
1-{[5-(4-Chloro-phenyl)-1-(2,4-dichloro-phenyl)-4,5-dihydro-1H-pyrazole-3-carbonyl]-amino}-piperidinium; bromide,
1-{[5(R)-(4-Chloro-phenyl)-1-(2,4-dichloro-phenyl)-4,5-dihydro-1H-pyrazole-3-carbonyl]-amino}-piperidinium; bromide,
1-{[5(S)-(4-Chloro-phenyl)-1-(2,4-dichloro-phenyl)-4,5-dihydro-1H-pyrazole-3-carbonyl]-amino}-piperidinium; bromide,
Ethanesulfonate1-{[5-(4-chloro-phenyl)-1-(2,4-dichloro-phenyl)-4,5-dihydro-1 H-pyrazole-3-carbonyl]-amino}-piperidinium,
Ethanesulfonate1-{[5(R)-(4-chloro-phenyl)-1-(2,4-dichloro-phenyl)-4,5-dihydro-1 H-pyrazole-3-carbonyl]-amino}-piperidinium,
Ethanesulfonate1-{[5(S)-(4-chloro-phenyl)-1-(2,4-dichloro-phenyl)-4,5-dihydro-1 H-pyrazole-3-carbonyl]-amino}-piperidinium,
Toluene-4-sulfonate1-{[5-(4-chloro-phenyl)-1-(2,4-dichloro-phenyl)-4,5-dihydro-1H-pyrazole-3-carbonyl]-amino}-piperidinium,
Toluene-4-sulfonate1-{[5(R)-(4-chloro-phenyl)-1-(2,4-dichloro-phenyl)-4,5-dihydro-1H-pyrazole-3-carbonyl]-amino}-piperidinium,
Toluene-4-sulfonate1-{[5(S)-(4-chloro-phenyl)-1-(2,4-dichloro-phenyl)-4,5-dihydro-1H-pyrazole-3-carbonyl]-amino}-piperidinium,
Benzenesulfonate1-{[5-(4-chloro-phenyl)-1-(2,4-dichloro-phenyl)-4,5-dihydro-1 H-pyrazole-3-carbonyl]-amino}-piperidinium,
Benzenesulfonate1-{[5(R)-(4-chloro-phenyl)-1-(2,4-dichloro-phenyl)-4,5-dihydro-1H-pyrazole-3-carbonyl]-amino}-piperidinium,
Benzenesulfonate1-{[5(S)-(4-chloro-phenyl)-1-(2,4-dichloro-phenyl)-4,5-dihydro-1H-pyrazole-3-carbonyl]-amino}-piperidinium,
2,5-Dihydroxy-benzenesulfonate1-{[5-(4-chloro-phenyl)-1-(2,4-dichlorophenyl)-4,5-dihydro-1H-pyrazole-3-carbonyl]-amino}-piperidinium,
2,5-Dihydroxy-benzenesulfonate1-{[5(R)-(4-chloro-phenyl)-1-(2,4-dichlorophenyl)-4,5-dihydro-1H-pyrazole-3-carbonyl]-amino}-piperidinium,
2,5-Dihydroxy-benzenesulfonate1-{[5(S)-(4-chloro-phenyl)-1-(2,4-dichlorophenyl)-4,5-dihydro-1H-pyrazole-3-carbonyl]-amino}-piperidinium,
Naphthalene-2-sulfonate1-{[5-(4-chloro-phenyl)-1-(2,4-dichloro-phenyl)-4,5-dihydro-1H-pyrazole-3-carbonyl]-amino}-piperidinium,
Naphthalene-2-sulfonate1-{[5(R)-(4-chloro-phenyl)-1-(2,4-dichloro-phenyl)-4,5-dihydro-1H-pyrazole-3-carbonyl]-amino}-piperidinium,
Naphthalene-2-sulfonate1-{[5(S)-(4-chloro-phenyl)-1-(2,4-dichloro-phenyl)-4,5-dihydro-1H-pyrazole-3-carbonyl]-amino}-piperidinium,
5-Sulfo-naphthalene-1-sulfonate1-{[5-(4-chloro-phenyl)-1-(2,4-dichlorophenyl)-4,5-dihydro-1H-pyrazole-3-carbonyl]-amino}-piperidinium,
5-Sulfo-naphthalene-1-sulfonate1-{[5(R)-(4-chloro-phenyl)-1-(2,4-dichlorophenyl)-4,5-dihydro-1H-pyrazole-3-carbonyl]-amino}-piperidinium,
5-Sulfo-naphthalene-1-sulfonate1-{[5(S)-(4-chloro-phenyl)-1-(2,4-dichlorophenyl)-4,5-dihydro-1H-pyrazole-3-carbonyl]-amino}-piperidinium,
Cyclamate1-{[5-(4-chloro-phenyl)-1-(2,4-dichloro-phenyl)-4,5-dihydro-1H-pyrazole-3-carbonyl]-amino}-piperidinium,
Cyclamate1-{[5(R)-(4-chloro-phenyl)-1-(2,4-dichloro-phenyl)-4,5-dihydro-1H-pyrazole-3-carbonyl]-amino}-piperidinium,
Cyclamate1-{[5(S)-(4-chloro-phenyl)-1-(2,4-dichloro-phenyl)-4,5-dihydro-1H-pyrazole-3-carbonyl]-amino}-piperidinium,
Dodecane-1-sulfonate1-{[5-(4-chloro-phenyl)-1-(2,4-dichloro-phenyl)-4,5-dihydro-1H-pyrazole-3-carbonyl]-amino}-piperidinium,
Dodecane-1-sulfonate1-{[5(R)-(4-chloro-phenyl)-1-(2,4-dichloro-phenyl)-4,5-dihydro-1 H-pyrazole-3-carbonyl]-amino}-piperidinium,
Dodecane-1-sulfonate1-{[5(S)-(4-chloro-phenyl)-1-(2,4-dichloro-phenyl)-4,5-dihydro-1H-pyrazole-3-carbonyl]-amino}-piperidinium,
Thiocyanate1-{[5-(4-chloro-phenyl)-1-(2,4-dichloro-phenyl)-4,5-dihydro-1H-pyrazole-3-carbonyl]-amino}-piperidinium,
Thiocyanate1-{[5(R)-(4-chloro-phenyl)-1-(2,4-dichloro-phenyl)-4,5-dihydro-1H-pyrazole-3-carbonyl]-amino}-piperidinium,
Thiocyanate1-{[5(S)-(4-chloro-phenyl)-1-(2,4-dichloro-phenyl)-4,5-dihydro-1H-pyrazole-3-carbonyl]-amino}-piperidinium,
1-{[5-(4-Chloro-phenyl)-1-(2,4-dichloro-phenyl)-4,5-dihydro-1H-pyrazole-3-carbonyl]-amino}-piperidinium; dihydrogen phosphate,
1-{[5(R)-(4-Chloro-phenyl)-1-(2,4-dichloro-phenyl)-4,5-dihydro-1H-pyrazole-3-carbonyl]-amino}-piperidinium; dihydrogen phosphate,
1-{[5(S)-(4-Chloro-phenyl)-1-(2,4-dichloro-phenyl)-4,5-dihydro-1H-pyrazole-3-carbonyl]-amino}-pipeddinium; dihydrogen phosphate,
3-Carboxy-acrylate1-{[5-(4-chloro-phenyl)-1-(2,4-dichloro-phenyl)-4,5-dihydro-1 H-pyrazole-3-carbonyl]-amino}-piperidinium,
3-Carboxy-acrylate1-{[5(R)-(4-chloro-phenyl)-1-(2,4-dichloro-phenyl)-4,5-dihydro-1H-pyrazole-3-carbonyl]-amino}-piperidinium,
3-Carboxy-acrylate1-{[5(S)-(4-chloro-phenyl)-1-(2,4-dichloro-phenyl)-4,5-dihydro-1H-pyrazole-3-carbonyl]-amino}-piperidinium,
(7,7-Dimethyl-2-oxo-bicyclo[2.2.1]hept-1-yl)-methanesulfonate1-{[5-(4-chlorophenyl)-1-(2,4-dichloro-phenyl)-4,5-dihydro-1H-pyrazole-3-carbonyl]-amino}-piperidinium,
(7,7-Dimethyl-2-oxo-bicyclo[2.2.1]hept-1-yl)-methanesulfonate1-{[5(R)-(4-chloro-phenyl)-1-(2,4-dichloro-phenyl)-4,5-dihydro-1H-pyrazole-3-carbonyl]-amino}-piperidinium,
(7,7-Dimethyl-2-oxo-bicyclo[2.2.1]hept-1-yiymethanesulfonatel-{[5(S)-(4-chloro-phenyl)-1-(2,4-dichloro-phenyl)-4,5-dihydro-1H-pyrazole-3-carbonyl]-amino}-piperidinium,
optionally in the form of a corresponding N-oxide or a corresponding solvate.

**11.** Salt according to one or more of daims 1 to 10, **characterized in that** the salt is pharmaceutically acceptable.

**12.** Salt according to one or more of claims 1 to 11, **characterized in that** the acid is an inorganic acid and has a $pk_a \leq 2.0$, preferably ≤ 1.5, more preferably ≤ 1.0, most preferably ≤ 0.7.

**13.** Salt according to one or more of claims 1 to 11, **characterized in that** the acid is an organic acid and has a $pk_a \leq 1.5$, preferably ≤ 1.0, more preferably ≤ 0.7.

**14.** Salt according to one or more of claims 1 to 13, **characterized in that** the acid is selected from:

- naphthalene-1,5-disulfonic acid
- ethanesulfonic acxid
- thiocyanic acid
- 2-naphthalenesulfonic acid or
- benzenesulfonic acid
- hydrobromic acid
- hydrochloric acid
- sulfuric acid
- ethane-1,2-disulfonic acid
- cyclohexylsulfamic acid
- p-toluenesulfonic acid
- methanesulfonic acid
- dodecylsulfuric acid
- benzenesulfonic acid
- nitric acid

**15.** Process for the manufacture of a salt of a substituted pyrazoline compounds of general formula I according to one or more of claims 1 to 14, **characterized in that** at least one benzaldehyde compound of general formula II

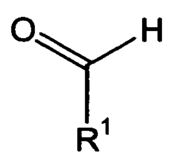

(II)

wherein $R^1$ has the meaning according to one or more of claims 1-9, is reacted with a pyruvate compound of general formula (III)

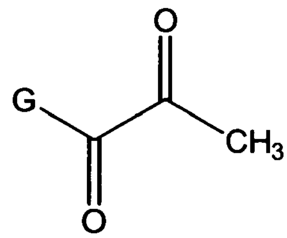

(III),

wherein G represents an OR group with R being a branched or unbranched $C_{1-6}$ alkyl radical or G represents an $O^-K$ group with K being a cation, to yield a compound of general formula (IV)

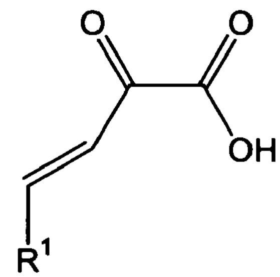

wherein $R^1$ has the meaning given above, which is optionally isolated and/or optionally purified, and which is reacted with an optionally substituted phenyl hydrazine of general formula (V)

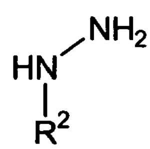

(V)

or a corresponding salt thereof, wherein $R^2$ has the meaning according to one or more of claims 1-9, under inert atmosphere, to yield a compound of general formula (VI)

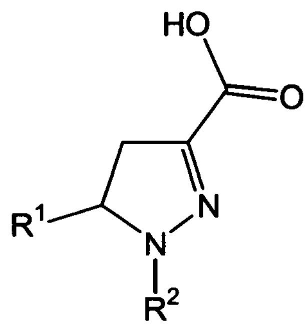

(VI)

wherein $R^1$ and $R^2$ have the meaning as given above, which is optionally isolated and/or optionally purified, and optionally transferred under inert atmosphere to a compound of general formula (VII) via the reaction with an activating agent

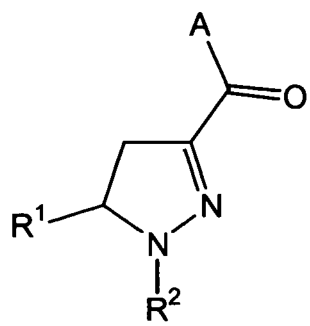

(VII)

wherein the substituents $R^1$ and $R^2$ have the meaning given above and A represents a leaving group, said compound being optionally isolated and/or optionally purified, and at least one compound of general formula (VI) is reacted with a compound of general formula $R^3H$, wherein $R^3$ represents an — $NR^4R^5$-moiety, with $R^4$ and $R^5$ having the meaning according to one or more of claims 1-9, under inert atmosphere to yield a substituted pyrazoline compound of general formula I, wherein $R^3$ represents an —$NR^4R^5$-moiety,
or at least one compound of general formula (VII) is reacted with a compound of the general formula $R^3H$, in which $R^3$ has the meaning according to one or more of claims 1-9 under inert atmosphere to yield a compound of general formula (I) according to one or more of claims 1-10, which is optionally isolated and/or optionally purified,
wherein the enantiomers according to general formula Ia or Ib of a compound of general formula I are optionally formed by either separating the enantiomers chromatographically or by reacting the compound of general formula I with a chiral base,
wherein the compound of general formula I or Ia or Ib is reacted with an acid with a pka $\leq 3.0$ to form a salt, according to one or more of claims 1-10, which is optionally isolated and/or optionally purified .

**16.** Medicament comprising at least one salt of a substituted pyrazoline compound of general formula I,

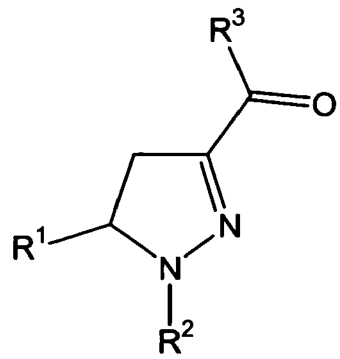

wherein

$R^1$ represents an optionally at least mono-substituted phenyl group;
$R^2$ represents an optionally at least mono-substituted phenyl group;
$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an $-NR^4R^5$-moiety,
$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an $-SO_2-R^6$-moiety; or an - $NR^7R^8$-moiety, with the proviso that $R^4$ and $R^5$ do not identically represent hydrogen;
$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono- or polycyclic ring system and/or bonded via a linear or branched alkylene group;
$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof,
with an acid with a $pk_a \leq 3.0$
with the proviso that the acid is not selected from hydrochloric acid, hydrobromic acid, phosphoric acid, sulphuric acid, nitric acid, citric acid, maleic acid, fumaric acid, tartaric acid, p-toluenesulfonic acid, methanesulfonic acid or camphersulfonic acid,
optionally in form of a corresponding solvate thereof,
and optionally one or more pharmaceutically acceptable excipients.

**17.** Medicament according to claim 16, **characterized in that** $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R'' whereby R' and R" for each substituent independently represent linear or branched $C_{1-6}$ alkyl, preferably $R^1$ represents a phenyl group, which is optionally substituted by one or more substituents selected from the group consisting of methyl, ethyl, F,

Cl, Br and $CF_3$, more preferably $R^1$ represents a phenyl group, which is mono-substituted with a chlorine atom in the 4-position.

**18.** Medicament according to claim 16 or 17, **characterized in that** $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R", -(C=O)-$NH_2$, -(C=O)-NHR' and -(C=O)-NR'R", whereby R' and optionally R" for each substituent independently represent linear or branched $C_{1-6}$ alkyl, preferably $R^2$ represents a phenyl group, which is optionally substituted by one or more substituents independently selected from the group consisting of methyl, ethyl, F, Cl, Br and $CF_3$, more preferably $R^2$ represents a phenyl group, which is di-substituted with two chlorine atoms in its 2- and 4-position.

**19.** Medicament according to one or more of claims 16-18, **characterized in that** $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an —$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an — $NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an —$NR^4R^5$-moiety.

**20.** Medicament according to one or more of claims 16-19, **characterized in that** $R^4$ and $R^5$, identical or different, represent a hydrogen atom; an unbranched or branched, saturated or unsaturated, optionally at least mono-substituted $C_{1-6}$-aliphatic radical; a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system and/or bonded via a methylene (-$CH_2$-) or ethylene (-$CH_2$-$CH_2$)-group; an —$SO_2$-$R^6$-moiety; or an -$NR^7R^8$-moiety, preferably one of these residues $R^4$ and $R^5$ represents a hydrogen atom and the other one of these residues $R^4$ and $R^5$ represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system; or an optionally at least mono-substituted, 5- or 6-membered aryl or heteroaryl group, which may be condensed with an optionally at least mono-substituted mono- or polycyclic ring system; an -$SO_2$-$R^6$-moiety; or an -$NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an -$SO_2$-$R^6$-moiety; or an - $NR^7R^8$-moiety, or $R^4$ and $R^5$, identical or different, represent a methyl group, an ethyl group, an n-propyl group, an isopropyl group, an n-butyl group, a sec-butyl group or a tert.-butyl group.

**21.** Medicament according to one or more of claims 16-20, **characterized in that** $R^6$ represents a linear or branched, saturated or unsaturated, optionally at least mono-substituted $C_{1-6}$ aliphatic group; a saturated or unsaturated, optionally at least mono-substituted, optionally at least one heteroatom as ring member containing $C_{3-8}$ cycloaliphatic group, which may be condensed with a mono- or polycyclic ring-system; or an optionally at least mono-substituted, 5- or 6-membered aryl or heteroaryl group, which may be condensed with a mono- or polycyclic ring system and/or bonded via a methylene (-$CH_2$-) or ethylene (-$CH_2$-$CH_2$)-group, preferably $R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups.

**22.** Medicament according to one or more of claims 16-21, **characterized in that** $R^7$ and $R^8$, identical or different, represent a hydrogen atom; an unbranched or branched, saturated or unsaturated, optionally at least mono-substituted $C_{1-6}$ aliphatic radical; a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system; or an optionally at least mono-substituted, 5- or 6 membered aryl or heteroaryl group, which may be condensed with an optionally at least mono-substituted mono- or

polycyclic ring system and/or bonded via a methylene ($-CH_2-$) or ethylene ($-CH_2-CH_2$)-group, preferably $R^7$ and $R^8$, identical or different, represent a hydrogen atom or a $C_{1-6}$ alkyl radical.

**23.** Medicament according to one or more of claims 16-22, **characterized in that** it comprises at least one salt of a compound of general formula I, Ia or Ib

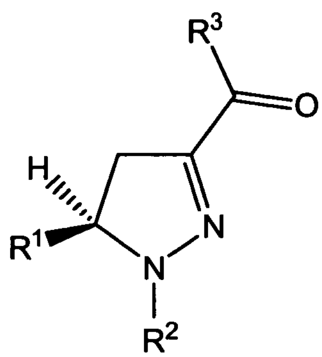
Ia

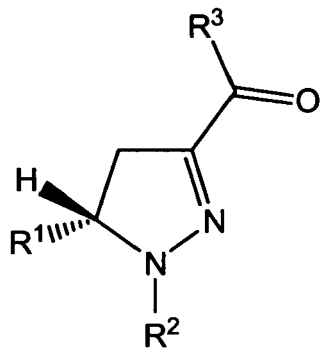
Ib

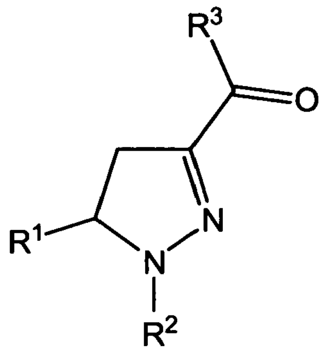
I

wherein

$R^1$ represents a phenyl group, which is optionally substituted by one or more substituents independently selected from the group consisting of methyl, ethyl, F, Cl, Br and $CF_3$,
$R^2$ represents a phenyl group, which is optionally substituted by one or more substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more $C_{1-6}$-alkyl groups, or $R^3$ represents an $—NR^4R^5$-moiety,
one of the residues $R^4$ and $R^5$ represents a hydrogen atom and the other one of these residues $R^4$ and $R^5$ represents an optionally at least mono-substituted pyrrolidinyl group; an optionally at least mono-substituted piperidinyl group; an optionally at least mono-substituted piperazinyl group; an optionally at least mono-substituted triazolyl group; an $—SO_2-R^6$-moiety; or an $-NR^7R^8$-moiety, or $R^4$ and $R^5$, identical or different, represent a methyl group, an ethyl group, an n-propyl group, an isopropyl group, an n-butyl group, a sec-butyl group or a tert.-butyl group,
$R^6$ represents a $C_{1-6}$-alkyl group; a saturated, optionally at least mono-substituted cycloaliphatic group, which may be condensed with a mono- or polycyclic ring-system; or a phenyl group, which is optionally substituted with one or more $C_{1-6}$ alkyl groups, and
$R^7$ and $R^8$, identical or different, represent a hydrogen atom or a $C_{1-6}$ alkyl radical
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof.

**24.** Medicament according to one or more of claims 16-22, **characterized in that** it comprises at least one compound of general formula I, Ia or Ib

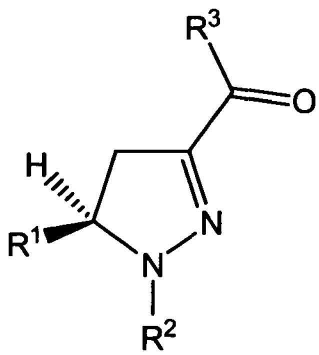

Ia

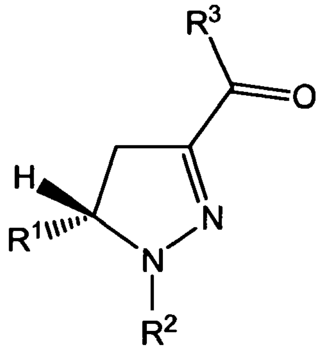

Ib

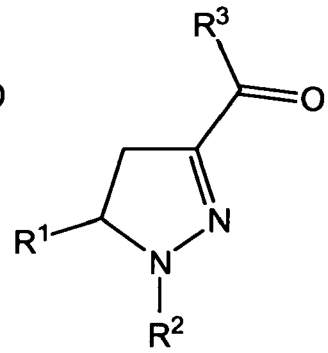

I

wherein

$R^1$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R'', - (C=O)-$NH_2$, -(C=O)-NHR' and -(C=O)-NR'R'', whereby R' and R'' at each ocurrence independently represent a linear or branched $C_{1-6}$ alkyl group,

$R^2$ represents a phenyl group, which is optionally substituted with 1, 2, 3, 4 or 5 substituents independently selected from the group consisting of linear or branched $C_{1-6}$-alkyl, linear or branched $C_{1-6}$-alkoxy, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R'', - (C=O)-$NH_2$, -(C=O)-NHR' and —(C=O)-NR'R'', whereby R' and R'' at each ocurrence independently represent a linear or branched $C_{1-6}$ alkyl group,

$R^3$ represents a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl, linear or branched $C_{1-6}$ alkoxy, OH, F, Cl, Br, I, CN, $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, or $R^3$ represents an —$NR^4R^5$-moiety,

$R^4$ represents a hydrogen atom or a linear or branched $C_{1-6}$-alkyl group,

$R^5$ represents a linear or branched $C_{1-6}$ alkyl group; an -$SO_2$-$R^6$-moiety; a saturated or unsaturated $C_{3-8}$ cycloaliphatic group, whereby said $C_{3-8}$ cycloaliphatic group is optionally substituted with 1, 2, 3 or 4 substituents independently selected from the group consisting of linear or branched $C_{1-6}$ alkyl group, a linear or branched $C_{1-6}$ alkoxy group, OH, F, Cl, Br, I, CN $CH_2F$, $CHF_2$, $CF_3$ and oxo (=O) and whereby said $C_{3-8}$ cycloaliphatic group may contain 1, 2 or 3 heteroatoms independently selected from the group consisting of N, O and S as ring members, and

$R^6$ represents a phenyl group, which is optionally substituted with 1, 2, 3, 4 or 5 substituents independently selected from the group consisting of a linear or branched $C_{1-6}$-alkyl group, a linear or branched $C_{1-6}$-alkoxy group, F, Cl, Br, I, $CH_2F$, $CHF_2$, $CF_3$, CN, OH, $NO_2$, -(C=O)-R', SH, SR', SOR', $SO_2R'$, $NH_2$, NHR', NR'R'', -(C=O)-$NH_2$, -(C=O)-NHR' and —(C=O)-NR'R'', whereby R' and R'' at each ocurrence independently represent a linear or branched $C_{1-6}$ alkyl group,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof.

**25.** Medicament according to one or more of claims 16-22 and 24, **characterized in that** it comprises at least one compound of general formula I, Ia or Ib

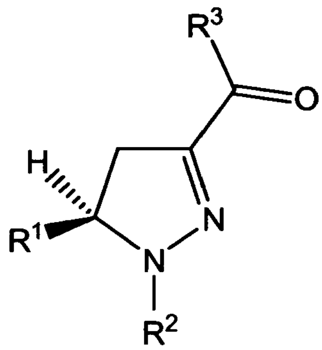
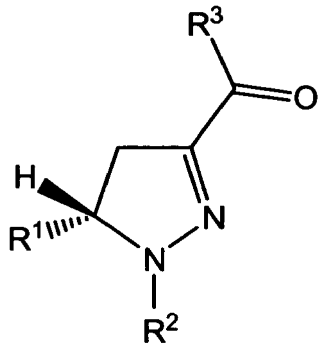
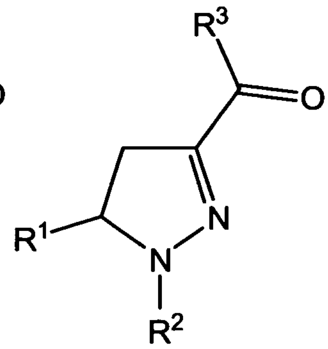

Ia Ib I

wherein

$R^1$ represents a phenyl group, which is optionally substituted with 1, 2, 3, 4 or 5 substituents independently selected from the group consisting of methyl, ethyl, F, Cl, Br and $CF_3$,
$R^2$ represents a phenyl group, which is optionally substituted with 1, 2, 3, 4 or 5 substituents independently selected from the group consisting of methyl, ethyl, F, Cl, Br and $CF_3$,
$R^3$ represents a pyrrolidinyl group, a piperidinyl group or a piperazinyl group, whereby each of these groups may be substituted with one or more of $C_{1-6}$-alkyl groups, or $R^3$ represents an —$NR^4R^5$-moiety,
$R^4$ represents a hydrogen atom or a linear or branched $C_{1-6}$-alkyl group,
$R^5$ represents a linear or branched $C_{1-6}$ alkyl group; an -$SO_2R^6$-moiety; a pyrrolidinyl group; a piperidinyl group; a piperazinyl group; a homo-piperazinyl group; a morpholinyl group; a triazolyl group; whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof.

**26.** Medicament according to one or more of claims 16-25, **characterized in that** it comprises at least one compound of general formula I, Ia or Ib

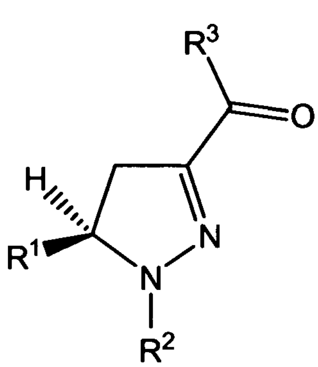
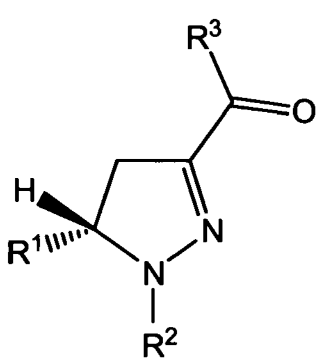
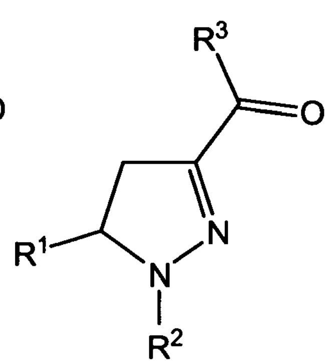

Ia Ib I

wherein

$R^1$ represents a phenyl ring, which is mono-substituted with a halogen atom, preferably a chlorine atom, in its 4-position,
$R^2$ represents a phenyl ring, which is di-substituted with two halogen atoms, preferably chlorine atoms, in its 2- and 4-position,
$R^3$ represents a pyrrolidinyl group, a piperidinyl group, a piperazinyl group, a homo-piperazinyl group, a morpholinyl group, or an —$NR^4R^5$-moiety,
$R^4$ represents a hydrogen atom or a linear or branched $C_{1-6}$-alkyl group,
$R^5$ represents a linear or branched $C_{1-6}$ alkyl group; an -$SO_2$-$R^6$-moiety; a pyrrolidinyl group; a piperidinyl group; a piperazinyl group; a homo-piperazinyl group; a morpholinyl group; or a triazolyl group whereby each of the heterocyclic rings may be substituted with one or more, identical or different, $C_{1-6}$-alkyl groups, and
$R^6$ represents a phenyl group, which is optionally substituted with one or more $C_{1-6}$ alkyl groups, which may be identical or different,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding solvate thereof.

**27.** Medicament according to one or more of claims 16 to 26, **characterized in that** it comprises at least one salt of a compound selected from the group consisting of:

N-piperidinyl-5-(4-chloro-phenyl)-1-(2,4-dichlorophenyl)-4,5-dihydro-1H-pyrazol-3-carboxamide,
(rac)-N-piperidinyl-5-(4-chloro-phenyl)-1-(2,4-dichlorophenyl)-4,5-dihydro-1H-pyrazol-3-carboxamide,
(R)-N-piperidinyl-5-(4-chloro-phenyl)-1-(2,4-dichlorophenyl)-4,5-dihydro-1H-pyrazol-3-carboxamide,
(S)-N-piperidinyl-5-(4-chloro-phenyl)-1-(2,4-dichlorophenyl)-4,5-dihydro-1H-pyrazol-3-carboxamide,
5-(4-Chloro-phenyl)-1-(2,4-dichloro-phenyl)-4,5-dihydro-1H-pyrazole-3-carboxylic acid-[1,2,4]-triazole-4-yl-amide,
5-(4-Chloro-phenyl)-1-(2,4-dichloro-phenyl)-4,5-dihydro-1H-pyrazole-3-carboxylic acid-(4-methyl-piperazin-1-yl)-amide,
5-(4-Chloro-phenyl)-1-(2,4-dichloro-phenyl)-4,5-dihydro-1H-pyrazole-3-carboxylic acid diethylamide,
[5-(4-Chloro-phenyl)-1-(2,4-dichloro-phenyl)-4,5-dihydro-1H-pyrazole-3-yl]-piperidine-1-yl-methanone,
N-[5-(4-Chloro-phenyl)-1-(2,4-dichlorophenyl)-4,5-dihydro-1H-pyrazole-3-carbonyl]-4-methylphenylsulfonamide,
1-{[5-(4-Chloro-phenyl)-1-(2,4-dichloro-phenyl)-4,5-dihydro-1H-pyrazole-3-carbonyl]-amino}-piperidinium; chloride,
1-{[5(R)-(4-Chloro-phenyl)-1-(2,4-dichloro-phenyl)-4,5-dihydro-1H-pyrazole-3-carbonyl]-amino}-piperidinium; chloride,
1-{[5(S)-(4-Chloro-phenyl)-1-(2,4-dichloro-phenyl)-4,5-dihydro-1H-pyrazole-3-carbonyl]-amino}-piperidinium; chloride
1-{[5-(4-Chloro-phenyl)-1-(2,4-dichloro-phenyl)-4,5-dihydro-1H-pyrazole-3-carbonyl]-amino}-piperidinium; nitrate,
1-{[5(R)-(4-Chloro-phenyl)-1-(2,4-dichloro-phenyl)-4.5-dihydrc-1H-pyrazloe-3-carbonyl]-amino}-piperidinium; nitrate,
1-{[5(S)-(4-Chloro-phenyl)-1-(2,4-dichloro-phenyl)-4,5-dihydro-1H-pyrazole-3-carbonyl]-amino}-piperidinium; nitrate,
1-{[5-(4-Chloro-phenyl)-1-(2,4-dichloro-phenyl)-4,5-dihydro-1H-pyrazole-3-carbonyl]-amino}-piperidinium; hydrogen sulfate,
1-{[5(R)-(4-Chloro-phenyl)-1-(2,4-dichloro-phenyl)-4,5-dihydro-1H-pyrazole-3-carbonyl]-amino}-piperidinium; hydrogen sulfate,
1-{[5(S)-(4-Chloro-phenyl)-1-(2,4-dichloro-phenyl)-4,5-dihydro-1H-pyrazole-3-carbonyl]-amino}-piperidinium; hydrogen sulfate,
Methanesulfonate1-{[5-(4-chloro-phenyl)-1-(2,4-dichloro-phenyl)-4,5-dihydro-1 H-pyrazole-3-carbonyl]-amino}-piperidinium,
Methanesulfonate1-{[5(R)-(4-chloro-phenyl)-1-(2,4-dichloro-phenyl)-4,5-dihydro-1H-pyrazole-3-carbonyl]-amino}-piperidinium,
Methanesulfonate1-{[5(S)-(4-chloro-phenyl)-1-(2,4-dichloro-phenyl)-4,5-dihydro-1H-pyrazole-3-carbonyl]-amino}-piperidinium,
1-{[5-(4-Chloro-phenyl)-1-(2,4-dichloro-phenyl)-4,5-dihydro-1H-pyrazole-3-carbonyl]-amino}-piperidinium; bromide,
1-{[5(R)-(4-Chloro-phenyl)-1-(2,4-dichloro-phenyl)-4,5-dihydro-1H-pyrazole-3-carbonyl]-amino}-piperidinium;

bromide,
1-{[5(S)-(4-Chloro-phenyl)-1-(2,4-dichloro-phenyl)-4,5-dihydro-1H-pyrazole-3-carbonyl]-amino}-piperidinium; bromide,
Ethanesulfonate1-{[5-(4-chloro-phenyl)-1-(2,4-dichloro-phenyl)-4,5-dihydro-1 H-pyrazole-3-carbonyl]-amino}-piperidinium,
Ethanesulfonate1-{[5(R)-(4-chloro-phenyl)-1-(2,4-dichloro-phenyl)-4,5-dihydro-1 H-pyrazole-3-carbonyl]-amino}-piperidinium,
Ethanesulfonate1-{[5(S)-(4-chloro-pheny)-1-(2,4-dichloro-phenyl)-4,5-dihydro-1 H-pyrazole-3-carbonyl]-amino}-piperidinium,
Toluene-4-sulfonate1-{[5-(4-chloro-phenyl)-1-(2,4-dichloro-phenyl)-4,5-dihydro-1H-pyrazole-3-carbonyl]-amino}-piperidinium,
Toluene-4-sulfonate1-{[5(R)-(4-chloro-phenyl)-1-(2,4-dichloro-phenyl)-4,5-dihydro-1H-pyrazole-3-carbonyl]-amino}-piperidinium,
Toluene-4-sulfonate1-{[5(S)-(4-chloro-phenyl)-1-(2,4-dichloro-phenyl)-4,5-dihydro-1H-pyrazole-3-carbonyl]-amino}-piperidinium,
Benzenesulfonate1-{[5-(4-chloro-phenyl)-1-(2,4-dichloro-phenyl)-4,5-dihydro-1 H-pyrazole-3-carbonyl]-amino}-piperidinium,
Benzenesulfonate1-{[5(R)-(4-chloro-phenyl)-1-(2,4-dichloro-phenyl)-4,5-dihydro-1H-pyrazole-3-carbonyl]-amino}-piperidinium,
Benzenesulfonate1-{[5(S)-(4-chloro-phenyl)-1-(2,4-dichloro-phenyl)-4,5-dihydro-1H-pyrazole-3-carbonyl]-amino}-piperidinium,
2,5-Dihydroxy-benzenesulfonate1-{[5-(4-chloro-phenyl)-1-(2,4-dichlorophenyl)-4,5-dihydro-1H-pyrazole-3-carbonyl]-amino}-piperidinium,
2,5-Dihydroxy-benzenesulfonate1-{[5(R)-(4-chloro-phenyl)-1-(2,4-dichlorophenyl)-4,5-dihydro-1H-pyrazole-3-carbonyl]-amino}-piperidinium,
2,5-Dihydroxy-benzenesulfonate1-{[5(S)-(4-chloro-phenyl)-1-(2,4-dichlorophenyl)-4,5-dihydro-1H-pyrazole-3-carbonyl]-amino}-piperidinium,
Naphthalene-2-sulfonate1-{[5-(4-chloro-phenyl) 1-(2,4-dichloro-phenyl)-4,5-dihydro-1H-pyrazole-3-carbonyl]-amino}-piperidinium,
Naphthalene-2-sulfonate1-{[5(R)-(4-chloro-phenyl)-1-(2,4-dichloro-phenyl)-4,5-dihydro-1H-pyrazole-3-carbonyl]-amino}-piperidinium,
Naphthalene-2-sulfonate1-{[5(S)-(4-chloro-phenyl)-1-(2,4-dichloro-phenyl)-4,5-dihydro-1H-pyrazole-3-carbonyl]-amino}-piperidinium,
5-Sulfo-naphthalene-1-sulfonate1-{[5-(4-chloro-phenyl)-1-(2,4-dichlorophenyl)-4,5-dihydro-1H-pyrazole-3-carbonyl]-amino}-piperidinium,
5-Sulfo-naphthalene-1-sulfonate1-{[5(R)-(4-chloro-phenyl)-1-(2,4-dichlorophenyl)-4,5-dihydro-1H-pyrazole-3-carbonyl]-amino}-piperidinium,
5-Sulfo-naphthalene-1-sulfonate1-{[5(S)-(4-chloro-phenyl)-1-(2,4-dichlorophenyl)-4,5-dihydro-1H-pyrazole-3-carbonyl]-amino}-piperidinium,
Cyclamate1-{[5-(4-chloro-phenyl)-1-(2,4-dichloro-phenyl)-4,5-dihydro-1H-pyrazole-3-carbonyl]-amino}-piperidinium,
Cyclamate1-{[5(R)-(4-chloro-phenyl)-1-(2,4-dichloro-phenyl)-4,5-dihydro-1H-pyrazole-3-carbonyl]-amino}-piperidinium,
Cyclamate1-{[5(S)-(4-chloro-phenyl)-1-(2,4-dichloro-phenyl}-4,5-dihydro-1H-pyrazole-3-carbonyl]-amino}-piperidinium,
Dodecane-1-sulfonate1-{[5-(4-chloro-phenyl)-1-(2,4-dichloro-phenyl)-4,5-dihydro-1H-pyrazole-3-carbonyl]-amino}-piperidinium,
Dodecane-1-sulfonate1-{[5(R)-(4-chloro-phenyl)-1-(2,4-dichloro-phenyl)-4,5-dihydro-1H-pyrazole-3-carbonyl]-amino}-piperidinium,
Dodecane-1-sulfonate1-{[5(S)-(4-chloro-phenyl)-1-(2,4-dichloro-phenyl)-4,5-dihydro-1H-pyrazole-3-carbonyl]-amino}-piperidinium,
Thiocyanate1-{[5-(4-chloro-phenyl)-1-(2,4-dichloro-phenyl)-4,5-dihydro-1H-pyrazole-3-carbonyl]-amino}-piperidinium,
Thiocyanate1-{[5(R)-(4-chloro-phenyl)-1-(2,4-dichloro-phenyl)-4,5-dihydro-1H-pyrazole-3-carbonyl]-amino}-piperidinium,
Thiocyanate1-{[5(S)-(4-chloro-phenyl)-1-(2,4-dichloro-phenyl)-4,5-dihydro-1H-pyrazole-3-carbonyl]-amino}-piperidinium,
1-{[5-(4-Chloro-phenyl)-1-(2,4-dichloro-phenyl)-4,5-dihydro-1H-pyrazole-3-carbonyl]-amino}-piperidinium; dihydrogen phosphate,
1-{[5(R)-(4-Chloro-phenyl)-1-(2,4-dichloro-phenyl)-4,5-dihydro-1H-pyrazole-3-carbonyl]-amino}-piperidinium; dihydrogen phosphate,
1-{[5(S)-(4-Chloro-phenyl)-1-(2,4-dichloro-phenyl)-4,5-dihydro-1H-pyrazole-3-carbonyl]-amino}-piperidinium; dihydrogen phosphate,
3-Carboxy-acrylate-1-{[5-(4-chloro-phenyl)-1-(2,4-dichloro-phenyl)-4,5-dihydro-1 H-pyrazole-3-carbonyl]-amino}-piperidinium,
3-Carboxy-acrylate1-{[5(R)-(4-chloro-phenyl)-1-(2,4-dichloro-phenyl)-4,5-dihydro-1H-pyrazole-3-carbonyl]-amino}-piperidinium,
3-Carboxy-acrylate1-{[5(S)-(4-chloro-phenyl)-1-(2,4-dichloro-phenyl)-4,5-dihydro-1H-pyrazole-3-carbonyl]-amino}-piperidinium,
(7,7-Dimethyl-2-oxo-bicyclo[2.2.1]hept-1-yl)-methanesulfonate1-{[5-(4-chlorophenyl)-1-(2,4-dichloro-phenyl)-4,5-dihydro-1H-pyrazole-3-carbonyl]-amino}-piperidinium,
(7,7-Dimethyl-2-oxo-bicyclo[2.2.1]hept-1-yl)-methanesulfonate1-{[5(R)-(4-chloro-phenyl)-1-(2,4-dichloro-phenyl)-4,5-dihydro-1H-pyrazole-3-carbonyl]-amino}-piperidinium,
(7,7-Dimethyl-2-oxo-bicyclo[2.2.1]hept-1-yl)-methanesulfonate1-{[5(S)-(4-chloro-phenyl)-1-(2,4-dichloro-phenyl)-4,5-dihydro-1H-pyrazole-3-carbonyl]-amino}-piperidinium,
optionally in the form of a corresponding N-oxide, or a corresponding solvate.

**28.** Medicament according to one or more of claims 16-27 for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**29.** Medicament according to one or more of claims 16-27 for the prophylaxis and/or treatment of food intake disorders, preferably bulimia, anorexia, cachexia, obesity, type II diabetus mellitus (non-insuline dependent diabetes mellitus), more preferably obesity.

**30.** Medicament according to one or more of claims 16-27 for the prophylaxis and/or treatment of psychosis.

**31.** Medicament according to one or more of claims 16-27 for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**32.** Medicament according to one or more of claims 16-27 for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**33.** Medicament according to one or more of claims 16-27 for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

**34.** Use of at least one salt of a substituted pyrazoline compound according to one or more of claims 1-15 including salts of the disclaimed substituted pyrazoline compounds and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the

respiratory system, disorders of the gastrointestinal tract or reproductive disorders.

**35.** Use of at least one salt of a substituted pyrazoline compound according to one or more of claims 1-15 including salts of the disclaimed substituted pyrazoline compounds and optionally one or more pharmaceutically acceptable excipients, for the preparation of a medicament for the prophylaxis and/or treatment of food intake disorders, preferably bulimia, anorexia, cachexia, obesity, type II diabetus mellitus (non-insuline dependent diabetes mellitus), more preferably obesity.

**36.** Use of at least one salt of a substituted pyrazoline compound according to one or more of claims 1-15 including salts of the disclaimed substituted pyrazoline compounds and optionally one or more pharmaceutically acceptable excipients, for the preparation of a medicament for the prophylaxis and/or treatment of psychosis.

**37.** Use of at least one salt of a substituted pyrazoline compound according to one or more of claims 1-15 including salts of the disclaimed substituted pyrazoline compounds and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**38.** Use of at least one salt of a substituted pyrazoline compound according to one or more of claims 1-15 including salts of the disclaimed substituted pyrazoline compounds and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**39.** Use of at least one salt of a substituted pyrazoline compound according to one or more of claims 1-15 including salts of the disclaimed substituted pyrazoline compounds and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Figure 2:

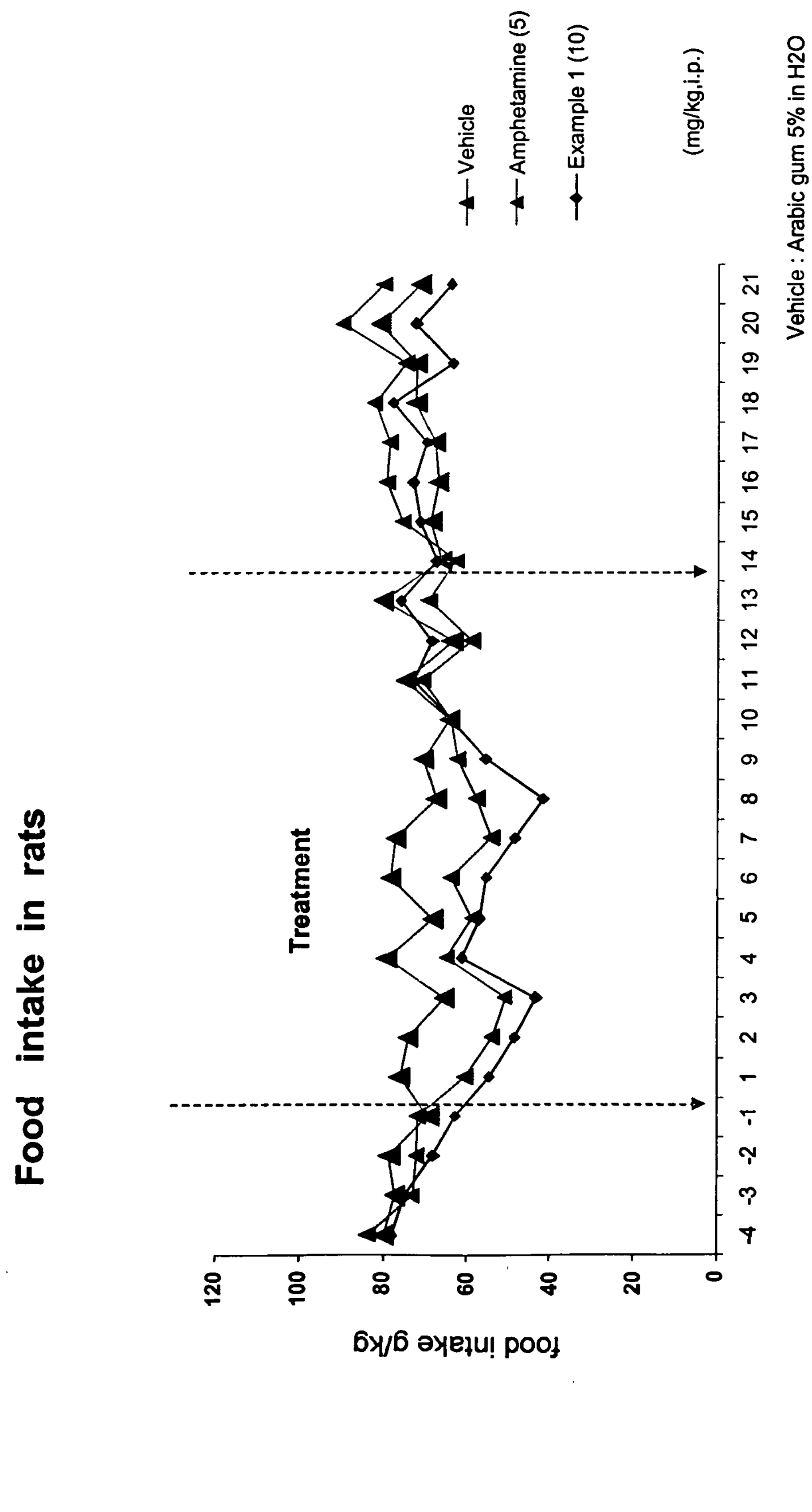

Food intake in rats
Treatment
food intake g/kg
120
100
80
60
40
20
0
-4 -3 -2 -1 1 2 3 4 5 6 7 8 9 10 11 12 13 14 15 16 17 18 19 20 21
Days
Vehicle
Amphetamine (5)
Example 1 (10)
(mg/kg,i.p.)
Vehicle : Arabic gum 5% in H2O

Figure 1

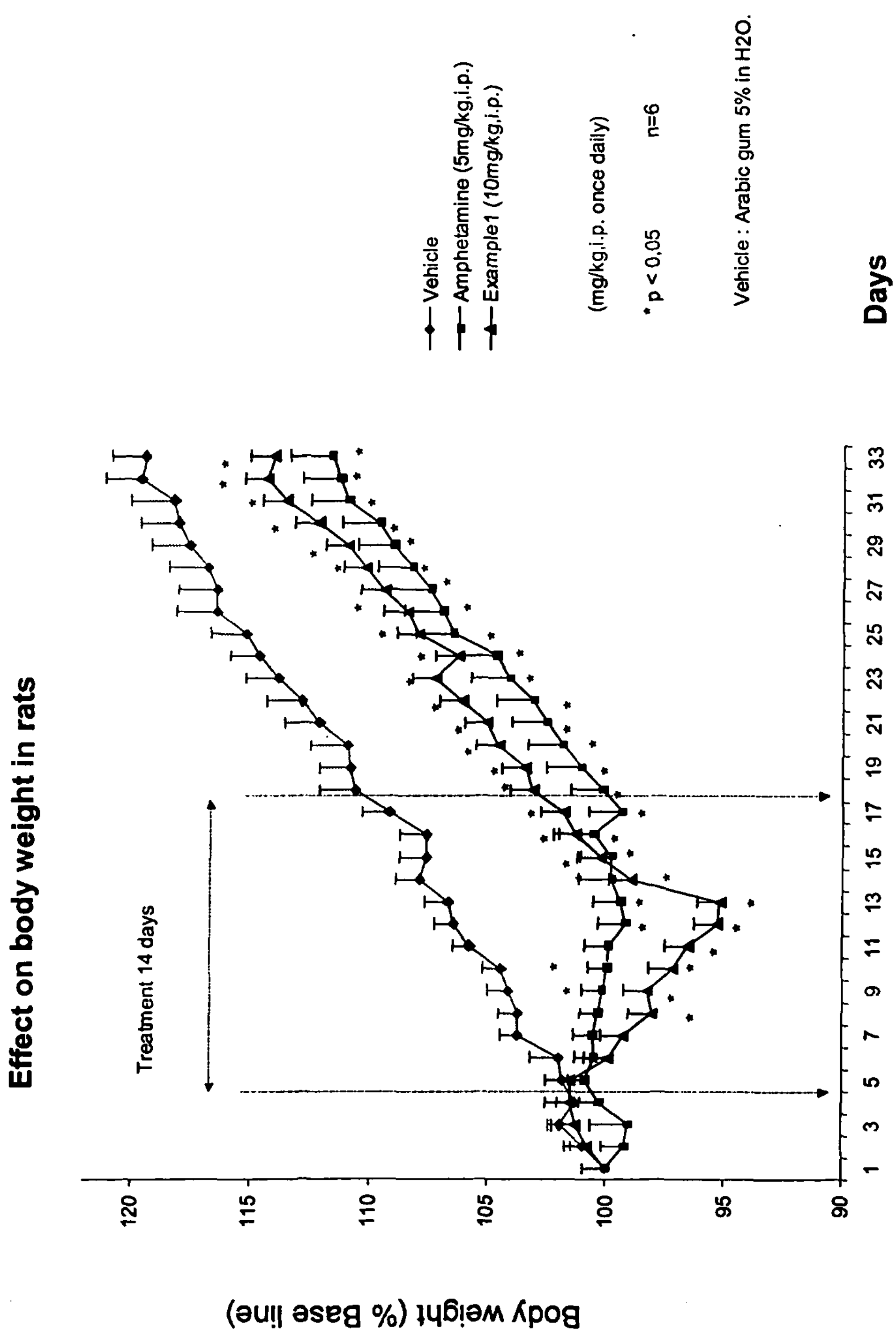
Effect on body weight in rats
Treatment 14 days
Body weight (% Base line)
120
115
110
105
100
95
90
1 3 5 7 9 11 13 15 17 19 21 23 25 27 29 31 33
Days
Vehicle
Amphetamine (5mg/kg,i.p.)
Example1 (10mg/kg,i.p.)
(mg/kg,i.p. once daily)
* p < 0,05
n=6
Vehicle : Arabic gum 5% in H2O.

European Patent Office

# EUROPEAN SEARCH REPORT

Application Number
EP 05 38 4025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 03/026647 A (SOLVAY PHARMACEUTICALS B.V; KRUSE, CORNELIS, G; LANGE, JOSEPHUS, H.M;) 3 April 2003 (2003-04-03) * the whole document * ----- | 1-39 | C07D231/06 A61K31/4155 A61P25/00 |
| Y | WO 01/70700 A (SOLVAY PHARMACEUTICALS B.V) 27 September 2001 (2001-09-27) * the whole document * ----- | 1-39 | |
| Y | SHIM J-Y ET AL: "Molecular interaction of the antagonist N-(piperidin-1-yl)-5-(4-chlorophenyl)-1-(2,4-dichlorophenyl)-4-methyl-1H-pyrazole-3-carboxamide with the CB1 cannabinoid receptor" JOURNAL OF MEDICINAL CHEMISTRY, AMERICAN CHEMICAL SOCIETY. WASHINGTON, US, vol. 45, no. 7, March 2002 (2002-03), pages 1447-1459, XP002968557 ISSN: 0022-2623 * the whole document * ----- | 1-39 | TECHNICAL FIELDS SEARCHED (IPC) |
| X | WO 88/05046 A (E.I. DU PONT DE NEMOURS AND COMPANY; STEVENSON, THOMAS, MARTIN) 14 July 1988 (1988-07-14) * examples * ----- | 1 | C07D A61K A61P |
| A | PATENT ABSTRACTS OF JAPAN vol. 014, no. 326 (C-0740), 12 July 1990 (1990-07-12) & JP 02 117605 A (NIPPON NOHYAKU CO LTD), 2 May 1990 (1990-05-02) * abstract * ----- | 1-39 | |
| E | WO 2005/077911 A (LABORATORIOS DEL DR. ESTEVE S.A; CUBERES ALTISEN, ROSA; FRIGOLA CONSTA) 25 August 2005 (2005-08-25) * the whole document * ----- -/-- | 1-39 | |

The present search report has been drawn up for all claims

| Place of search | Date of completion of the search | Examiner |
|---|---|---|
| Munich | 12 December 2005 | Lauro, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

European Patent Office

# EUROPEAN SEARCH REPORT

Application Number
EP 05 38 4025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2005/074920 A (SOLVAY PHARMACEUTICALS B.V; LANGE, JOSEPHUS H.M; KRUSE, CORNELIS G; VA) 18 August 2005 (2005-08-18)<br>* the whole document *<br>----- | 1-39 | |

TECHNICAL FIELDS SEARCHED (IPC)

The present search report has been drawn up for all claims

| Place of search | Date of completion of the search | Examiner |
|---|---|---|
| Munich | 12 December 2005 | Lauro, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

4

EPO FORM 1503 03.82 (P04C01)

**ANNEX TO THE EUROPEAN SEARCH REPORT ON EUROPEAN PATENT APPLICATION NO.** EP 05 38 4025

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2005

| Patent document cited in search report | | Publication date | Patent family member(s) | | | Publication date |
|---|---|---|---|---|---|---|
| WO 03026647 | A | 03-04-2003 | BR | 0212044 | A | 17-08-2004 |
| | | | CA | 2456606 | A1 | 03-04-2003 |
| | | | CN | 1555262 | A | 15-12-2004 |
| | | | HR | 20040085 | A2 | 31-08-2004 |
| | | | HU | 0401567 | A2 | 28-01-2005 |
| | | | JP | 2005503427 | T | 03-02-2005 |
| | | | MX | PA04002583 | A | 18-06-2004 |
| | | | NO | 20041176 | A | 21-06-2004 |
| | | | US | 2004248944 | A1 | 09-12-2004 |
| | | | ZA | 200402099 | A | 26-04-2005 |
| WO 0170700 | A | 27-09-2001 | AU | 4250101 | A | 03-10-2001 |
| | | | BR | 0109457 | A | 03-06-2003 |
| | | | CA | 2401832 | A1 | 27-09-2001 |
| | | | CN | 1419546 | A | 21-05-2003 |
| | | | HK | 1052349 | A1 | 23-09-2005 |
| | | | HU | 0204519 | A2 | 28-05-2003 |
| | | | JP | 2004500401 | T | 08-01-2004 |
| | | | NO | 20024531 | A | 19-11-2002 |
| | | | PL | 358101 | A1 | 09-08-2004 |
| | | | SK | 13522002 | A3 | 04-03-2003 |
| | | | US | 2001053788 | A1 | 20-12-2001 |
| WO 8805046 | A | 14-07-1988 | AU | 1154488 | A | 26-09-1988 |
| | | | BR | 8707672 | A | 03-10-1989 |
| | | | CN | 88100104 | A | 20-07-1988 |
| | | | EP | 0330678 | A1 | 06-09-1989 |
| | | | ES | 2008408 | A6 | 16-07-1989 |
| | | | JP | 1502513 | T | 31-08-1989 |
| | | | JP | 5081591 | B | 15-11-1993 |
| | | | WO | 8806583 | A1 | 07-09-1988 |
| JP 02117605 | A | 02-05-1990 | NONE | | | |
| WO 2005077911 | A | 25-08-2005 | NONE | | | |
| WO 2005074920 | A | 18-08-2005 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992, vol. 459
- *Synthetic communications,* 1996, vol. 26 (11), 2229-33
- *Synlett,* 2001, 147-149
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- **KROGSGAARD-LARSEN et al.** Textbook of Drugdesign and Discovery. Taylor & Francis, April 2002
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **A. C. HOWLETT et al.** International Union of Pharmacology XXVII. Classification of Cannabinoid Receptors. *Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117
- **E.T. TZAVARA et al.** The CB1 receptor antagonist SR141716A selectively increases monoaminergic neurotransmission in the medial prefrontal cortex: implications for therapeutic actions. *Br. J. Pharmacol.,* 2003, vol. 138 (4), 544-553